# EUROPEAN PATENT SPECIFICATION

(11) **EP 3 530 739 B1**
(45) Date of publication and mention of the grant of the patent: **19.08.2020**
(21) Application number: 18460010.4
(22) Date of filing: 22.02.2018
(51) Int. Cl.: C12N 15/31, C12N 15/81, C12N 1/16, C12P 21/02, C07K 14/39

(54) **EXPRESSION SYSTEM FOR PRODUCTIN OF A HETEROLOGOUS PROTEIN, PLASMID EXPRESSION VECTORS, METHOD OF CONSTRUCTION OF A RECOMBINANT STRAIN OF PSYCHROTOLERANT YEAST DEBARYOMYCES MACQUARIENSIS AND METHOD OF PROTEIN PRODUCTION BY THE RECOMBINANT YEAST STRAIN**
EXPRESSIONSSYSTEM ZUR HERSTELLUNG VON HETEROLOGEM PROTEIN, PLASMIDEXPRESSIONSVEKTOREN, VERFAHREN ZUR HERSTELLUNG EINES REKOMBINANTEN STAMMS VON PSYCHROTOLERANTER HEFE DEBARYOMYCES MACQUARIENSIS UND VERFAHREN ZUR PROTEINHERSTELLUNG MIT REKOMBINANTEM HEFESTAMM
SYSTÈME D'EXPRESSION POUR LA PRODUCTION D'UNE PROTÉINE HÉTÉROLOGUE, VECTEURS D'EXPRESSION PLASMIDIQUE, PROCÉDÉ DE CONSTRUCTION D'UNE SOUCHE DE RECOMBINAISON DE DEBARYOMYCES DE LEVURE PSYCHROTOLÉRANTS MACQUARIENSIS ET PROCÉDÉ DE PRODUCTION DE PROTÉINES AU MOYEN DE LA SOUCHE DE LEVURE RECOMBINÉE

(43) Date of publication of application: 28.08.2019
(73) Proprietor: Politechnika Gdanska, 80-233 Gdansk (PL); Politechnika Lódzka, 90-924 Lodz (PL)
(72) Inventor: Filipowicz, Natalia, 80-298 Gdansk (PL); Gromek, Ewa, 93-259 Lodz (PL); Turkiewicz, Marianna, 90-303 Lodz (PL); Bialkowska, Aneta, 90-924 Lodz (PL); Krysiak, Joanna, 91-022 Lodz (PL); Florczak, Tomasz, 95-015 Glowno (PL); Wanarska, Marta, 80-262 Gdansk (PL); Cieslinski, Hubert, 80-180 Gdansk (PL); Krajewska, Ewelina, 81-732 Sopot (PL); Wicka-Grochocka, Monika, 83-110 Tczew (PL); Pawlak-Szukalska, Anna, 80-253 Gdansk (PL)
(74) Representative: Pawlowska, Justyna

(56) References cited:
- JACQUES NOEMIE ET AL: "Delimitation of the species of the Debaryomyces hansenii complex by intron sequence analysis", INTERNATIONAL JOURNAL OF SYSTEMATIC AND EVOLUTIONARY MICROBIOLOGY, vol. 59, no. Part 5, May 2009 (2009-05), pages 1242-1251, XP055493028, ISSN: 1466-5026
- ANNA WIERZBICKA-WOS ET AL: "A novel cold-active [beta]-D-galactosidase from the Paracoccus sp. 32d - gene cloning, purification and characterization", MICROBIAL CELL FACTORIES,, vol. 10, no. 1, 13 December 2011 (2011-12-13), page 108, XP021093666, ISSN: 1475-2859, DOI: 10.1186/1475-2859-10-108
- RYOMA MIYAKE ET AL: "Construction of a low-temperature protein expression system using a cold adapted bacterium, Shewanella sp. strain Ac10, as the host", APPLIED AND ENVIRONMENTAL MICROBIOLOGY, AMERICAN SOCIETY FOR MICROBIOLOGY, US, vol. 73, no. 15, 25 May 2007 (2007-05-25), pages 4849-4856, XP008129206, ISSN: 0099-2240, DOI: 10.1128/AEM.00824-07
- SYBIRNA KATERYNA ET AL: "Shewanella oneidensis: a new and efficient System for Expression and Maturation of heterologous [Fe-Fe] Hydrogenase from Chlamydomonas reinhardtii", BMC BIOTECHNOLOGY, BIOMED CENTRAL LTD. LONDON, GB, vol. 8, no. 1, 18 September 2008 (2008-09-18), page 73, XP021043270, ISSN: 1472-6750, DOI: 10.1186/1472-6750-8-73
- CUSANO ANGELA MARIA ET AL: "A novel genetic system for recombinant protein secretion in the Antarctic Pseudoalteromonas haloplanktis TAC125", MICROBIAL CELL FACTORIES,, vol. 5, no. 1, 14 December 2006 (2006-12-14), page 40, XP021023936, ISSN: 1475-2859, DOI: 10.1186/1475-2859-5-40
- PAPA R.; RIPPA V.; SANNIA G.; MARINO G.; DUILIO A.: "An efficient cold inducible expression-system developed in Pseudoalteromonas haloplanktis TAC125", J. BIOTECHNOL., vol. 127, 2007, pages 199-210, XP002783114,
- CHUNLI SONG ET AL: "Beta-Galactosidase production by the psychrotolerant yeast Guehomyces pullulans 17-1 isolated from sea sediment in Antarctica and lactose hydrolysis", BIOPROCESS AND BIOSYSTEMS ENGINEERING, SPRINGER, BERLIN, DE, vol. 33, no. 9, 18 April 2010 (2010-04-18) , pages 1025-1031, XP019849814, ISSN: 1615-7605

### Sequence Listing

<110> Gdansk University of Technology, Lodz University of Technology
<120> An expression system for production of a heterologous protein, plasmid
   expression vectors, a method of construction of a recombinant strain of psychrotolerant yeast *Debaryomyces macquariensis* and a method of protein production by the recombinant yeast strain
<160> 38
<210> 1
   <211> 500
   <212> DNA
   <213> *Debaryomyces macquariensis* D50
<400> 1
   taccacaatt caagacggcc cttatgtgcc tgttctccaa cgtgtcctta tcagttcata     60
   atcgccacct ggttgtgtct caatcgccag atttaccggt tctcctaaat tgcgcctaca    120
   tttgtcgttc tcacgtcctg tgctctcttt ctacatggac gagttgctgc caacgaccat    180
   ttttcacggt atcaagtggt ggttagcctc ccggcgaccc tggacggctc gcttccgttg    240
   atccttccat actaattaac ggcctcggac ttttgtagcg cgaaatcatg agaatcacca    300
   aactcccggc aactagctcg gtcgtgcccg gcaccggggc ctctatggga ttcttcgccc    360
   cgggagtcca gcaataagaa catagtggaa aattataaat atgagtaatt cgccgtgcaa    420
   tcgacatgga gtatatttta aaggttatct tattattcaa ttgaatataa ttaactagta    480
   agaatattta ataattaaac                                                500
<210> 2
   <211> 659
   <212> DNA
   <213> *Debaryomyces macquariensis* D50
<400> 2
   cactactcag acttaccacc gcatatacag acattaacca taatagtgcc atctacaggg     60
   cacaatgcat ttaatacaca atctaatata cagcttaagt agagcccatc tgtaattaaa    120
   cccacacaaa gagtaattca gtcacatata ctaatgtgaa cggcattcat ttgacctagt    180
   gtcaacatgg caaaagcttc cgccgcagtt tcgcaatttc actacatgtc gcctgtcatc    240
   agctacgaac tgctatgtaa gatcaactgc aaaatgttcc actggtccat aatcgaagtt    300
   tctaggagtt tgccaaaaaa tttagtccca tactatccca accaaatgtc ctcaaataac    360
   taccatcaca cctattttgc caagttcttc ctatctacta tatagctact tcatgctgct    420
   tgcaataccg tagtctacgg ccatagcact tggattctga atacttagag gaaatagggt    480
   aaagccctat ccgggtaaat taaatatttg tcctgctgaa aaaaaaaatt tcgccacgac    540
   accctctgat gaatggaacg caattggctc tgcaaaaatt tataaggaat taaattgaag    600
   aacttctttt tccttctctt tttttcaata cagtatacta ttattataca ttaaacaaa     659
<210> 3
   <211> 339
   <212> DNA
   <213> *Candida santamariae* G12
<400> 3
   acttctcttc ttactttcac tcctttccct acacggttat tgtacgtgtc tattccggta     60
   tttaaattag ctatgctgaa aattacgaca gaatgttttt attgccgcgt gtattttcaa    120
   ttatatagta gtatttaagc atcgtcgacc tgcacaaaac cctcatcgta ccatatttta    180
   cccatactag ttgaaaattt tttttttttt tgctctgcgg gtgagggggc gttgcccaat    240
   atataaggag tggcccttgt cttagacaaa aattttccag gttataagtt tttttttttt    300
   cttcttcttt tacttcatct aaactaaact taattaaaa                          339
<210> 4
   <211> 266
   <212> DNA
   <213> *Debaryomyces macquariensis* D50
<400> 4
   accatttatt agatgttgaa aagtttactc gtcgttttgt acaaacactg tttaatgggc     60
   ctcgaattta gctagttaca catttatctg tatatacatg aataatatta acgaacgatg    120
   ttagattaaa tgcttacagt agagtacaag cagtgggggt tatatttgga tggacattat    180
   ctgttcacgt gacccagtat gagtgaaaag ttcgccaagt caaccactgt attacagacg    240
   gcaattatat tacaattagt ggcaac                                         266
<210> 5
   <211> 576
   <212> DNA
   <213> *Candida santamariae* G12
<400> 5
   gatatcattt agaacactct agataagcaa aagcaaaata aaagcgaaat gctctgtata     60
   taataattta tgttttattt aatgatagag tgtagagagt gagagagatt gtaagccccc    120
   agaaaatgaa gaaaaatatt tattgatgaa gatgagaata cttttatgag accctccatg    180
   ggttgcttta tgcaagggag atgcagtttt gcatgagatc gagattactt atcctagaag    240
   ttgaatttat aataggcgta ttagaggtag aaaatagcac ttttttaaga ataacttgaa    300
   taatctccat taatcaaaaa cacaaacatt aactaagcgt gtcatatttt acttttttct    360
   ataatcatct ctatcgcctt tttctttttt ttctcagatt ctatttctca aacacactct    420
   ctctgactct cactctcact ttcactcttt gacttgtttc atctgaaaaa tcctgcatat    480
   gtgcaactag tgtttcgata tatcgatctg taccttcttg actaaccgct ctttcacttc    540
   tctttcgtca aacttcgctt acattataat acatgt                              576
<210> 6
   <211> 55
   <212> DNA
   <213> Artificial
<220>
   <223> A primer with the symbol CYCD50zaAOX1for used to obtain a PCR product with a
   symbol H3.1
<400> 6 aagaccggtc ttgctagatt ctaaaccatt tattagatgt tgaaaagttt actcg 55
<210> 7
   <211> 52
   <212> DNA
   <213> Artificial
<220>
   <223> A primer with the symbol CYCD50zaAOXlrev used to obtain a PCR product with a
   symbol H3.1
<400> 7
   gggggatccg cacaaacgaa gggttgccac taattgtaat ataattgccg tc 52
<210> 8
   <211> 312
   <212> DNA
   <213> Artificial
<220>
   <223> A PCR product with a symbol H3.1 used to obtain a plasmid with a symbol
   pGAPzCYCD50bezAOX1
<400> 8
   aagaccggtc ttgctagatt ctaaaccatt tattagatgt tgaaaagttt actcgtcgtt      60
   ttgtacaaac actgtttaat gggcctcgaa tttagctagt tacacattta tctgtatata     120
   catgaataat attaacgaac gatgttagat taaatgctta cagtagagta caagcagtgg     180
   gggttatatt tggatggaca ttatctgttc acgtgaccca gtatgagtga aaagttcgcc     240
   aagtcaacca ctgtattaca gacggcaatt atattacaat tagtggcaac ccttcgtttg     300
   tgcggatccc cc                                                         312
<210> 9
   <211> 3170
   <212> DNA
   <213> Artificial
<220>
   <223> A plasmid with a symbol pGAPzCYCD50bezAOX1 used to obtain a plasmid
   expression vector with a symbol pH3
<400> 9
   agatcttttt tgtagaaatg tcttggtgtc ctcgtccaat caggtagcca tctctgaaat      60
   atctggctcc gttgcaactc cgaacgacct gctggcaacg taaaattctc cggggtaaaa     120
   cttaaatgtg gagtaatgga accagaaacg tctcttccct tctctctcct tccaccgccc     180
   gttaccgtcc ctaggaaatt ttactctgct ggagagcttc ttctacggcc cccttgcagc     240
   aatgctcttc ccagcattac gttgcgggta aaacggaggt cgtgtacccg acctagcagc     300
   ccagggatgg aaaagtcccg gccgtcgctg gcaataatag cgggcggacg catgtcatga     360
   gattattgga aaccaccaga atcgaatata aaaggcgaac acctttccca attttggttt     420
   ctcctgaccc aaagacttta aatttaattt atttgtccct atttcaatca attgaacaac     480
   tatttcgaaa cgatgagatt tccttcaatt tttactgctg ttttattcgc agcatcctcc     540
   gcattagctg ctccagtcaa cactacaaca gaagatgaaa cggcacaaat tccggctgaa     600
   gctgtcatcg gttactcaga tttagaaggg gatttcgatg ttgctgtttt gccattttcc     660
   aacagcacaa ataacgggtt attgtttata aatactacta ttgccagcat tgctgctaaa     720
   gaagaagggg tatctctcga gaaaagagag gctgaagctg caggaattca cgtggcccag     780
   ccggccgtct cggatcggta cctcgagccg cggcggccgc cagctttcta gaacaaaaac     840
   tcatctcaga agaggatctg aatagcgccg tcgaccatca tcatcatcat cattgagttt     900
   tagccttaga catgactgtt cctcagttca agttgggcac ttacgagaag accggtcttg     960
   ctagattcta aaccatttat tagatgttga aaagtttact cgtcgttttg tacaaacact    1020
   gtttaatggg cctcgaattt agctagttac acatttatct gtatatacat gaataatatt    1080
   aacgaacgat gttagattaa atgcttacag tagagtacaa gcagtggggg ttatatttgg    1140
   atggacatta tctgttcacg tgacccagta tgagtgaaaa gttcgccaag tcaaccactg    1200
   tattacagac ggcaattata ttacaattag tggcaaccct tcgtttgtgc ggatccccca    1260
   cacaccatag cttcaaaatg tttctactcc ttttttactc ttccagattt tctcggactc     1320
   cgcgcatcgc cgtaccactt caaaacaccc aagcacagca tactaaattt tccctctttc    1380
   ttcctctagg gtgtcgttaa ttacccgtac taaaggtttg gaaaagaaaa aagagaccgc    1440
   ctcgtttctt tttcttcgtc gaaaaaggca ataaaaattt ttatcacgtt tctttttctt    1500
   gaaatttttt tttttagttt ttttctcttt cagtgacctc cattgatatt taagttaata    1560
   aacggtcttc aatttctcaa gtttcagttt catttttctt gttctattac aacttttttt      1620
   acttcttgtt cattagaaag aaagcatagc aatctaatct aagggcggtg ttgacaatta      1680
   atcatcggca tagtatatcg gcatagtata atacgacaag gtgaggaact aaaccatggc      1740
   caagttgacc agtgccgttc cggtgctcac cgcgcgcgac gtcgccggag cggtcgagtt      1800
   ctggaccgac cggctcgggt tctcccggga cttcgtggag gacgacttcg ccggtgtggt      1860
   ccgggacgac gtgaccctgt tcatcagcgc ggtccaggac caggtggtgc cggacaacac      1920
   cctggcctgg gtgtgggtgc gcggcctgga cgagctgtac gccgagtggt cggaggtcgt      1980
   gtccacgaac ttccgggacg cctccgggcc ggccatgacc gagatcggcg agcagccgtg      2040
   ggggcgggag ttcgccctgc gcgacccggc cggcaactgc gtgcacttcg tggccgagga      2100
   gcaggactga cacgtccgac ggcggcccac gggtcccagg cctcggagat ccgtccccct      2160
   tttcctttgt cgatatcatg taattagtta tgtcacgctt acattcacgc cctcccccca      2220
   catccgctct aaccgaaaag gaaggagtta gacaacctga agtctaggtc cctatttatt      2280
   tttttatagt tatgttagta ttaagaacgt tatttatatt tcaaattttt cttttttttc      2340
   tgtacagacg cgtgtacgca tgtaacatta tactgaaaac cttgcttgag aaggttttgg      2400
   gacgctcgaa ggctttaatt tgcaagctgg agaccaacat gtgagcaaaa ggccagcaaa      2460
   aggccaggaa ccgtaaaaag gccgcgttgc tggcgttttt ccataggctc cgcccccctg      2520
   acgagcatca caaaaatcga cgctcaagtc agaggtggcg aaacccgaca ggactataaa      2580
   gataccaggc gtttccccct ggaagctccc tcgtgcgctc tcctgttccg accctgccgc      2640
   ttaccggata cctgtccgcc tttctccctt cgggaagcgt ggcgctttct caatgctcac      2700
   gctgtaggta tctcagttcg gtgtaggtcg ttcgctccaa gctgggctgt gtgcacgaac      2760
   cccccgttca gcccgaccgc tgcgccttat ccggtaacta tcgtcttgag tccaacccgg      2820
   taagacacga cttatcgcca ctggcagcag ccactggtaa caggattagc agagcgaggt      2880
   atgtaggcgg tgctacagag ttcttgaagt ggtggcctaa ctacggctac actagaagga      2940
   cagtatttgg tatctgcgct ctgctgaagc cagttacctt cggaaaaaga gttggtagct      3000
   cttgatccgg caaacaaacc accgctggta gcggtggttt ttttgtttgc aagcagcaga      3060
   ttacgcgcag aaaaaaagga tctcaagaag atcctttgat cttttctacg gggtctgacg      3120
   ctcagtggaa cgaaaactca cgttaaggga ttttggtcat gcatgagatc                 3170
<210> 10
   <211> 29
   <212> DNA
   <213> Artificial
<220>
   <223> A primer with a symbol pGsTD50_forward used to obtain a PCR product with a
   symbol H3.2
<400> 10
   aaaagatctt accacaattc aagacggcc 29
<210> 11
   <211> 48
   <212> DNA
   <213> Artificial
<220>
   <223> A primer with a symbol pGsTD50_reverse used to obtain a PCR product with a
   symbol H3.2
<400> 11
   gggttcgaag tttaattatt aaatattctt actagttaat tatattca 48
<210> 12
   <211> 518
   <212> DNA
   <213> Artificial
<220>
   <223> A PCR product with a symbol H3.2 used to obtain a plasmid expression vector
   with a symbol pH3
<400> 12
   aaaagatctt accacaattc aagacggccc ttatgtgcct gttctccaac gtgtccttat      60
   cagttcataa tcgccacctg gttgtgtctc aatcgccaga tttaccggtt ctcctaaatt     120
   gcgcctacat ttgtcgttct cacgtcctgt gctctctttc tacatggacg agttgctgcc     180
   aacgaccatt tttcacggta tcaagtggtg gttagcctcc cggcgaccct ggacggctcg     240
   cttccgttga tccttccata ctaattaacg gcctcggact tttgtagcgc gaaatcatga     300
   gaatcaccaa actcccggca actagctcgg tcgtgcccgg caccggggcc tctatgggat     360
   tcttcgcccc gggagtccag caataagaac atagtggaaa attataaata tgagtaattc     420
   gccgtgcaat cgacatggag tatattttaa aggttatctt attattcaat tgaatataat     480
   taactagtaa gaatatttaa taattaaact tcgaaccc                             518
<210> 13
   <211> 3193
   <212> DNA
   <213> Artificial
<220>
   <223> A plasmid expression vector with a symbol pH3
<400> 13
   agatcttacc acaattcaag acggccctta tgtgcctgtt ctccaacgtg tccttatcag      60
   ttcataatcg ccacctggtt gtgtctcaat cgccagattt accggttctc ctaaattgcg     120
   cctacatttg tcgttctcac gtcctgtgct ctctttctac atggacgagt tgctgccaac      180
   gaccattttt cacggtatca agtggtggtt agcctcccgg cgaccctgga cggctcgctt      240
   ccgttgatcc ttccatacta attaacggcc tcggactttt gtagcgcgaa atcatgagaa      300
   tcaccaaact cccggcaact agctcggtcg tgcccggcac cggggcctct atgggattct      360
   tcgccccggg agtccagcaa taagaacata gtggaaaatt ataaatatga gtaattcgcc      420
   gtgcaatcga catggagtat attttaaagg ttatcttatt attcaattga atataattaa      480
   ctagtaagaa tatttaataa ttaaacttcg aaacgatgag atttccttca atttttactg      540
   ctgttttatt cgcagcatcc tccgcattag ctgctccagt caacactaca acagaagatg      600
   aaacggcaca aattccggct gaagctgtca tcggttactc agatttagaa ggggatttcg      660
   atgttgctgt tttgccattt tccaacagca caaataacgg gttattgttt ataaatacta      720
   ctattgccag cattgctgct aaagaagaag gggtatctct cgagaaaaga gaggctgaag      780
   ctgcaggaat tcacgtggcc cagccggccg tctcggatcg gtacctcgag ccgcggcggc      840
   cgccagcttt ctagaacaaa aactcatctc agaagaggat ctgaatagcg ccgtcgacca      900
   tcatcatcat catcattgag ttttagcctt agacatgact gttcctcagt tcaagttggg      960
   cacttacgag aagaccggtc ttgctagatt ctaaaccatt tattagatgt tgaaaagttt     1020
   actcgtcgtt ttgtacaaac actgtttaat gggcctcgaa tttagctagt tacacattta     1080
   tctgtatata catgaataat attaacgaac gatgttagat taaatgctta cagtagagta     1140
   caagcagtgg gggttatatt tggatggaca ttatctgttc acgtgaccca gtatgagtga     1200
   aaagttcgcc aagtcaacca ctgtattaca gacggcaatt atattacaat tagtggcaac     1260
   ccttcgtttg tgcggatccc ccacacacca tagcttcaaa atgtttctac tcctttttta      1320
   ctcttccaga ttttctcgga ctccgcgcat cgccgtacca cttcaaaaca cccaagcaca     1380
   gcatactaaa ttttccctct ttcttcctct agggtgtcgt taattacccg tactaaaggt     1440
   ttggaaaaga aaaaagagac cgcctcgttt ctttttcttc gtcgaaaaag gcaataaaaa     1500
   tttttatcac gtttcttttt cttgaaattt ttttttttag tttttttctc tttcagtgac       1560
   ctccattgat atttaagtta ataaacggtc ttcaatttct caagtttcag tttcattttt     1620
   cttgttctat tacaactttt tttacttctt gttcattaga aagaaagcat agcaatctaa     1680
   tctaagggcg gtgttgacaa ttaatcatcg gcatagtata tcggcatagt ataatacgac     1740
   aaggtgagga actaaaccat ggccaagttg accagtgccg ttccggtgct caccgcgcgc     1800
   gacgtcgccg gagcggtcga gttctggacc gaccggctcg ggttctcccg ggacttcgtg     1860
   gaggacgact tcgccggtgt ggtccgggac gacgtgaccc tgttcatcag cgcggtccag     1920
   gaccaggtgg tgccggacaa caccctggcc tgggtgtggg tgcgcggcct ggacgagctg     1980
   tacgccgagt ggtcggaggt cgtgtccacg aacttccggg acgcctccgg gccggccatg     2040
   accgagatcg gcgagcagcc gtgggggcgg gagttcgccc tgcgcgaccc ggccggcaac     2100
   tgcgtgcact tcgtggccga ggagcaggac tgacacgtcc gacggcggcc cacgggtccc     2160
   aggcctcgga gatccgtccc ccttttcctt tgtcgatatc atgtaattag ttatgtcacg     2220
   cttacattca cgccctcccc ccacatccgc tctaaccgaa aaggaaggag ttagacaacc     2280
   tgaagtctag gtccctattt atttttttat agttatgtta gtattaagaa cgttatttat     2340
   atttcaaatt tttctttttt ttctgtacag acgcgtgtac gcatgtaaca ttatactgaa      2400
   aaccttgctt gagaaggttt tgggacgctc gaaggcttta atttgcaagc tggagaccaa     2460
   catgtgagca aaaggccagc aaaaggccag gaaccgtaaa aaggccgcgt tgctggcgtt     2520
   tttccatagg ctccgccccc ctgacgagca tcacaaaaat cgacgctcaa gtcagaggtg     2580
   gcgaaacccg acaggactat aaagatacca ggcgtttccc cctggaagct ccctcgtgcg     2640
   ctctcctgtt ccgaccctgc cgcttaccgg atacctgtcc gcctttctcc cttcgggaag     2700
   cgtggcgctt tctcaatgct cacgctgtag gtatctcagt tcggtgtagg tcgttcgctc     2760
   caagctgggc tgtgtgcacg aaccccccgt tcagcccgac cgctgcgcct tatccggtaa     2820
   ctatcgtctt gagtccaacc cggtaagaca cgacttatcg ccactggcag cagccactgg     2880
   taacaggatt agcagagcga ggtatgtagg cggtgctaca gagttcttga agtggtggcc 2940
   taactacggc tacactagaa ggacagtatt tggtatctgc gctctgctga agccagttac 3000
   cttcggaaaa agagttggta gctcttgatc cggcaaacaa accaccgctg gtagcggtgg 3060
   tttttttgtt tgcaagcagc agattacgcg cagaaaaaaa ggatctcaag aagatccttt 3120
   gatcttttct acggggtctg acgctcagtg gaacgaaaac tcacgttaag ggattttggt 3180
   catgcatgag atc 3193
<210> 14
   <211> 41
   <212> DNA
   <213> Artificial
<220>
   <223> A primer with a symbol pTEF1pH4_forward used to obtain a PCR product with a
   symbol H4
<400> 14 tgcggatcca cttctcttct tactttcact cctttcccta c 41
<210> 15
   <211> 58
   <212> DNA
   <213> Artificial
<220>
   <223> A primer with a symbol pTEF1pH4_reverse used to obtain a PCR product with a
   symbol H4
<400> 15 atgattaatt gtcaacaccg ccttttaatt aagtttagtt tagatgaagt aaaagaag 58
<210> 16
   <211> 370
   <212> DNA
   <213> Artificial
<220>
   <223> A-product PCR with a symbol H4 used to obtain a plasmid expression vector
   with a symbol pH4
<400> 16
   tgcggatcca cttctcttct tactttcact cctttcccta cacggttatt gtacgtgtct 60
   attccggtat ttaaattagc tatgctgaaa attacgacag aatgttttta ttgccgcgtg 120
   tattttcaat tatatagtag tatttaagca tcgtcgacct gcacaaaacc ctcatcgtac 180
   catattttac ccatactagt tgaaaatttt tttttttttt gctctgcggg tgagggggcg 240
   ttgcccaata tataaggagt ggcccttgtc ttagacaaaa attttccagg ttataagttt 300
   tttttttttc ttcttctttt acttcatcta aactaaactt aattaaaagg cggtgttgac 360
   aattaatcat 370
<210> 17
   <211> 3125
   <212> DNA
   <213> Artificial
<220>
   <223> A plasmid expression vector with a symbol pH4
<400> 17
   gatccacttc tcttcttact ttcactcctt tccctacacg gttattgtac gtgtctattc      60
   cggtatttaa attagctatg ctgaaaatta cgacagaatg tttttattgc cgcgtgtatt     120
   ttcaattata tagtagtatt taagcatcgt cgacctgcac aaaaccctca tcgtaccata     180
   ttttacccat actagttgaa aatttttttt ttttttgctc tgcgggtgag ggggcgttgc      240
   ccaatatata aggagtggcc cttgtcttag acaaaaattt tccaggttat aagttttttt     300
   tttttcttct tcttttactt catctaaact aaacttaatt aaaaggcggt gttgacaatt     360
   aatcatcggc atagtatatc ggcatagtat aatacgacaa ggtgaggaac taaaccatgg     420
   ccaagttgac cagtgccgtt ccggtgctca ccgcgcgcga cgtcgccgga gcggtcgagt     480
   tctggaccga ccggctcggg ttctcccggg acttcgtgga ggacgacttc gccggtgtgg     540
   tccgggacga cgtgaccctg ttcatcagcg cggtccagga ccaggtggtg ccggacaaca     600
   ccctggcctg ggtgtgggtg cgcggcctgg acgagctgta cgccgagtgg tcggaggtcg     660
   tgtccacgaa cttccgggac gcctccgggc cggccatgac cgagatcggc gagcagccgt     720
   gggggcggga gttcgccctg cgcgacccgg ccggcaactg cgtgcacttc gtggccgagg     780
   agcaggactg acacgtccga cggcggccca cgggtcccag gcctcggaga tccgtccccc     840
   ttttcctttg tcgatatcat gtaattagtt atgtcacgct tacattcacg ccctcccccc     900
   acatccgctc taaccgaaaa ggaaggagtt agacaacctg aagtctaggt ccctatttat     960
   ttttttatag ttatgttagt attaagaacg ttatttatat ttcaaatttt tctttttttt     1020
   ctgtacagac gcgtgtacgc atgtaacatt atactgaaaa ccttgcttga gaaggttttg    1080
   ggacgctcga aggctttaat ttgcaagctg gagaccaaca tgtgagcaaa aggccagcaa    1140
   aaggccagga accgtaaaaa ggccgcgttg ctggcgtttt tccataggct ccgcccccct     1200
   gacgagcatc acaaaaatcg acgctcaagt cagaggtggc gaaacccgac aggactataa    1260
   agataccagg cgtttccccc tggaagctcc ctcgtgcgct ctcctgttcc gaccctgccg    1320
   cttaccggat acctgtccgc ctttctccct tcgggaagcg tggcgctttc tcaatgctca    1380
   cgctgtaggt atctcagttc ggtgtaggtc gttcgctcca agctgggctg tgtgcacgaa    1440
   ccccccgttc agcccgaccg ctgcgcctta tccggtaact atcgtcttga gtccaacccg     1500
   gtaagacacg acttatcgcc actggcagca gccactggta acaggattag cagagcgagg 1560
   tatgtaggcg gtgctacaga gttcttgaag tggtggccta actacggcta cactagaagg 1620
   acagtatttg gtatctgcgc tctgctgaag ccagttacct tcggaaaaag agttggtagc 1680
   tcttgatccg gcaaacaaac caccgctggt agcggtggtt tttttgtttg caagcagcag 1740
   attacgcgca gaaaaaaagg atctcaagaa gatcctttga tcttttctac ggggtctgac 1800
   gctcagtgga acgaaaactc acgttaaggg attttggtca tgcatgagat cagatcttac 1860
   cacaattcaa gacggccctt atgtgcctgt tctccaacgt gtccttatca gttcataatc 1920
   gccacctggt tgtgtctcaa tcgccagatt taccggttct cctaaattgc gcctacattt 1980
   gtcgttctca cgtcctgtgc tctctttcta catggacgag ttgctgccaa cgaccatttt 2040
   tcacggtatc aagtggtggt tagcctcccg gcgaccctgg acggctcgct tccgttgatc 2100
   cttccatact aattaacggc ctcggacttt tgtagcgcga aatcatgaga atcaccaaac 2160
   tcccggcaac tagctcggtc gtgcccggca ccggggcctc tatgggattc ttcgccccgg 2220
   gagtccagca ataagaacat agtggaaaat tataaatatg agtaattcgc cgtgcaatcg 2280
   acatggagta tattttaaag gttatcttat tattcaattg aatataatta actagtaaga 2340
   atatttaata attaaacttc gaaacgatga gatttccttc aatttttact gctgttttat 2400
   tcgcagcatc ctccgcatta gctgctccag tcaacactac aacagaagat gaaacggcac 2460
   aaattccggc tgaagctgtc atcggttact cagatttaga aggggatttc gatgttgctg 2520
   ttttgccatt ttccaacagc acaaataacg ggttattgtt tataaatact actattgcca 2580
   gcattgctgc taaagaagaa ggggtatctc tcgagaaaag agaggctgaa gctgcaggaa 2640
   ttcacgtggc ccagccggcc gtctcggatc ggtacctcga gccgcggcgg ccgccagctt 2700
   tctagaacaa aaactcatct cagaagagga tctgaatagc gccgtcgacc atcatcatca 2760
   tcatcattga gttttagcct tagacatgac tgttcctcag ttcaagttgg gcacttacga 2820
   gaagaccggt cttgctagat tctaaaccat ttattagatg ttgaaaagtt tactcgtcgt 2880
   tttgtacaaa cactgtttaa tgggcctcga atttagctag ttacacattt atctgtatat 2940
   acatgaataa tattaacgaa cgatgttaga ttaaatgctt acagtagagt acaagcagtg 3000
   ggggttatat ttggatggac attatctgtt cacgtgaccc agtatgagtg aaaagttcgc 3060
   caagtcaacc actgtattac agacggcaat tatattacaa ttagtggcaa cccttcgttt 3120
   gtgcg 3125
<210> 18
   <211> 37
   <212> DNA
   <213> Artificial
<220>
   <223> A primer with a symbol BleoRCpGfree_For used to obtain a PCR product with a
   symbol H4.1
<400> 18
   ctaaaccatg gccaagttga ccagtgctgt cccagtg 37
<210> 19
   <211> 38
   <212> DNA
   <213> Artificial
<220>
   <223> A primer with a symbol BleoRCpGfree_Rev used to obtain a PCR product with a
   symbol H4.1
<400> 19 tttacgtgtc agtcctgctc ctctgccaca aagtgcac 38
<210> 20
   <211> 390
   <212> DNA
   <213> Artificial
<220>
   <223> A PCR product with a symbol H4.1 used to obtain a plasmid with a symbol pH4A
<400> 20
   ctaaaccatg gccaagttga ccagtgctgt cccagtgctc acagccaggg atgtggctgg      60
   agctgttgag ttctggactg acaggttggg gttctccaga gattttgtgg aggatgactt     120
   tgcaggtgtg gtcagagatg atgtcaccct gttcatctca gcagtccagg accaggtggt     180
   gcctgacaac accctggctt gggtgtgggt gagaggactg gatgagctgt atgctgagtg     240
   gagtgaggtg gtctccacca acttcaggga tgccagtggc cctgccatga cagagattgg     300
   agagcagccc tgggggagag agtttgccct gagagaccca gcaggcaact gtgtgcactt     360
   tgtggcagag gagcaggact gacacgtaaa                                      390
<210> 21
   <211> 3125
   <212> DNA
   <213> Artificial
<220>
   <223> A plasmid with a symbol pH4A used to obtain a plasmid expression vector with
   a symbol pH5A
<400> 21
   catggccaag ttgaccagtg ctgtcccagt gctcacagcc agggatgtgg ctggagctgt      60
   tgagttctgg actgacaggt tggggttctc cagagatttt gtggaggatg actttgcagg     120
   tgtggtcaga gatgatgtca ccctgttcat ctcagcagtc caggaccagg tggtgcctga 180
   caacaccctg gcttgggtgt gggtgagagg actggatgag ctgtatgctg agtggagtga 240
   ggtggtctcc accaacttca gggatgccag tggccctgcc atgacagaga ttggagagca 300
   gccctggggg agagagtttg ccctgagaga cccagcaggc aactgtgtgc actttgtggc 360
   agaggagcag gactgacacg tccgacggcg gcccacgggt cccaggcctc ggagatccgt 420
   cccccttttc ctttgtcgat atcatgtaat tagttatgtc acgcttacat tcacgccctc 480
   cccccacatc cgctctaacc gaaaaggaag gagttagaca acctgaagtc taggtcccta 540
   tttatttttt tatagttatg ttagtattaa gaacgttatt tatatttcaa atttttcttt 600
   tttttctgta cagacgcgtg tacgcatgta acattatact gaaaaccttg cttgagaagg 660
   ttttgggacg ctcgaaggct ttaatttgca agctggagac caacatgtga gcaaaaggcc 720
   agcaaaaggc caggaaccgt aaaaaggccg cgttgctggc gtttttccat aggctccgcc 780
   cccctgacga gcatcacaaa aatcgacgct caagtcagag gtggcgaaac ccgacaggac 840
   tataaagata ccaggcgttt ccccctggaa gctccctcgt gcgctctcct gttccgaccc 900
   tgccgcttac cggatacctg tccgcctttc tcccttcggg aagcgtggcg ctttctcaat 960
   gctcacgctg taggtatctc agttcggtgt aggtcgttcg ctccaagctg ggctgtgtgc 1020
   acgaaccccc cgttcagccc gaccgctgcg ccttatccgg taactatcgt cttgagtcca 1080
   acccggtaag acacgactta tcgccactgg cagcagccac tggtaacagg attagcagag 1140
   cgaggtatgt aggcggtgct acagagttct tgaagtggtg gcctaactac ggctacacta 1200
   gaaggacagt atttggtatc tgcgctctgc tgaagccagt taccttcgga aaaagagttg 1260
   gtagctcttg atccggcaaa caaaccaccg ctggtagcgg tggttttttt gtttgcaagc 1320
   agcagattac gcgcagaaaa aaaggatctc aagaagatcc tttgatcttt tctacggggt 1380
   ctgacgctca gtggaacgaa aactcacgtt aagggatttt ggtcatgcat gagatcagat 1440
   cttaccacaa ttcaagacgg cccttatgtg cctgttctcc aacgtgtcct tatcagttca 1500
   taatcgccac ctggttgtgt ctcaatcgcc agatttaccg gttctcctaa attgcgccta 1560
   catttgtcgt tctcacgtcc tgtgctctct ttctacatgg acgagttgct gccaacgacc 1620
   atttttcacg gtatcaagtg gtggttagcc tcccggcgac cctggacggc tcgcttccgt 1680
   tgatccttcc atactaatta acggcctcgg acttttgtag cgcgaaatca tgagaatcac 1740
   caaactcccg gcaactagct cggtcgtgcc cggcaccggg gcctctatgg gattcttcgc 1800
   cccgggagtc cagcaataag aacatagtgg aaaattataa atatgagtaa ttcgccgtgc 1860
   aatcgacatg gagtatattt taaaggttat cttattattc aattgaatat aattaactag 1920
   taagaatatt taataattaa acttcgaaac gatgagattt ccttcaattt ttactgctgt 1980
   tttattcgca gcatcctccg cattagctgc tccagtcaac actacaacag aagatgaaac 2040
   ggcacaaatt ccggctgaag ctgtcatcgg ttactcagat ttagaagggg atttcgatgt 2100
   tgctgttttg ccattttcca acagcacaaa taacgggtta ttgtttataa atactactat 2160
   tgccagcatt gctgctaaag aagaaggggt atctctcgag aaaagagagg ctgaagctgc 2220
   aggaattcac gtggcccagc cggccgtctc ggatcggtac ctcgagccgc ggcggccgcc 2280
   agctttctag aacaaaaact catctcagaa gaggatctga atagcgccgt cgaccatcat 2340
   catcatcatc attgagtttt agccttagac atgactgttc ctcagttcaa gttgggcact 2400
   tacgagaaga ccggtcttgc tagattctaa accatttatt agatgttgaa aagtttactc 2460
   gtcgttttgt acaaacactg tttaatgggc ctcgaattta gctagttaca catttatctg 2520
   tatatacatg aataatatta acgaacgatg ttagattaaa tgcttacagt agagtacaag 2580
   cagtgggggt tatatttgga tggacattat ctgttcacgt gacccagtat gagtgaaaag 2640
   ttcgccaagt caaccactgt attacagacg gcaattatat tacaattagt ggcaaccctt 2700
   cgtttgtgcg gatccacttc tcttcttact ttcactcctt tccctacacg gttattgtac 2760
   gtgtctattc cggtatttaa attagctatg ctgaaaatta cgacagaatg tttttattgc 2820
   cgcgtgtatt ttcaattata tagtagtatt taagcatcgt cgacctgcac aaaaccctca 2880
   tcgtaccata ttttacccat actagttgaa aatttttttt ttttttgctc tgcgggtgag 2940
   ggggcgttgc ccaatatata aggagtggcc cttgtcttag acaaaaattt tccaggttat 3000
   aagttttttt tttttcttct tcttttactt catctaaact aaacttaatt aaaaggcggt 3060
   gttgacaatt aatcatcggc atagtatatc ggcatagtat aatacgacaa ggtgaggaac 3120
   taaac 3125
<210> 22
   <211> 39
   <212> DNA
   <213> Artificial
<220>
   <223> A primer with a symbol CYC1G12_For used to obtain a PCR product with a symbol
   H5.1
<400> 22 gtcgatgata tcatttagaa cactctagat aagcaaaag 39
<210> 23
   <211> 36
   <212> DNA
   <213> Artificial
<220>
   <223> A primer with a symbol CYC1G12_Rev used to obtain a PCR product with a symbol
   H5.1
<400> 23 ttgctcacat gtattataat gtaagcgaag tttgac 36
<210> 24
   <211> 588
   <212> DNA
   <213> Artificial
<220>
   <223> A PCR product with a symbol H5.1 used to obtain a plasmid expression vector
   with a symbol pH5A
<400> 24
   gtcgatgata tcatttagaa cactctagat aagcaaaagc aaaataaaag cgaaatgctc 60
   tgtatataat aatttatgtt ttatttaatg atagagtgta gagagtgaga gagattgtaa 120
   gcccccagaa aatgaagaaa tatatttatt gatgaagatg agaatacttt tatgagaccc 180
   tccataggtt gctttatgca agggagatgc agttttgcat gagatcgaga ttacttatcc 240
   tagaagttga atttataata ggcgtattag aggtagaaaa tagcactttt ttaagaataa 300
   cttgaataat ctccattaat caaaaacaca aacattaact aagcgtgtca tattttactt 360
   ttttctataa tcatctctat cgcctttttc ttttttttct cagattctat ttctcaaaca 420
   cactctctct gactctcact ctcactttca ctctttgact tgtttcatct gaaaaatcct 480
   gcatatgtgc aactagtgtt tcgatatatc gatctgtacc ttcttgacta accgctcttt 540
   cacttctctt tcgtcaaact tcgcttacat tataatacat gtgagcaa 588
<210> 25
   <211> 3430
   <212> DNA
   <213> Artificial
<220>
   <223> A plasmid expression vector with a symbol pH5A
<400> 25
   atcatttaga acactctaga taagcaaaag caaaataaaa gcgaaatgct ctgtatataa      60
   taatttatgt tttatttaat gatagagtgt agagagtgag agagattgta agcccccaga     120
   aaatgaagaa atatatttat tgatgaagat gagaatactt ttatgagacc ctccataggt     180
   tgctttatgc aagggagatg cagttttgca tgagatcgag attacttatc ctagaagttg     240
   aatttataat aggcgtatta gaggtagaaa atagcacttt tttaagaata acttgaataa     300
   tctccattaa tcaaaaacac aaacattaac taagcgtgtc atattttact tttttctata     360
   atcatctcta tcgccttttt cttttttttc tcagattcta tttctcaaac acactctctc     420
   tgactctcac tctcactttc actctttgac ttgtttcatc tgaaaaatcc tgcatatgtg     480
   caactagtgt ttcgatatat cgatctgtac cttcttgact aaccgctctt tcacttctct     540
   ttcgtcaaac ttcgcttaca ttataataca tgtgagcaaa aggccagcaa aaggccagga     600
   accgtaaaaa ggccgcgttg ctggcgtttt tccataggct ccgcccccct gacgagcatc     660
   acaaaaatcg acgctcaagt cagaggtggc gaaacccgac aggactataa agataccagg     720
   cgtttccccc tggaagctcc ctcgtgcgct ctcctgttcc gaccctgccg cttaccggat     780
   acctgtccgc ctttctccct tcgggaagcg tggcgctttc tcaatgctca cgctgtaggt     840
   atctcagttc ggtgtaggtc gttcgctcca agctgggctg tgtgcacgaa ccccccgttc     900
   agcccgaccg ctgcgcctta tccggtaact atcgtcttga gtccaacccg gtaagacacg     960
   acttatcgcc actggcagca gccactggta acaggattag cagagcgagg tatgtaggcg    1020
   gtgctacaga gttcttgaag tggtggccta actacggcta cactagaagg acagtatttg    1080
   gtatctgcgc tctgctgaag ccagttacct tcggaaaaag agttggtagc tcttgatccg    1140
   gcaaacaaac caccgctggt agcggtggtt tttttgtttg caagcagcag attacgcgca    1200
   gaaaaaaagg atctcaagaa gatcctttga tcttttctac ggggtctgac gctcagtgga    1260
   acgaaaactc acgttaaggg attttggtca tgcatgagat cagatcttac cacaattcaa    1320
   gacggccctt atgtgcctgt tctccaacgt gtccttatca gttcataatc gccacctggt    1380
   tgtgtctcaa tcgccagatt taccggttct cctaaattgc gcctacattt gtcgttctca 1440
   cgtcctgtgc tctctttcta catggacgag ttgctgccaa cgaccatttt tcacggtatc 1500
   aagtggtggt tagcctcccg gcgaccctgg acggctcgct tccgttgatc cttccatact 1560
   aattaacggc ctcggacttt tgtagcgcga aatcatgaga atcaccaaac tcccggcaac 1620
   tagctcggtc gtgcccggca ccggggcctc tatgggattc ttcgccccgg gagtccagca 1680
   ataagaacat agtggaaaat tataaatatg agtaattcgc cgtgcaatcg acatggagta 1740
   tattttaaag gttatcttat tattcaattg aatataatta actagtaaga atatttaata 1800
   attaaacttc gaaacgatga gatttccttc aatttttact gctgttttat tcgcagcatc 1860
   ctccgcatta gctgctccag tcaacactac aacagaagat gaaacggcac aaattccggc 1920
   tgaagctgtc atcggttact cagatttaga aggggatttc gatgttgctg ttttgccatt 1980
   ttccaacagc acaaataacg ggttattgtt tataaatact actattgcca gcattgctgc 2040
   taaagaagaa ggggtatctc tcgagaaaag agaggctgaa gctgcaggaa ttcacgtggc 2100
   ccagccggcc gtctcggatc ggtacctcga gccgcggcgg ccgccagctt tctagaacaa 2160
   aaactcatct cagaagagga tctgaatagc gccgtcgacc atcatcatca tcatcattga 2220
   gttttagcct tagacatgac tgttcctcag ttcaagttgg gcacttacga gaagaccggt 2280
   cttgctagat tctaaaccat ttattagatg ttgaaaagtt tactcgtcgt tttgtacaaa 2340
   cactgtttaa tggcctcgga atttagctag ttacacattt atctgtatat acatgaataa 2400
   tattaacgaa cgatgttaga ttaaatgctt acagtagagt acaagcagtg ggggttatat 2460
   ttggatggac attatctgtt cacgtgaccc agtatgagtg aaaagttcgc caagtcaacc 2520
   actgtattac agacggcaat tatattacaa ttagtggcaa cccttcgttt gtgcggatcc 2580
   acttctcttc ttactttcac tcctttccct acacggttat tgtacgtgtc tattccggta 2640
   tttaaattag ctatgctgaa aattacgaca gaatgttttt attgccgcgt gtattttcaa 2700
   ttatatagta gtatttaagc atcgtcgacc tgcacaaaac cctcatcgta ccatatttta 2760
   cccatactag ttgaaaattt tttttttttt tgctctgcgg gtgagggggc gttgcccaat 2820
   atataaggag tggcccttgt cttagacaaa aattttccag gttataagtt tttttttttt 2880
   cttcttcttt tacttcatct aaactaaact taattaaaag gcggtgttga caattaatca 2940
   tcggcatagt atatcggcat agtataatac gacaaggtga ggaactaaac catggccaag 3000
   ttgaccagtg ctgtcccagt gctcacagcc agggatgtgg ctggagctgt tgagttctgg 3060
   actgacaggt tggggttctc cagagatttt gtggaggatg actttgcagg tgtggtcaga 3120
   gatgatgtca ccctgttcat ctcagcagtc caggaccagg tggtgcctga caacaccctg 3180
   gcttgggtgt gggtgagagg actggatgag ctgtatgctg agtggagtga ggtggtctcc 3240
   accaacttca gggatgccag tggccctgcc atgacagaga ttggagagca gccctggggg 3300
   agagagtttg ccctgagaga cccagcaggc aactgtgtgc actttgtggc agaggagcag 3360
   gactgacacg tccgacggcg gcccacgggt cccaggcctc ggagatccgt cccccttttc 3420
   ctttgtcgat 3430
<210> 26
   <211> 39
   <212> DNA
   <213> Artificial
<220>
   <223> A primer with a symbol pIEF2pH6_forward used to obtain a PCR product with a
   symbol H6
<400> 26
   tgcggatccc actactcaga cttaccaccg catatacag 39
<210> 27
   <211> 50
   <212> DNA
   <213> Artificial
<220>
   <223> A primer with a symbol pIEF2pH6_reverse used to obtain a PCR product with a
   symbol H6
<400> 27
   atgattaatt gtcaactttg tttaatgtat aataatagta tactgtattg 50
<210> 28
   <211> 684
   <212> DNA
   <213> Artificial
<220>
   <223> A PCR product with a symbol H6 used to obtain a plasmid expression vector
   with a symbol pH6
<400> 28
   tgcggatccc actactcaga cttaccaccg catatacaga cattaaccat aatagtgcca    60
   tctacagggc acaatgcatt taatacacaa tctaatatac agcttaagta gagcccatct   120
   gtaattaaac ccacacaaag agtaattcag tcacatatac taatgtgaac ggcattcatt   180
   tgacctagtg tcaacatggc aaaagcttcc gccgcagttt cgcaatttca ctacatgtcg   240
   cctgtcatca gctacgaact gctatgtaag atcaactgca aaatgttcca ctggtccata   300
   atcgaagttt ctaggagttt gccaaaaaat ttagtcccat actatcccaa ccaaatgtcc   360
   tcaaataact accatcacac ctattttgcc aagttcttcc tatctactat atagctactt   420
   catgctgctt gcaataccgt agtctacggc catagcactt ggattctgaa tacttagagg   480
   aaatagggta aagccctatc cgggtaaatt aaatatttgt cctgctgaaa aaaaaaattt   540
   cgccacgaca ccctctgatg aatggaacgc aattggctct gcaaaaattt ataaggaatt   600
   aaattgaaga acttcttttt ccttctcttt ttttcaatac agtatactat tattatacat   660
   taaacaaagt tgacaattaa tcat                                          684
<210> 29
   <211> 3439
   <212> DNA
   <213> Artificial
<220>
   <223> A plasmid expression vector with a symbol pH6
<400> 29
   agatcttacc acaattcaag acggccctta tgtgcctgtt ctccaacgtg tccttatcag 60
   ttcataatcg ccacctggtt gtgtctcaat cgccagattt accggttctc ctaaattgcg 120
   cctacatttg tcgttctcac gtcctgtgct ctctttctac atggacgagt tgctgccaac 180
   gaccattttt cacggtatca agtggtggtt agcctcccgg cgaccctgga cggctcgctt 240
   ccgttgatcc ttccatacta attaacggcc tcggactttt gtagcgcgaa atcatgagaa 300
   tcaccaaact cccggcaact agctcggtcg tgcccggcac cggggcctct atgggattct 360
   tcgccccggg agtccagcaa taagaacata gtggaaaatt ataaatatga gtaattcgcc 420
   gtgcaatcga catggagtat attttaaagg ttatcttatt attcaattga atataattaa 480
   ctagtaagaa tatttaataa ttaaacttcg aaacgatgag atttccttca atttttactg 540
   ctgttttatt cgcagcatcc tccgcattag ctgctccagt caacactaca acagaagatg 600
   aaacggcaca aattccggct gaagctgtca tcggttactc agatttagaa ggggatttcg 660
   atgttgctgt tttgccattt tccaacagca caaataacgg gttattgttt ataaatacta 720
   ctattgccag cattgctgct aaagaagaag gggtatctct cgagaaaaga gaggctgaag 780
   ctgcaggaat tcacgtggcc cagccggccg tctcggatcg gtacctcgag ccgcggcggc 840
   cgccagcttt ctagaacaaa aactcatctc agaagaggat ctgaatagcg ccgtcgacca 900
   tcatcatcat catcattgag ttttagcctt agacatgact gttcctcagt tcaagttggg 960
   cacttacgag aagaccggtc ttgctagatt ctaaaccatt tattagatgt tgaaaagttt 1020
   actcgtcgtt ttgtacaaac actgtttaat gggcctcgaa tttagctagt tacacattta 1080
   tctgtatata catgaataat attaacgaac gatgttagat taaatgctta cagtagagta 1140
   caagcagtgg gggttatatt tggatggaca ttatctgttc acgtgaccca gtatgagtga 1200
   aaagttcgcc aagtcaacca ctgtattaca gacggcaatt atattacaat tagtggcaac 1260
   ccttcgtttg tgcggatccc actactcaga cttaccaccg catatacaga cattaaccat 1320
   aatagtgcca tctacagggc acaatgcatt taatacacaa tctaatatac agcttaagta 1380
   gagcccatct gtaattaaac ccacacaaag agtaattcag tcacatatac taatgtgaac 1440
   ggcattcatt tgacctagtg tcaacatggc aaaagcttcc gccgcagttt cgcaatttca 1500
   ctacatgtcg cctgtcatca gctacgaact gctatgtaag atcaactgca aaatgttcca 1560
   ctggtccata atcgaagttt ctaggagttt gccaaaaaat ttagtcccat actatcccaa 1620
   ccaaatgtcc tcaaataact accatcacac ctattttgcc aagttcttcc tatctactat 1680
   atagctactt catgctgctt gcaataccgt agtctacggc catagcactt ggattctgaa 1740
   tacttagagg aaatagggta aagccctatc cgggtaaatt aaatatttgt cctgctgaaa 1800
   aaaaaaattt cgccacgaca ccctctgatg aatggaacgc aattggctct gcaaaaattt 1860
   ataaggaatt aaattgaaga acttcttttt ccttctcttt ttttcaatac agtatactat 1920
   tattatacat taaacaaagt tgacaattaa tcatcggcat agtatatcgg catagtataa 1980
   tacgacaagg tgaggaacta aaccatggcc aagttgacca gtgccgttcc ggtgctcacc 2040
   gcgcgcgacg tcgccggagc ggtcgagttc tggaccgacc ggctcgggtt ctcccgggac 2100
   ttcgtggagg acgacttcgc cggtgtggtc cgggacgacg tgaccctgtt catcagcgcg     2160
   gtccaggacc aggtggtgcc ggacaacacc ctggcctggg tgtgggtgcg cggcctggac     2220
   gagctgtacg ccgagtggtc ggaggtcgtg tccacgaact tccgggacgc ctccgggccg     2280
   gccatgaccg agatcggcga gcagccgtgg gggcgggagt tcgccctgcg cgacccggcc     2340
   ggcaactgcg tgcacttcgt ggccgaggag caggactgac acgtccgacg gcggcccacg     2400
   ggtcccaggc ctcggagatc cgtccccctt ttcctttgtc gatatcatgt aattagttat     2460
   gtcacgctta cattcacgcc ctccccccac atccgctcta accgaaaagg aaggagttag     2520
   acaacctgaa gtctaggtcc ctatttattt ttttatagtt atgttagtat taagaacgtt     2580
   atttatattt caaatttttc ttttttttct gtacagacgc gtgtacgcat gtaacattat     2640
   actgaaaacc ttgcttgaga aggttttggg acgctcgaag gctttaattt gcaagctgga     2700
   gaccaacatg tgagcaaaag gccagcaaaa ggccaggaac cgtaaaaagg ccgcgttgct     2760
   ggcgtttttc cataggctcc gcccccctga cgagcatcac aaaaatcgac gctcaagtca     2820
   gaggtggcga aacccgacag gactataaag ataccaggcg tttccccctg gaagctccct     2880
   cgtgcgctct cctgttccga ccctgccgct taccggatac ctgtccgcct ttctcccttc     2940
   gggaagcgtg gcgctttctc aatgctcacg ctgtaggtat ctcagttcgg tgtaggtcgt     3000
   tcgctccaag ctgggctgtg tgcacgaacc ccccgttcag cccgaccgct gcgccttatc     3060
   cggtaactat cgtcttgagt ccaacccggt aagacacgac ttatcgccac tggcagcagc     3120
   cactggtaac aggattagca gagcgaggta tgtaggcggt gctacagagt tcttgaagtg     3180
   gtggcctaac tacggctaca ctagaaggac agtatttggt atctgcgctc tgctgaagcc     3240
   agttaccttc ggaaaaagag ttggtagctc ttgatccggc aaacaaacca ccgctggtag     3300
   cggtggtttt tttgtttgca agcagcagat tacgcgcaga aaaaaaggat ctcaagaaga     3360
   tcctttgatc ttttctacgg ggtctgacgc tcagtggaac gaaaactcac gttaagggat     3420
   tttggtcatg catgagatc                                                  3439
<210> 30
   <211> 5310
   <212> DNA
   <213> Artificial
<220>
   <223> An expression plasmid with a symbol pH3Bgal32d
<400> 30
   agatcttacc acaattcaag acggccctta tgtgcctgtt ctccaacgtg tccttatcag       60
   ttcataatcg ccacctggtt gtgtctcaat cgccagattt accggttctc ctaaattgcg      120
   cctacatttg tcgttctcac gtcctgtgct ctctttctac atggacgagt tgctgccaac      180
   gaccattttt cacggtatca agtggtggtt agcctcccgg cgaccctgga cggctcgctt      240
   ccgttgatcc ttccatacta attaacggcc tcggactttt gtagcgcgaa atcatgagaa      300
   tcaccaaact cccggcaact agctcggtcg tgcccggcac cggggcctct atgggattct      360
   tcgccccggg agtccagcaa taagaacata gtggaaaatt ataaatatga gtaattcgcc      420
   gtgcaatcga catggagtat attttaaagg ttatcttatt attcaattga atataattaa      480
   ctagtaagaa tatttaataa ttaaacttcg aaacgatgag atttccttca atttttactg      540
   ctgttttatt cgcagcatcc tccgcattag ctgctccagt caacactaca acagaagatg      600
   aaacggcaca aattccggct gaagctgtca tcggttactc agatttagaa ggggatttcg 660
   atgttgctgt tttgccattt tccaacagca caaataacgg gttattgttt ataaatacta 720
   ctattgccag cattgctgct aaagaagaag ggtatctctc cgagaaaaga atgcgggtga 780
   cccagaaact gaaccatggc tggatctttg ccgaaggtgc cgccgacccg gcgacaccgc 840
   tggcaggcga gacggtaacc ctgccgcata acgcagtcga tctgccgctg agctatttcg 900
   atgagaccag ctatcagcgc gccttcacct atcagcgcgt gatcgcgtgg gatgacgcgt 960
   ggcagggccg ccgggtgcag ctgcgctttg acggggcgat ggccgacaat gtggtctggg 1020
   tgaacggcgt gcaggtcgtg gcgcatcccg acggctacac ccccttcgtg gccgacctga 1080
   ccgaccatct gcgccccggc gacaatctgg tgacggtgcg gatcgacggg tcggaaaacc 1140
   ccgccatccc gccctttggc gcgcagatcg actatctgac ctatgcaggc atctatcgcg 1200
   atgtctggct gatggtgctg cccgagcggc acctgaccaa tgcccgcatc ctgacgcccg 1260
   atgcgctgtc ggacgcgaaa acggtcgtga tccgaccgga ggtcaccgct cccggcccgg 1320
   tccgcgcccg cctgctggac ggcgaccgcg agattgccgc gaccgaagga gagggcgagc 1380
   tgaccctggc gggcctgacc ggcctgtcgc tgtggtccac cgacaaccca cagctttaca 1440
   cggtggagct gaccctgccc gacagcggcg atgtgaccac gcatcgcttt ggtttccgca 1500
   ccgcggaatg gacaccgcag ggcttcctgc tgaatggcca gccgatgaag ctgcgcgggt 1560
   tgaaccggca tcaaagctgg gcgcatcagg gctatgccgc gggccgtcac gcgcaggaac 1620
   gcgacgccga gatcgtccgc catgatctgt gctgcaacat ggtgcgcaca tcgcattacc 1680
   cgcaaagcac gtggttcctg gaccgctgcg acgagatcgg gttgctggtg ttcgaggaga 1740
   tccccggctg gcagcatatc ggcgatcagg cctggcagga tcgcagcgtg gacaatgtgc 1800
   gcgccatgat cacccgcgac tggaaccacc caagcatcgt catctggggc gtgcgcatca 1860
   acgaaagccc ggacaaccac gatttctatg tccgtaccaa cgcgctggcg cgcgagctgg 1920
   acccgacgcg ggccatcggc ggcgtgcgct gcatcaccga cagcgagatg ctggaggatg 1980
   tctatacgat gaatgatttc atcctggacg aatccgagct gccgctgatc aaccggccgc 2040
   gcaccgcgct gcgtccgacc gaggaggtca cggggatcaa gaaaccggtg ccttatctgg 2100
   tcaccgaata taacggccac atgttcccga ccaaggcgca ggacccggag ctgcgccaga 2160
   tggagcatgt gatccgccac cttgaggtgc tgaacgccgc ccatggcgac ccggcgattt 2220
   cggggtgcat cggctggtgc atgttcgatt acaacacgca caaggatttc ggcgcgggcg 2280
   accgcatctg ccatcatggc gtcatggaca tctggcgaga gccgaaattc gcggcccatg 2340
   cctatggcag ccagaagccg ccctcggaag gcatcgtgat ggagccggtg accttctggg 2400
   cgcggggcga acgcaatatc ggcggtgtcc tgccgctgat cgtgctgacg aactgcgacg 2460
   aggtcgagtt cgaatgcgcg ggcgtcaccc gccgcgtagg tccggaccga gagcggttcc 2520
   cccatctgcc gcgcccgccc gtcatcatcg accaccgcca tatcagcgcc gaggaactgg 2580
   gccagtgggg gatgagctgg catccgggcc gcatcacagg ttggctgaac ggcgaacagg 2640
   tcgcgctgcg cgaatatgtc gccgacccgc tgcccaccac gttgcagatc gcgcccgacc 2700
   gcgacacgct gccggcggat ggggatatcg acctgcgggt gatgctgcgg gcgctggacc 2760
   aagtcggcaa ccggctgcct ttcctggacg cgggcatcgc ggtgacggtg gacggccccg 2820
   cgcgcctgat cgggcccgat ctgcgcatgc tgcagggggg cacgacgggc atgttgctgc 2880
   gtctgacggg cgatgccggg accatccgca tcaccgcccg ccatccgcag tttccggagg 2940
   cggtggccac ggtcaccgtc ggctagtcta gaacaaaaac tcatctcaga agaggatctg 3000
   aatagcgccg tcgaccatca tcatcatcat cattgagttt tagccttaga catgactgtt 3060
   cctcagttca agttgggcac ttacgagaag accggtcttg ctagattcta aaccatttat 3120
   tagatgttga aaagtttact cgtcgttttg tacaaacact gtttaatggg cctcgaattt 3180
   agctagttac acatttatct gtatatacat gaataatatt aacgaacgat gttagattaa 3240
   atgcttacag tagagtacaa gcagtggggg ttatatttgg atggacatta tctgttcacg 3300
   tgacccagta tgagtgaaaa gttcgccaag tcaaccactg tattacagac ggcaattata 3360
   ttacaattag tggcaaccct tcgtttgtgc ggatccccca cacaccatag cttcaaaatg      3420
   tttctactcc ttttttactc ttccagattt tctcggactc cgcgcatcgc cgtaccactt     3480
   caaaacaccc aagcacagca tactaaattt tccctctttc ttcctctagg gtgtcgttaa      3540
   ttacccgtac taaaggtttg gaaaagaaaa aagagaccgc ctcgtttctt tttcttcgtc      3600
   gaaaaaggca ataaaaattt ttatcacgtt tctttttctt gaaatttttt tttttagttt      3660
   ttttctcttt cagtgacctc cattgatatt taagttaata aacggtcttc aatttctcaa      3720
   gtttcagttt catttttctt gttctattac aacttttttt acttcttgtt cattagaaag      3780
   aaagcatagc aatctaatct aagggcggtg ttgacaatta atcatcggca tagtatatcg      3840
   gcatagtata atacgacaag gtgaggaact aaaccatggc caagttgacc agtgccgttc      3900
   cggtgctcac cgcgcgcgac gtcgccggag cggtcgagtt ctggaccgac cggctcgggt      3960
   tctcccggga cttcgtggag gacgacttcg ccggtgtggt ccgggacgac gtgaccctgt      4020
   tcatcagcgc ggtccaggac caggtggtgc cggacaacac cctggcctgg gtgtgggtgc      4080
   gcggcctgga cgagctgtac gccgagtggt cggaggtcgt gtccacgaac ttccgggacg      4140
   cctccgggcc ggccatgacc gagatcggcg agcagccgtg ggggcgggag ttcgccctgc      4200
   gcgacccggc cggcaactgc gtgcacttcg tggccgagga gcaggactga cacgtccgac      4260
   ggcggcccac gggtcccagg cctcggagat ccgtccccct tttcctttgt cgatatcatg      4320
   taattagtta tgtcacgctt acattcacgc cctcccccca catccgctct aaccgaaaag      4380
   gaaggagtta gacaacctga agtctaggtc cctatttatt tttttatagt tatgttagta      4440
   ttaagaacgt tatttatatt tcaaattttt cttttttttc tgtacagacg cgtgtacgca      4500
   tgtaacatta tactgaaaac cttgcttgag aaggttttgg gacgctcgaa ggctttaatt      4560
   tgcaagctgg agaccaacat gtgagcaaaa ggccagcaaa aggccaggaa ccgtaaaaag      4620
   gccgcgttgc tggcgttttt ccataggctc cgcccccctg acgagcatca caaaaatcga      4680
   cgctcaagtc agaggtggcg aaacccgaca ggactataaa gataccaggc gtttccccct      4740
   ggaagctccc tcgtgcgctc tcctgttccg accctgccgc ttaccggata cctgtccgcc      4800
   tttctccctt cgggaagcgt ggcgctttct caatgctcac gctgtaggta tctcagttcg      4860
   gtgtaggtcg ttcgctccaa gctgggctgt gtgcacgaac cccccgttca gcccgaccgc      4920
   tgcgccttat ccggtaacta tcgtcttgag tccaacccgg taagacacga cttatcgcca      4980
   ctggcagcag ccactggtaa caggattagc agagcgaggt atgtaggcgg tgctacagag      5040
   ttcttgaagt ggtggcctaa ctacggctac actagaagga cagtatttgg tatctgcgct      5100
   ctgctgaagc cagttacctt cggaaaaaga gttggtagct cttgatccgg caaacaaacc      5160
   accgctggta gcggtggttt ttttgtttgc aagcagcaga ttacgcgcag aaaaaaagga      5220
   tctcaagaag atcctttgat cttttctacg gggtctgacg ctcagtggaa cgaaaactca      5280
   cgttaaggga ttttggtcat gcatgagatc                                      5310
<210> 31
   <211> 44
   <212> DNA
   <213> Artificial
<220>
   <223> A primer with a symbol F32dBgalXho used to obtain a PCR product with a symbol
   H346_BGa132d and a PCR product with a symbol H5A_BGa132d
<400> 31
   actgctcgag aaaagaatgc gggtgaccca gaaactgaac catg 44
<210> 32
   <211> 34
   <212> DNA
   <213> Artificial
<220>
   <223> A primer with a symbol R32dBgalXba used to obtain a PCR product with a symbol
   H346 BGa132d
<400> 32
   agtctctaga ctagccgacg gtgaccgtgg ccac 34
<210> 33
   <211> 2222
   <212> DNA
   <213> Artificial
<220>
   <223> A PCR product with a symbol H346_BGa132d used to obtain plasmid expression
   vectors with symbols pH3Bga132d, pH4Bga132d i pH6Bgal32d
<400> 33
   actgctcgag aaaagaatgc gggtgaccca gaaactgaac catggctgga tctttgccga      60
   aggtgccgcc gacccggcga caccgctggc aggcgagacg gtaaccctgc cgcataacgc     120
   agtcgatctg ccgctgagct atttcgatga gaccagctat cagcgcgcct tcacctatca     180
   gcgcgtgatc gcgtgggatg acgcgtggca gggccgccgg gtgcagctgc gctttgacgg     240
   ggcgatggcc gacaatgtgg tctgggtgaa cggcgtgcag gtcgtggcgc atcccgacgg     300
   ctacaccccc ttcgtggccg acctgaccga ccatctgcgc cccggcgaca atctggtgac     360
   ggtgcggatc gacgggtcgg aaaaccccgc catcccgccc tttggcgcgc agatcgacta     420
   tctgacctat gcaggcatct atcgcgatgt ctggctgatg gtgctgcccg agcggcacct     480
   gaccaatgcc cgcatcctga cgcccgatgc gctgtcggac gcgaaaacgg tcgtgatccg     540
   accggaggtc accgctcccg gcccggtccg cgcccgcctg ctggacggcg accgcgagat     600
   tgccgcgacc gaaggagagg gcgagctgac cctggcgggc ctgaccggcc tgtcgctgtg     660
   gtccaccgac aacccacagc tttacacggt ggagctgacc ctgcccgaca gcggcgatgt     720
   gaccacgcat cgctttggtt tccgcaccgc ggaatggaca ccgcagggct tcctgctgaa     780
   tggccagccg atgaagctgc gcgggttgaa ccggcatcaa agctgggcgc atcagggcta     840
   tgccgcgggc cgtcacgcgc aggaacgcga cgccgagatc gtccgccatg atctgtgctg     900
   caacatggtg cgcacatcgc attacccgca aagcacgtgg ttcctggacc gctgcgacga     960
   gatcgggttg ctggtgttcg aggagatccc cggctggcag catatcggcg atcaggcctg    1020
   gcaggatcgc agcgtggaca atgtgcgcgc catgatcacc cgcgactgga accacccaag    1080
   catcgtcatc tggggcgtgc gcatcaacga aagcccggac aaccacgatt tctatgtccg    1140
   taccaacgcg ctggcgcgcg agctggaccc gacgcgggcc atcggcggcg tgcgctgcat    1200
   caccgacagc gagatgctgg aggatgtcta tacgatgaat gatttcatcc tggacgaatc    1260
   cgagctgccg ctgatcaacc ggccgcgcac cgcgctgcgt ccgaccgagg aggtcacggg    1320
   gatcaagaaa ccggtgcctt atctggtcac cgaatataac ggccacatgt tcccgaccaa    1380
   ggcgcaggac ccggagctgc gccagatgga gcatgtgatc cgccaccttg aggtgctgaa    1440
   cgccgcccat ggcgacccgg cgatttcggg gtgcatcggc tggtgcatgt tcgattacaa    1500
   cacgcacaag gatttcggcg cgggcgaccg catctgccat catggcgtca tggacatctg    1560
   gcgagagccg aaattcgcgg cccatgccta tggcagccag aagccgccct cggaaggcat    1620
   cgtgatggag ccggtgacct tctgggcgcg gggcgaacgc aatatcggcg gtgtcctgcc    1680
   gctgatcgtg ctgacgaact gcgacgaggt cgagttcgaa tgcgcgggcg tcacccgccg    1740
   cgtaggtccg gaccgagagc ggttccccca tctgccgcgc ccgcccgtca tcatcgacca    1800
   ccgccatatc agcgccgagg aactgggcca gtgggggatg agctggcatc cgggccgcat    1860
   cacaggttgg ctgaacggcg aacaggtcgc gctgcgcgaa tatgtcgccg acccgctgcc    1920
   caccacgttg cagatcgcgc ccgaccgcga cacgctgccg gcggatgggg atatcgacct    1980
   gcgggtgatg ctgcgggcgc tggaccaagt cggcaaccgg ctgcctttcc tggacgcggg    2040
   catcgcggtg acggtggacg gccccgcgcg cctgatcggg cccgatctgc gcatgctgca    2100
   ggggggcacg acgggcatgt tgctgcgtct gacgggcgat gccgggacca tccgcatcac    2160
   cgcccgccat ccgcagtttc cggaggcggt ggccacggtc accgtcggct agtctagaga    2220
   ct                                                                   2222
<210> 34
   <211> 5242
   <212> DNA
   <213> Artificial
<220>
   <223> An expression plasmid with a symbol pH4Bgal32d
<400> 34
   gatccacttc tcttcttact ttcactcctt tccctacacg gttattgtac gtgtctattc      60
   cggtatttaa attagctatg ctgaaaatta cgacagaatg tttttattgc cgcgtgtatt     120
   ttcaattata tagtagtatt taagcatcgt cgacctgcac aaaaccctca tcgtaccata     180
   ttttacccat actagttgaa aatttttttt ttttttgctc tgcgggtgag ggggcgttgc      240
   ccaatatata aggagtggcc cttgtcttag acaaaaattt tccaggttat aagttttttt      300
   tttttcttct tcttttactt catctaaact aaacttaatt aaaaggcggt gttgacaatt     360
   aatcatcggc atagtatatc ggcatagtat aatacgacaa ggtgaggaac taaaccatgg     420
   ccaagttgac cagtgccgtt ccggtgctca ccgcgcgcga cgtcgccgga gcggtcgagt     480
   tctggaccga ccggctcggg ttctcccggg acttcgtgga ggacgacttc gccggtgtgg     540
   tccgggacga cgtgaccctg ttcatcagcg cggtccagga ccaggtggtg ccggacaaca     600
   ccctggcctg ggtgtgggtg cgcggcctgg acgagctgta cgccgagtgg tcggaggtcg     660
   tgtccacgaa cttccgggac gcctccgggc cggccatgac cgagatcggc gagcagccgt     720
   gggggcggga gttcgccctg cgcgacccgg ccggcaactg cgtgcacttc gtggccgagg 780
   agcaggactg acacgtccga cggcggccca cgggtcccag gcctcggaga tccgtccccc 840
   ttttcctttg tcgatatcat gtaattagtt atgtcacgct tacattcacg ccctcccccc 900
   acatccgctc taaccgaaaa ggaaggagtt agacaacctg aagtctaggt ccctatttat 960
   ttttttatag ttatgttagt attaagaacg ttatttatat ttcaaatttt tctttttttt 1020
   ctgtacagac gcgtgtacgc atgtaacatt atactgaaaa ccttgcttga gaaggttttg 1080
   ggacgctcga aggctttaat ttgcaagctg gagaccaaca tgtgagcaaa aggccagcaa 1140
   aaggccagga accgtaaaaa ggccgcgttg ctggcgtttt tccataggct ccgcccccct 1200
   gacgagcatc acaaaaatcg acgctcaagt cagaggtggc gaaacccgac aggactataa 1260
   agataccagg cgtttccccc tggaagctcc ctcgtgcgct ctcctgttcc gaccctgccg 1320
   cttaccggat acctgtccgc ctttctccct tcgggaagcg tggcgctttc tcaatgctca 1380
   cgctgtaggt atctcagttc ggtgtaggtc gttcgctcca agctgggctg tgtgcacgaa 1440
   ccccccgttc agcccgaccg ctgcgcctta tccggtaact atcgtcttga gtccaacccg 1500
   gtaagacacg acttatcgcc actggcagca gccactggta acaggattag cagagcgagg 1560
   tatgtaggcg gtgctacaga gttcttgaag tggtggccta actacggcta cactagaagg 1620
   acagtatttg gtatctgcgc tctgctgaag ccagttacct tcggaaaaag agttggtagc 1680
   tcttgatccg gcaaacaaac caccgctggt agcggtggtt tttttgtttg caagcagcag 1740
   attacgcgca gaaaaaaagg atctcaagaa gatcctttga tcttttctac ggggtctgac 1800
   gctcagtgga acgaaaactc acgttaaggg attttggtca tgcatgagat cagatcttac 1860
   cacaattcaa gacggccctt atgtgcctgt tctccaacgt gtccttatca gttcataatc 1920
   gccacctggt tgtgtctcaa tcgccagatt taccggttct cctaaattgc gcctacattt 1980
   gtcgttctca cgtcctgtgc tctctttcta catggacgag ttgctgccaa cgaccatttt 2040
   tcacggtatc aagtggtggt tagcctcccg gcgaccctgg acggctcgct tccgttgatc 2100
   cttccatact aattaacggc ctcggacttt tgtagcgcga aatcatgaga atcaccaaac 2160
   tcccggcaac tagctcggtc gtgcccggca ccggggcctc tatgggattc ttcgccccgg 2220
   gagtccagca ataagaacat agtggaaaat tataaatatg agtaattcgc cgtgcaatcg 2280
   acatggagta tattttaaag gttatcttat tattcaattg aatataatta actagtaaga 2340
   atatttaata attaaacttc gaaacgatga gatttccttc aatttttact gctgttttat 2400
   tcgcagcatc ctccgcatta gctgctccag tcaacactac aacagaagat gaaacggcac 2460
   aaattccggc tgaagctgtc atcggttact cagatttaga aggggatttc gatgttgctg 2520
   ttttgccatt ttccaacagc acaaataacg ggttattgtt tataaatact actattgcca 2580
   gcattgctgc taaagaagaa ggggtatctc tcgagaaaag aatgcgggtg acccagaaac 2640
   tgaaccatgg ctggatcttt gccgaaggtg ccgccgaccc ggcgacaccg ctggcaggcg 2700
   agacggtaac cctgccgcat aacgcagtcg atctgccgct gagctatttc gatgagacca 2760
   gctatcagcg cgccttcacc tatcagcgcg tgatcgcgtg ggatgacgcg tggcagggcc 2820
   gccgggtgca gctgcgcttt gacggggcga tggccgacaa tgtggtctgg gtgaacggcg    2880
   tgcaggtcgt ggcgcatccc gacggctaca cccccttcgt ggccgacctg accgaccatc    2940
   tgcgccccgg cgacaatctg gtgacggtgc ggatcgacgg gtcggaaaac cccgccatcc 3000
   cgccctttgg cgcgcagatc gactatctga cctatgcagg catctatcgc gatgtctggc 3060
   tgatggtgct gcccgagcgg cacctgacca atgcccgcat cctgacgccc gatgcgctgt 3120
   cggacgcgaa aacggtcgtg atccgaccgg aggtcaccgc tcccggcccg gtccgcgccc 3180
   gcctgctgga cggcgaccgc gagattgccg cgaccgaagg agagggcgag ctgaccctgg 3240
   cgggcctgac cggcctgtcg ctgtggtcca ccgacaaccc acagctttac acggtggagc 3300
   tgaccctgcc cgacagcggc gatgtgacca cgcatcgctt tggtttccgc accgcggaat 3360
   ggacaccgca gggcttcctg ctgaatggcc agccgatgaa gctgcgcggg ttgaaccggc 3420
   atcaaagctg ggcgcatcag ggctatgccg cgggccgtca cgcgcaggaa cgcgacgccg 3480
   agatcgtccg ccatgatctg tgctgcaaca tggtgcgcac atcgcattac ccgcaaagca      3540
   cgtggttcct ggaccgctgc gacgagatcg ggttgctggt gttcgaggag atccccggct      3600
   ggcagcatat cggcgatcag gcctggcagg atcgcagcgt ggacaatgtg cgcgccatga      3660
   tcacccgcga ctggaaccac ccaagcatcg tcatctgggg cgtgcgcatc aacgaaagcc      3720
   cggacaacca cgatttctat gtccgtacca acgcgctggc gcgcgagctg gacccgacgc      3780
   gggccatcgg cggcgtgcgc tgcatcaccg acagcgagat gctggaggat gtctatacga      3840
   tgaatgattt catcctggac gaatccgagc tgccgctgat caaccggccg cgcaccgcgc      3900
   tgcgtccgac cgaggaggtc acggggatca agaaaccggt gccttatctg gtcaccgaat      3960
   ataacggcca catgttcccg accaaggcgc aggacccgga gctgcgccag atggagcatg      4020
   tgatccgcca ccttgaggtg ctgaacgccg cccatggcga cccggcgatt tcggggtgca      4080
   tcggctggtg catgttcgat tacaacacgc acaaggattt cggcgcgggc gaccgcatct      4140
   gccatcatgg cgtcatggac atctggcgag agccgaaatt cgcggcccat gcctatggca      4200
   gccagaagcc gccctcggaa ggcatcgtga tggagccggt gaccttctgg gcgcggggcg      4260
   aacgcaatat cggcggtgtc ctgccgctga tcgtgctgac gaactgcgac gaggtcgagt      4320
   tcgaatgcgc gggcgtcacc cgccgcgtag gtccggaccg agagcggttc ccccatctgc     4380
   cgcgcccgcc cgtcatcatc gaccaccgcc atatcagcgc cgaggaactg ggccagtggg      4440
   ggatgagctg gcatccgggc cgcatcacag gttggctgaa cggcgaacag gtcgcgctgc      4500
   gcgaatatgt cgccgacccg ctgcccacca cgttgcagat cgcgcccgac cgcgacacgc      4560
   tgccggcgga tggggatatc gacctgcggg tgatgctgcg ggcgctggac caagtcggca      4620
   accggctgcc tttcctggac gcgggcatcg cggtgacggt ggacggcccc gcgcgcctga      4680
   tcgggcccga tctgcgcatg ctgcaggggg gcacgacggg catgttgctg cgtctgacgg      4740
   gcgatgccgg gaccatccgc atcaccgccc gccatccgca gtttccggag gcggtggcca      4800
   cggtcaccgt cggctagtct agaacaaaaa ctcatctcag aagaggatct gaatagcgcc      4860
   gtcgaccatc atcatcatca tcattgagtt ttagccttag acatgactgt tcctcagttc      4920
   aagttgggca cttacgagaa gaccggtctt gctagattct aaaccattta ttagatgttg      4980
   aaaagtttac tcgtcgtttt gtacaaacac tgtttaatgg gcctcgaatt tagctagtta      5040
   cacatttatc tgtatataca tgaataatat taacgaacga tgttagatta aatgcttaca      5100
   gtagagtaca agcagtgggg gttatatttg gatggacatt atctgttcac gtgacccagt      5160
   atgagtgaaa agttcgccaa gtcaaccact gtattacaga cggcaattat attacaatta      5220
   gtggcaaccc ttcgtttgtg cg                                               5242
<210> 35
   <211> 5560
   <212> DNA
   <213> Artificial
<220>
   <223> An expression plasmid with a symbol pH5ABga132d
<400> 35
   tcgagaaaag aatgcgggtg acccagaaac tgaaccatgg ctggatcttt gccgaaggtg        60
   ccgccgaccc ggcgacaccg ctggcaggcg agacggtaac cctgccgcat aacgcagtcg       120
   atctgccgct gagctatttc gatgagacca gctatcagcg cgccttcacc tatcagcgcg       180
   tgatcgcgtg ggatgacgcg tggcagggcc gccgggtgca gctgcgcttt gacggggcga 240
   tggccgacaa tgtggtctgg gtgaacggcg tgcaggtcgt ggcgcatccc gacggctaca 300
   cccccttcgt ggccgacctg accgaccatc tgcgccccgg cgacaatctg gtgacggtgc 360
   ggatcgacgg gtcggaaaac cccgccatcc cgccctttgg cgcgcagatc gactatctga 420
   cctatgcagg catctatcgc gatgtctggc tgatggtgct gcccgagcgg cacctgacca 480
   atgcccgcat cctgacgccc gatgcgctgt cggacgcgaa aacggtcgtg atccgaccgg 540
   aggtcaccgc tcccggcccg gtccgcgccc gcctgctgga cggcgaccgc gagattgccg 600
   cgaccgaagg agagggcgag ctgaccctgg cgggcctgac cggcctgtcg ctgtggtcca 660
   ccgacaaccc acagctttac acggtggagc tgaccctgcc cgacagcggc gatgtgacca 720
   cgcatcgctt tggtttccgc accgcggaat ggacaccgca gggcttcctg ctgaatggcc 780
   agccgatgaa gctgcgcggg ttgaaccggc atcaaagctg ggcgcatcag ggctatgccg 840
   cgggccgtca cgcgcaggaa cgcgacgccg agatcgtccg ccatgatctg tgctgcaaca 900
   tggtgcgcac atcgcattac ccgcaaagca cgtggttcct ggaccgctgc gacgagatcg 960
   ggttgctggt gttcgaggag atccccggct ggcagcatat cggcgatcag gcctggcagg 1020
   atcgcagcgt ggacaatgtg cgcgccatga tcacccgcga ctggaaccac ccaagcatcg 1080
   tcatctgggg cgtgcgcatc aacgaaagcc cggacaacca cgatttctat gtccgtacca 1140
   acgcgctggc gcgcgagctg gacccgacgc gggccatcgg cggcgtgcgc tgcatcaccg 1200
   acagcgagat gctggaggat gtctatacga tgaatgattt catcctggac gaatccgagc 1260
   tgccgctgat caaccggccg cgcaccgcgc tgcgtccgac cgaggaggtc acggggatca 1320
   agaaaccggt gccttatctg gtcaccgaat ataacggcca catgttcccg accaaggcgc 1380
   aggacccgga gctgcgccag atggagcatg tgatccgcca ccttgaggtg ctgaacgccg 1440
   cccatggcga cccggcgatt tcggggtgca tcggctggtg catgttcgat tacaacacgc 1500
   acaaggattt cggcgcgggc gaccgcatct gccatcatgg cgtcatggac atctggcgag 1560
   agccgaaatt cgcggcccat gcctatggca gccagaagcc gccctcggaa ggcatcgtga 1620
   tggagccggt gaccttctgg gcgcggggcg aacgcaatat cggcggtgtc ctgccgctga 1680
   tcgtgctgac gaactgcgac gaggtcgagt tcgaatgcgc gggcgtcacc cgccgcgtag 1740
   gtccggaccg agagcggttc ccccatctgc cgcgcccgcc cgtcatcatc gaccaccgcc 1800
   atatcagcgc cgaggaactg ggccagtggg ggatgagctg gcatccgggc cgcatcacag 1860
   gttggctgaa cggcgaacag gtcgcgctgc gcgaatatgt cgccgacccg ctgcccacca 1920
   cgttgcagat cgcgcccgac cgcgacacgc tgccggcgga tggggatatc gacctgcggg 1980
   tgatgctgcg ggcgctggac caagtcggca accggctgcc tttcctggac gcgggcatcg 2040
   cggtgacggt ggacggcccc gcgcgcctga tcgggcccga tctgcgcatg ctgcaggggg 2100
   gcacgacggg catgttgctg cgtctgacgg gcgatgccgg gaccatccgc atcaccgccc 2160
   gccatccgca gtttccggag gcggtggcca cggtcaccgt cggctagcgg ccgccagctt 2220
   tctagaacaa aaactcatct cagaagagga tctgaatagc gccgtcgacc atcatcatca 2280
   tcatcattga gttttagcct tagacatgac tgttcctcag ttcaagttgg gcacttacga 2340
   gaagaccggt cttgctagat tctaaaccat ttattagatg ttgaaaagtt tactcgtcgt 2400
   tttgtacaaa cactgtttaa tgggcctcga atttagctag ttacacattt atctgtatat 2460
   acatgaataa tattaacgaa cgatgttaga ttaaatgctt acagtagagt acaagcagtg 2520
   ggggttatat ttggatggac attatctgtt cacgtgaccc agtatgagtg aaaagttcgc 2580
   caagtcaacc actgtattac agacggcaat tatattacaa ttagtggcaa cccttcgttt 2640
   gtgcggatcc acttctcttc ttactttcac tcctttccct acacggttat tgtacgtgtc 2700
   tattccggta tttaaattag ctatgctgaa aattacgaca gaatgttttt attgccgcgt 2760
   gtattttcaa ttatatagta gtatttaagc atcgtcgacc tgcacaaaac cctcatcgta 2820
   ccatatttta cccatactag ttgaaaattt tttttttttt tgctctgcgg gtgagggggc 2880
   gttgcccaat atataaggag tggcccttgt cttagacaaa aattttccag gttataagtt 2940
   tttttttttt cttcttcttt tacttcatct aaactaaact taattaaaag gcggtgttga 3000
   caattaatca tcggcatagt atatcggcat agtataatac gacaaggtga ggaactaaac 3060
   catggccaag ttgaccagtg ctgtcccagt gctcacagcc agggatgtgg ctggagctgt 3120
   tgagttctgg actgacaggt tggggttctc cagagatttt gtggaggatg actttgcagg 3180
   tgtggtcaga gatgatgtca ccctgttcat ctcagcagtc caggaccagg tggtgcctga 3240
   caacaccctg gcttgggtgt gggtgagagg actggatgag ctgtatgctg agtggagtga 3300
   ggtggtctcc accaacttca gggatgccag tggccctgcc atgacagaga ttggagagca 3360
   gccctggggg agagagtttg ccctgagaga cccagcaggc aactgtgtgc actttgtggc 3420
   agaggagcag gactgacacg tccgacggcg gcccacgggt cccaggcctc ggagatccgt 3480
   cccccttttc ctttgtcgat atcatttaga acactctaga taagcaaaag caaaataaaa 3540
   gcgaaatgct ctgtatataa taatttatgt tttatttaat gatagagtgt agagagtgag 3600
   agagattgta agcccccaga aaatgaagaa atatatttat tgatgaagat gagaatactt 3660
   ttatgagacc ctccataggt tgctttatgc aagggagatg cagttttgca tgagatcgag 3720
   attacttatc ctagaagttg aatttataat aggcgtatta gaggtagaaa atagcacttt 3780
   tttaagaata acttgaataa tctccattaa tcaaaaacac aaacattaac taagcgtgtc 3840
   atattttact tttttctata atcatctcta tcgccttttt cttttttttc tcagattcta 3900
   tttctcaaac acactctctc tgactctcac tctcactttc actctttgac ttgtttcatc 3960
   tgaaaaatcc tgcatatgtg caactagtgt ttcgatatat cgatctgtac cttcttgact 4020
   aaccgctctt tcacttctct ttcgtcaaac ttcgcttaca ttataataca tgtgagcaaa 4080
   aggccagcaa aaggccagga accgtaaaaa ggccgcgttg ctggcgtttt tccataggct 4140
   ccgcccccct gacgagcatc acaaaaatcg acgctcaagt cagaggtggc gaaacccgac 4200
   aggactataa agataccagg cgtttccccc tggaagctcc ctcgtgcgct ctcctgttcc 4260
   gaccctgccg cttaccggat acctgtccgc ctttctccct tcgggaagcg tggcgctttc 4320
   tcaatgctca cgctgtaggt atctcagttc ggtgtaggtc gttcgctcca agctgggctg 4380
   tgtgcacgaa ccccccgttc agcccgaccg ctgcgcctta tccggtaact atcgtcttga 4440
   gtccaacccg gtaagacacg acttatcgcc actggcagca gccactggta acaggattag 4500
   cagagcgagg tatgtaggcg gtgctacaga gttcttgaag tggtggccta actacggcta 4560
   cactagaagg acagtatttg gtatctgcgc tctgctgaag ccagttacct tcggaaaaag 4620
   agttggtagc tcttgatccg gcaaacaaac caccgctggt agcggtggtt tttttgtttg 4680
   caagcagcag attacgcgca gaaaaaaagg atctcaagaa gatcctttga tcttttctac 4740
   ggggtctgac gctcagtgga acgaaaactc acgttaaggg attttggtca tgcatgagat 4800
   cagatcttac cacaattcaa gacggccctt atgtgcctgt tctccaacgt gtccttatca 4860
   gttcataatc gccacctggt tgtgtctcaa tcgccagatt taccggttct cctaaattgc 4920
   gcctacattt gtcgttctca cgtcctgtgc tctctttcta catggacgag ttgctgccaa 4980
   cgaccatttt tcacggtatc aagtggtggt tagcctcccg gcgaccctgg acggctcgct 5040
   tccgttgatc cttccatact aattaacggc ctcggacttt tgtagcgcga aatcatgaga 5100
   atcaccaaac tcccggcaac tagctcggtc gtgcccggca ccggggcctc tatgggattc 5160
   ttcgccccgg gagtccagca ataagaacat agtggaaaat tataaatatg agtaattcgc 5220
   cgtgcaatcg acatggagta tattttaaag gttatcttat tattcaattg aatataatta 5280
   actagtaaga atatttaata attaaacttc gaaacgatga gatttccttc aatttttact 5340
   gctgttttat tcgcagcatc ctccgcatta gctgctccag tcaacactac aacagaagat 5400
   gaaacggcac aaattccggc tgaagctgtc atcggttact cagatttaga aggggatttc 5460
   gatgttgctg ttttgccatt ttccaacagc acaaataacg ggttattgtt tataaatact 5520
   actattgcca gcattgctgc taaagaagaa ggggtatctc 5560
<210> 36
   <211> 35
   <212> DNA
   <213> Artificial
<220>
   <223> A primer with a symbol R32dBgalNot used to obtain a PCR product with a symbol
   H5A BGa132d
<400> 36
   ttatgcggcc gctagccgac ggtgaccgtg gccac 35
<210> 37
   <211> 2223
   <212> DNA
   <213> Artificial
<220>
   <223> A PCR product H5A_BGa132d used to obtain an expression plasmid with a symbol
   pH5ABga132d
<400> 37
   actgctcgag aaaagaatgc gggtgaccca gaaactgaac catggctgga tctttgccga      60
   aggtgccgcc gacccggcga caccgctggc aggcgagacg gtaaccctgc cgcataacgc     120
   agtcgatctg ccgctgagct atttcgatga gaccagctat cagcgcgcct tcacctatca     180
   gcgcgtgatc gcgtgggatg acgcgtggca gggccgccgg gtgcagctgc gctttgacgg     240
   ggcgatggcc gacaatgtgg tctgggtgaa cggcgtgcag gtcgtggcgc atcccgacgg     300
   ctacaccccc ttcgtggccg acctgaccga ccatctgcgc cccggcgaca atctggtgac     360
   ggtgcggatc gacgggtcgg aaaaccccgc catcccgccc tttggcgcgc agatcgacta     420
   tctgacctat gcaggcatct atcgcgatgt ctggctgatg gtgctgcccg agcggcacct     480
   gaccaatgcc cgcatcctga cgcccgatgc gctgtcggac gcgaaaacgg tcgtgatccg     540
   accggaggtc accgctcccg gcccggtccg cgcccgcctg ctggacggcg accgcgagat     600
   tgccgcgacc gaaggagagg gcgagctgac cctggcgggc ctgaccggcc tgtcgctgtg     660
   gtccaccgac aacccacagc tttacacggt ggagctgacc ctgcccgaca gcggcgatgt     720
   gaccacgcat cgctttggtt tccgcaccgc ggaatggaca ccgcagggct tcctgctgaa     780
   tggccagccg atgaagctgc gcgggttgaa ccggcatcaa agctgggcgc atcagggcta     840
   tgccgcgggc cgtcacgcgc aggaacgcga cgccgagatc gtccgccatg atctgtgctg     900
   caacatggtg cgcacatcgc attacccgca aagcacgtgg ttcctggacc gctgcgacga     960
   gatcgggttg ctggtgttcg aggagatccc cggctggcag catatcggcg atcaggcctg    1020
   gcaggatcgc agcgtggaca atgtgcgcgc catgatcacc cgcgactgga accacccaag    1080
   catcgtcatc tggggcgtgc gcatcaacga aagcccggac aaccacgatt tctatgtccg    1140
   taccaacgcg ctggcgcgcg agctggaccc gacgcgggcc atcggcggcg tgcgctgcat    1200
   caccgacagc gagatgctgg aggatgtcta tacgatgaat gatttcatcc tggacgaatc 1260
   cgagctgccg ctgatcaacc ggccgcgcac cgcgctgcgt ccgaccgagg aggtcacggg 1320
   gatcaagaaa ccggtgcctt atctggtcac cgaatataac ggccacatgt tcccgaccaa 1380
   ggcgcaggac ccggagctgc gccagatgga gcatgtgatc cgccaccttg aggtgctgaa 1440
   cgccgcccat ggcgacccgg cgatttcggg gtgcatcggc tggtgcatgt tcgattacaa 1500
   cacgcacaag gatttcggcg cgggcgaccg catctgccat catggcgtca tggacatctg 1560
   gcgagagccg aaattcgcgg cccatgccta tggcagccag aagccgccct cggaaggcat 1620
   cgtgatggag ccggtgacct tctgggcgcg gggcgaacgc aatatcggcg gtgtcctgcc 1680
   gctgatcgtg ctgacgaact gcgacgaggt cgagttcgaa tgcgcgggcg tcacccgccg 1740
   cgtaggtccg gaccgagagc ggttccccca tctgccgcgc ccgcccgtca tcatcgacca 1800
   ccgccatatc agcgccgagg aactgggcca gtgggggatg agctggcatc cgggccgcat 1860
   cacaggttgg ctgaacggcg aacaggtcgc gctgcgcgaa tatgtcgccg acccgctgcc 1920
   caccacgttg cagatcgcgc ccgaccgcga cacgctgccg gcggatgggg atatcgacct 1980
   gcgggtgatg ctgcgggcgc tggaccaagt cggcaaccgg ctgcctttcc tggacgcggg 2040
   catcgcggtg acggtggacg gccccgcgcg cctgatcggg cccgatctgc gcatgctgca 2100
   ggggggcacg acgggcatgt tgctgcgtct gacgggcgat gccgggacca tccgcatcac 2160
   cgcccgccat ccgcagtttc cggaggcggt ggccacggtc accgtcggct agcggccgca 2220
   taa 2223
<210> 38
   <211> 5556
   <212> DNA
   <213> Artificial
<220>
   <223> An expression plasmid with symbol pH6Bgal32d
<400> 38
   agatcttacc acaattcaag acggccctta tgtgcctgtt ctccaacgtg tccttatcag 60
   ttcataatcg ccacctggtt gtgtctcaat cgccagattt accggttctc ctaaattgcg 120
   cctacatttg tcgttctcac gtcctgtgct ctctttctac atggacgagt tgctgccaac 180
   gaccattttt cacggtatca agtggtggtt agcctcccgg cgaccctgga cggctcgctt 240
   ccgttgatcc ttccatacta attaacggcc tcggactttt gtagcgcgaa atcatgagaa 300
   tcaccaaact cccggcaact agctcggtcg tgcccggcac cggggcctct atgggattct 360
   tcgccccggg agtccagcaa taagaacata gtggaaaatt ataaatatga gtaattcgcc 420
   gtgcaatcga catggagtat attttaaagg ttatcttatt attcaattga atataattaa 480
   ctagtaagaa tatttaataa ttaaacttcg aaacgatgag atttccttca atttttactg 540
   ctgttttatt cgcagcatcc tccgcattag ctgctccagt caacactaca acagaagatg 600
   aaacggcaca aattccggct gaagctgtca tcggttactc agatttagaa ggggatttcg 660
   atgttgctgt tttgccattt tccaacagca caaataacgg gttattgttt ataaatacta 720
   ctattgccag cattgctgct aaagaagaag gggtatctct cgagaaaaga atgcgggtga 780
   cccagaaact gaaccatggc tggatctttg ccgaaggtgc cgccgacccg gcgacaccgc 840
   tggcaggcga gacggtaacc ctgccgcata acgcagtcga tctgccgctg agctatttcg 900
   atgagaccag ctatcagcgc gccttcacct atcagcgcgt gatcgcgtgg gatgacgcgt 960
   ggcagggccg ccgggtgcag ctgcgctttg acggggcgat ggccgacaat gtggtctggg 1020
   tgaacggcgt gcaggtcgtg gcgcatcccg acggctacac ccccttcgtg gccgacctga 1080
   ccgaccatct gcgccccggc gacaatctgg tgacggtgcg gatcgacggg tcggaaaacc 1140
   ccgccatccc gccctttggc gcgcagatcg actatctgac ctatgcaggc atctatcgcg 1200
   atgtctggct gatggtgctg cccgagcggc acctgaccaa tgcccgcatc ctgacgcccg 1260
   atgcgctgtc ggacgcgaaa acggtcgtga tccgaccgga ggtcaccgct cccggcccgg 1320
   tccgcgcccg cctgctggac ggcgaccgcg agattgccgc gaccgaagga gagggcgagc 1380
   tgaccctggc gggcctgacc ggcctgtcgc tgtggtccac cgacaaccca cagctttaca 1440
   cggtggagct gaccctgccc gacagcggcg atgtgaccac gcatcgcttt ggtttccgca 1500
   ccgcggaatg gacaccgcag ggcttcctgc tgaatggcca gccgatgaag ctgcgcgggt 1560
   tgaaccggca tcaaagctgg gcgcatcagg gctatgccgc gggccgtcac gcgcaggaac 1620
   gcgacgccga gatcgtccgc catgatctgt gctgcaacat ggtgcgcaca tcgcattacc 1680
   cgcaaagcac gtggttcctg gaccgctgcg acgagatcgg gttgctggtg ttcgaggaga 1740
   tccccggctg gcagcatatc ggcgatcagg cctggcagga tcgcagcgtg gacaatgtgc 1800
   gcgccatgat cacccgcgac tggaaccacc caagcatcgt catctggggc gtgcgcatca 1860
   acgaaagccc ggacaaccac gatttctatg tccgtaccaa cgcgctggcg cgcgagctgg 1920
   acccgacgcg ggccatcggc ggcgtgcgct gcatcaccga cagcgagatg ctggaggatg 1980
   tctatacgat gaatgatttc atcctggacg aatccgagct gccgctgatc aaccggccgc 2040
   gcaccgcgct gcgtccgacc gaggaggtca cggggatcaa gaaaccggtg ccttatctgg 2100
   tcaccgaata taacggccac atgttcccga ccaaggcgca ggacccggag ctgcgccaga 2160
   tggagcatgt gatccgccac cttgaggtgc tgaacgccgc ccatggcgac ccggcgattt 2220
   cggggtgcat cggctggtgc atgttcgatt acaacacgca caaggatttc ggcgcgggcg 2280
   accgcatctg ccatcatggc gtcatggaca tctggcgaga gccgaaattc gcggcccatg 2340
   cctatggcag ccagaagccg ccctcggaag gcatcgtgat ggagccggtg accttctggg 2400
   cgcggggcga acgcaatatc ggcggtgtcc tgccgctgat cgtgctgacg aactgcgacg 2460
   aggtcgagtt cgaatgcgcg ggcgtcaccc gccgcgtagg tccggaccga gagcggttcc 2520
   cccatctgcc gcgcccgccc gtcatcatcg accaccgcca tatcagcgcc gaggaactgg 2580
   gccagtgggg gatgagctgg catccgggcc gcatcacagg ttggctgaac ggcgaacagg 2640
   tcgcgctgcg cgaatatgtc gccgacccgc tgcccaccac gttgcagatc gcgcccgacc 2700
   gcgacacgct gccggcggat ggggatatcg acctgcgggt gatgctgcgg gcgctggacc 2760
   aagtcggcaa ccggctgcct ttcctggacg cgggcatcgc ggtgacggtg gacggccccg 2820
   cgcgcctgat cgggcccgat ctgcgcatgc tgcagggggg cacgacgggc atgttgctgc 2880
   gtctgacggg cgatgccggg accatccgca tcaccgcccg ccatccgcag tttccggagg 2940
   cggtggccac ggtcaccgtc ggctagtcta gaacaaaaac tcatctcaga agaggatctg 3000
   aatagcgccg tcgaccatca tcatcatcat cattgagttt tagccttaga catgactgtt 3060
   cctcagttca agttgggcac ttacgagaag accggtcttg ctagattcta aaccatttat 3120
   tagatgttga aaagtttact cgtcgttttg tacaaacact gtttaatggg cctcgaattt 3180
   agctagttac acatttatct gtatatacat gaataatatt aacgaacgat gttagattaa 3240
   atgcttacag tagagtacaa gcagtggggg ttatatttgg atggacatta tctgttcacg 3300
   tgacccagta tgagtgaaaa gttcgccaag tcaaccactg tattacagac ggcaattata 3360
   ttacaattag tggcaaccct tcgtttgtgc ggatcccact actcagactt accaccgcat 3420
   atacagacat taaccataat agtgccatct acagggcaca atgcatttaa tacacaatct 3480
   aatatacagc ttaagtagag cccatctgta attaaaccca cacaaagagt aattcagtca 3540
   catatactaa tgtgaacggc attcatttga cctagtgtca acatggcaaa agcttccgcc 3600
   gcagtttcgc aatttcacta catgtcgcct gtcatcagct acgaactgct atgtaagatc 3660
   aactgcaaaa tgttccactg gtccataatc gaagtttcta ggagtttgcc aaaaaattta      3720
   gtcccatact atcccaacca aatgtcctca aataactacc atcacaccta ttttgccaag      3780
   ttcttcctat ctactatata gctacttcat gctgcttgca ataccgtagt ctacggccat      3840
   agcacttgga ttctgaatac ttagaggaaa tagggtaaag ccctatccgg gtaaattaaa      3900
   tatttgtcct gctgaaaaaa aaaatttcgc cacgacaccc tctgatgaat ggaacgcaat      3960
   tggctctgca aaaatttata aggaattaaa ttgaagaact tctttttcct tctctttttt       4020
   tcaatacagt atactattat tatacattaa acaaagttga caattaatca tcggcatagt      4080
   atatcggcat agtataatac gacaaggtga ggaactaaac catggccaag ttgaccagtg      4140
   ccgttccggt gctcaccgcg cgcgacgtcg ccggagcggt cgagttctgg accgaccggc      4200
   tcgggttctc ccgggacttc gtggaggacg acttcgccgg tgtggtccgg gacgacgtga      4260
   ccctgttcat cagcgcggtc caggaccagg tggtgccgga caacaccctg gcctgggtgt      4320
   gggtgcgcgg cctggacgag ctgtacgccg agtggtcgga ggtcgtgtcc acgaacttcc      4380
   gggacgcctc cgggccggcc atgaccgaga tcggcgagca gccgtggggg cgggagttcg      4440
   ccctgcgcga cccggccggc aactgcgtgc acttcgtggc cgaggagcag gactgacacg      4500
   tccgacggcg gcccacgggt cccaggcctc ggagatccgt cccccttttc ctttgtcgat       4560
   atcatgtaat tagttatgtc acgcttacat tcacgccctc cccccacatc cgctctaacc      4620
   gaaaaggaag gagttagaca acctgaagtc taggtcccta tttatttttt tatagttatg       4680
   ttagtattaa gaacgttatt tatatttcaa atttttcttt tttttctgta cagacgcgtg      4740
   tacgcatgta acattatact gaaaaccttg cttgagaagg ttttgggacg ctcgaaggct      4800
   ttaatttgca agctggagac caacatgtga gcaaaaggcc agcaaaaggc caggaaccgt      4860
   aaaaaggccg cgttgctggc gtttttccat aggctccgcc cccctgacga gcatcacaaa      4920
   aatcgacgct caagtcagag gtggcgaaac ccgacaggac tataaagata ccaggcgttt      4980
   ccccctggaa gctccctcgt gcgctctcct gttccgaccc tgccgcttac cggatacctg      5040
   tccgcctttc tcccttcggg aagcgtggcg ctttctcaat gctcacgctg taggtatctc      5100
   agttcggtgt aggtcgttcg ctccaagctg ggctgtgtgc acgaaccccc cgttcagccc      5160
   gaccgctgcg ccttatccgg taactatcgt cttgagtcca acccggtaag acacgactta      5220
   tcgccactgg cagcagccac tggtaacagg attagcagag cgaggtatgt aggcggtgct      5280
   acagagttct tgaagtggtg gcctaactac ggctacacta gaaggacagt atttggtatc      5340
   tgcgctctgc tgaagccagt taccttcgga aaaagagttg gtagctcttg atccggcaaa      5400
   caaaccaccg ctggtagcgg tggttttttt gtttgcaagc agcagattac gcgcagaaaa      5460
   aaaggatctc aagaagatcc tttgatcttt tctacggggt ctgacgctca gtggaacgaa      5520
   aactcacgtt aagggatttt ggtcatgcat gagatc                                5556

## Description

The invention relates to an expression system for production of heterologous protein, in particular of thermolabile proteins, comprising a psychrotolerant strain of *Debaryomyces macquariensis* yeast and a plasmid expression vector. The invention relates to plasmid expression vectors comprising an expression cassette for expression of a heterologous gene, a method for construction of a recombinant strain of psychrotolerant yeast *Debaryomyces macquariensis* and a method of protein production by this recombinant strain of *Debaryomyces macquariensis* yeast. The invention enables constitutive expression of a heterologous gene - production of a heterologous protein in the expression system, preferably in a recombinant yeast strain, in particular in the psychrotolerant yeast *Debaryomyces macquariensis* strain D50.

The *Debaryomyces macquariensis* strain D50 according to an embodiment of the invention was deposited with the Culture Collection of Industrial Microorganisms maintained by the prof. Waclaw Dabrowski Institute of Agricultural and Food Biotechnology in Warsaw on 29.01.2018, accesion number assigned by the International Depositary Authority IBPRS is KKP 2066p.

Yeast expression systems combine features of procaryotic and eucaryotic expression systems used in production of heterologous proteins. On one hand, the use of yeast cells as an expression host of heterologous genes allows production of large biomass quantities in a short time - which is a feature typical for bacterial expression systems, whilst on the other hand, yeasts show an ability to post-translational modification of proteins typical for eucaryotic cells, i.e. glycosylation of selected proteins. An advantage of yeast-based systems producing proteins coded by a heterologous genes compared to other expression systems, e.g. bacterial expression systems, is efficient secretion of proteins and peptides outside the cell, enabling pre-purified protein preparations to be obtained - without the need for cell lysis and complicated and low yield protein isolation and purification from the biomass, lack of toxic metabolites, resistance to viral infections, genetic stability and low cost of culture media.

There are numerous known yeast expression systems using yeast cells of the following species as expression hosts of heterologous genes: *Saccharomyces cerevisiae, Pichia pastoris, Hansenula polymorpha, Arxula adeninivorans, Yarrowia lipolytica, Schizosaccharomyces pombe* or *Kluyveromyces lactis* (Wanarska M., Hildebrandt P., Kur J. Yeast expression systems as a tool in modern industrial microbiology. Postep. Mikrobiol. 2008, 47:249-256; Celik E., Calik P. Production of recombinant proteins by yeast cells. Biotechnol. Adv. 2012, 30(5):1108-1118; Ahmad M., Hirz M., Pichler H., Schwab H. Protein expression in Pichia pastoris: recent achievements and perspectives for heterologous protein production. Appl. Microbiol. Biotechnol. 2014, 98:5301-5317). The yeast species listed above are mesophilic or thermotolerant organisms, the optimal growth temperature of which is equal to or higher than 30°C. Because of the usually observed high thermal lability of proteins obtained from cold-adapted organisms, such systems are not optimal from the point of view of thermolabile protein production. Moveover, they may cause inactivation at cultivation temperature. Thus, they prevent or largely limit the ability to produce thermolabile proteins and peptides. Additionally, results of previously performed studies indicate that even in the case of proteins stable in elevated temperatures, effectiveness of their production often increases with decreasing cultivation temperature of the producing microbes, including *S*. *cerevisiae* or *P. pastoris* yeasts. Lowering the cultivation temperature of microorganisms has a very significant influence on cell metabolism, including expression regulation of genes coding proteins enabling correct folding and transport of other proteins, e.g. of chaperones of the hsp70 family or of proteins bound to the membrane of endoplasmic reticulum. This fact is particularly important for production of polypeptides foreign for the host, which are also produced in extremely large quantities, as incorrect protein folding triggers fast degradation systems in yeast cells. In addition, elevated temperature facilitates hydrophobic interactions and protein aggregation, also leading to their degradation in the cell and as a result, to decreased productivity of the expression system (Gasser B., Saloheimo M., Rinas U., Dragosits M., Rodriguez-Carmona E., Baumann K., Giuliani M., Parrilli E., Branduardi P., Lang C., Porro D., Ferrer P., Tutino M.L., Mattanovich D., Villaverde A. Protein folding and conformational stress in microbial cells producing recombinant proteins: a host comparative overview. Microb. Cell Fact. 2008, 7: 11).

An expression system based on a psychrotolerant yeast expression host is potentially the best system for production of proteins obtained from cold-adapted organisms. It combines advantages of procaryotic and eucaryotic expression systems. It enables fast biomass growth in the temperature range of 20-25°C, and post-translational modifications occurring in yeast cells allow correctly folded proteins to be obtained or their molecules to be modified via post-translational modifications typical for eucaryotic cells. This feature of psychrotolerant yeasts as a heterologous gene expression host causes expression systems based thereon to be potentially more useful than system based on psychrotolerant bacteria e.g. *Shewanella* sp. strain Ac 10 (Miyake R., Kawamoto J., Wei Y.L., Kitagawa M., Kato I., Kurihara T., Esaki N. Construction of a low-temperature protein expression system using a cold-adapted bacterium, Shewanella sp. strain Ac10, as the host. Appl. Environ. Microbiol. 2007, 73:4849-4856), *Shewanella oneidensis* AS5 (Sybirna K., Antoine T., Lindberg P., Fourmond V., Rousset M., Méjean V., Bottin H. Shewanella oneidensis: a new and efficient system for expression and maturation of heterologous [Fe-Fe] hydrogenase from Chlamydomonas reinhardtii. BMC Biotechnol. 2008, 8: 73*), Pseudoalteromonas haloplanktis* TAC125 (Cusano A.M., Parrilli E., Marino G., Tutino M.L. A novel genetic system for recombinant protein secretion in the Antarctic Pseudoalteromonas haloplanktis TAC125. Microb. Cell Fact. 2006, 5: 40; Papa R., Rippa V., Sannia G., Marino G., Duilio A. An efficient cold inducible expression-system developed in Pseudoalteromonas haloplanktis TAC125. J. Biotechnol. 2007, 127:199-210).

The objective of the invention is to provide useful methods for expression of a gene coding a heterologous protein, in particular enabling correct production of a thermolabile protein of cold-adapted organism as expected. This objective has been unexpectedly achieved using an expression system based on a developed plasmid expression vector containing an expression cassette used to express a gene coding heterologous protein, which comprises selected nucleotide sequences. In a preferred embodiment of the invention, an application of nucleotid sequences obtained from a psychrotolerant yeast *Debaryomyces macquariensis* strain D50, and a yeast *Debaryomyces macquariensis* strain D50 as the target host of heterologous gene expression was developed. The receipt of deposit, accesion number for the the yeast *Debaryomyces macquariensis* strain D50 deposit KKP 2066p, issued by the International Depositary Authority - IBPRS.

According to the invention, for production of heterologous protein, in particular thermolabile proteins, psychrotolerant yeast of the *Debaryomyces macquariensis* species is used, in an embodiment of the invention, a D50 strain of *Debaryomyces macquariensis* isolated from soil obtained from a penguin habitat from the area of Henryk Arctowski Polish Antarctic Station on King George Island, Antarctica. The *Debaryomyces macquariensis* D50 yeast, hereinafter abbreviated as *D. macquariensis* D50 or D50, assimilates various carbon sources, i.e. glucose, xylose, xylitol, galactose, calcium 2-keto-gluconate, N-acetylglucosamine, cellobiose, maltose, sucrose and melezitose. The growth temperature range in a YPD medium (2% peptone K, 1% yeast extract, 2% glucose) for *D. macquariensis* D50 is 0-30°C, with an optimum at 20°C. Optimal physical culture conditions for *Debaryomyces macquariensis* D50 yeast in a fermenter, in YPD (2% peptone K, 1% yeast extract, 2% glucose), YD (2% yeast extract, 5% glucose) and YP-TD (1% peptone-tryptone, 2% yeast extract, 5% glucose) are as follows: temperature 20°C, pH 5.5, aeration rate of 1.5 vvm and stirring speed of 300 rpm.

In accordance with the invention, an expression system for production of a heterologous protein comprises a host for cloning of a gene coding heterologous protein, namely a psychrotolerant *Debaryomyces macquariensis* yeast strain, and this system comprises a plasmid expression vector containing an expression cassette enabling expression of a heterologous gene coding a heterologous protein and an expression cassette enabling expression of a selection marker, namely an antibiotic resistance gene. The expression cassette, enabling expression of a heterologous gene, present in the plasmid expression vectors, comprises a *GAP* promoter obtained, isolated from a *Debaryomyces macquariensis* and a *CYC1* terminator obtained, isolated from a *Debaryomyces macquariensis.* Additionally, the plasmid expression vector contains an expression cassette for antibiotic resistance gene expression. In a preferred embodiment of the invention, the plasmid expression vector contains an expression cassette enabling expression of a gene coding a heterologous protein comprising a *GAP* promoter from the *Debaryomyces macquariensis* strain D50 with a DNA sequence shows in SEQ ID No.: 1 and a *CYC1* terminator from *Debaryomyces macquariensis* D50 with a DNA sequence shows in SEQ ID No.: 4. The invention enables constitutive expression of a heterologous gene - which means production of a heterologous protein in the presented expression system, especially in the recombinant strain of psychrotolerant yeast *Debaryomyces macquariensis* D50.

The gene coding a heterologous protein in expression system is preferably a gene coding a thermolabile protein. The system preferably uses the psychrotolerant *Debaryomyces macquariensis* D50 yeast strain. The plasmid expression vector preferably contains an expression cassette for antibiotic Zeocin resistance gene expression.

In preferred embodiment, the expression cassette for expression of a selection marker, i.e. of an antibiotic resistance gene, preferably antibioticZeocin, present in the plasmid expression vector preferably contains the *IEF2* promoter with a DNA sequence shows in SEQ ID No.: 2, obtained from the psychrotolerant yeast *Debaryomyces macquariensis* strain D50 or the *TEF1* promoter with a DNA sequence shows in SEQ ID No.: 3, obtained from the psychrotolerant yeast *Candida santamariae* strain G12, or the *TEF1* promoter from *Saccharomyces cerevisiae.* The expression cassette enabling expression of a selection marker, i.e. of an antibiotic resistance gene, preferably to Zeocin, present in the plasmid expression vector preferably also contains a *CYC1* terminator with a DNA sequence shows in SEQ ID No.: 5 obtained from a psychrotolerant yeast *Candida santamariae* strain G12 or the *CYC1* terminator obtained from the *Saccharomyces cerevisiae.*

According to the invention, the expression cassette enabling expression of a selection marker, i.e. of an antibiotic resistance gene, preferably to Zeocin, present in the plasmid expression vectors, enables easy identification of recombinant strains of psychrotolerant *Debaryomyces macquariensis* yeast, preferably recombinant strains of the *Debaryomyces macquariensis* D50, on a growth medium containing an antibiotic, preferably Zeocin.

In a preferred embodiment of the invention, plasmid expression vectors are integrated with the *D. macquariensis* D50 genome, in the area of the *GAP* promoter from *Debaryomyces macquariensis* D50 with a DNA sequence shows in SEQ ID No.: 1, thus allowing genetically stable recombinant strains of psychrotolerant yeast *Debaryomyces macquariensis* D50 to be obtained.

The invention also includes plasmid expression vectors of the pH series designated as pH3, pH4, pH5A and pH6, allowing cloning the heterologous gene into plasmid expression vector in each case, and eventually - its constitutive expression in cells of psychrotolerant yeast *Debaryomyces macquariensis,* preferably of the D50 strain.

The pH3 plasmid expression vector with a DNA sequence shows in SEQ ID No. 13 according to the invention is characterised in that, it comprises an expression cassette for expression of a heterologous gene, which contains the *GAP* promoter from *Debaryomyces macquariensis* D50 with a DNA sequence shows in SEQ ID No.: 1 and a *CYC1* terminator from *Debaryomyces macquariensis* D50 with a DNA sequence shows in SEQ ID No.:4, and it also comorises an expression cassette for expression of an antibiotic resistance gene containing the *TEF1* promoter from *Saccharomyces cerevisiae* and the *CYC1* terminator from *Saccharomyces cerevisiae.*

In the preferred embodiment, the heterologous gene is a gene coding a heterologous protein as a thermolabile protein. The plasmid expression vector preferably contains an expression cassette for antibiotic Zeocin resistance gene expression.

The pH4 plasmid expression vector with a DNA sequence shows in SEQ ID No. 17 according to the invention is characterised in that, it comprises an expression cassette for expression of a heterologous gene, which contains the *GAP* promoter from *Debaryomyces macquariensis* D50 with a DNA sequence shows in SEQ ID No.: 1 and a *CYC1* terminator from *Debaryomyces macquariensis* D50 with a DNA sequence shows in SEQ ID No.: 4, and it also comprises an expression cassette for expression of an antibiotic resistance gene containing the *TEF1* promoter from *Candida santamariae* G12 with a DNA sequence shows in SEQ ID No.: 3 and the *CYC1* terminator from *Saccharomyces cerevisiae.*

In the preferred embodiment, the heterologous gene is preferably a gene coding a heterologous protein as a thermolabile protein. The plasmid expression vector preferably contains an expression cassette for antibiotic Zeocin resistance gene expression.

The pH5A plasmid expression vector with a DNA sequence shows in SEQ ID No. 25 according to the invention is characterised in that, it comprises an expression cassette for expression of a heterologous gene, which contains the *GAP* promoter from *Debaryomyces macquariensis* D50 with a DNA sequence shows in SEQ ID No.: 1 and a *CYC1* terminator from *Debaryomyces macquariensis* D50 with a DNA sequence shows in SEQ ID No.: 4, and it also comprises an expression cassette for expression of an antibiotic resistance gene containing the *TEF1* promoter from *Candida santamariae* G12 with a DNA sequence shows in SEQ ID No.: 3 and a *CYC1* terminator from *Candida santamariae* G12 with a DNA sequence shows in SEQ ID No.: 5.

In the preferred embodiment, the heterologous gene is a gene coding a heterologous protein as a thermolabile protein. The plasmid expression vector preferably contains an expression cassette for antibiotic Zeocin resistance gene expression.

The pH6 plasmid expression vector with a DNA sequence shows in SEQ ID No. 29 according to the invention is characterised in that, it comprises an expression cassette for expression of a heterologous gene, which contains the *GAP* promoter from *Debaryomyces macquariensis* D50 with a DNA sequence shows in SEQ ID No.: 1 and a *CYC1* terminator from *Debaryomyces macquariensis* D50 with a DNA sequence shows in SEQ ID No.: 4, and it also comprises an expression cassette for expression of an antibiotic resistance gene containing the *IEF2* promoter from *Debaryomyces macquariensis* D50 with a DNA sequence shows in SEQ ID No.: 2 and the *CYC1* terminator from *Saccharomyces cerevisiae.*

In the preferred embodiment, the heterologous gene is a gene coding a heterologous protein as a thermolabile protein. The plasmid expression vector preferably contains an expression cassette for antibiotic Zeocin resistance gene expression.

The initial vector used to construct the pH series of plasmid expression vectors designated as pH3, pH4, pH5A and pH6 was pGAPZa B, a vector purchased from Invitrogen (USA). Thus, all plasmid expression vectors of the pH series contain: 1) *origin* of plasmid DNA replication ColEl/pMBl/pBR322/pUC ensuring high copy number autonomous replication of recombinant plasmids in *E. coli* bacteria cells, 2) a MCS sequence enabling directional gene cloning, 3) a sequence coding a signal peptide (pre-pro-peptide) of the α-factor from *S. cerevisiae,* 4) a sequence of a synthetic transcription promoter EM7 enabling expression of *Sh ble* (BleoR) and *Sh ble* CpG free (BleoR CpG free) genes in *E. coli* cells, 5) a "6xHis" sequence coding a C-terminal fusion domain, the presence of which in the recombinant protein, a product of a heterologous gene expression, enables protein purification via metal affinity chromatography using a matrix containing nickel ions and 6) a "Myc" sequence coding an epitope of the c-Myc human proto-oncogene, the presence of which in the heterologous gene expression product enables its detection using e.g. Western blotting. The pH series vectors also contain 7) the *Sh ble* (BleoR; pH3, pH4 and pH6 vectors) gene or 8) the *Sh ble* CpG free (BleoR CpG free; pH5A vector) gene, coding a protein ensuring resistance of recombinant *Escherichia coli* and *D*. *macquariensis* D50 to Zeocin. Structural elements of pH series vectors numbered 1-7 were obtaiend from the pGAPZa B vector, while element 8 was obtained from the pMOD2-Zeo plasmid purchased from InvivoGen (USA). At all stages of the construction of pH series plasmid expression vectors and of their derivatives presented in Examples 1-12, known methods of DNA digestion using restriction enzymes, PCR reaction procedures, purification methods of PCR products and of products of DNA digestion using restriction enzymes, known conditions of DNA ligation using the T4 DNA ligase, known conditions of chemical transformation of *E. coli* cells using ligation mixtures were used, as described in literature on molecular DNA cloning.

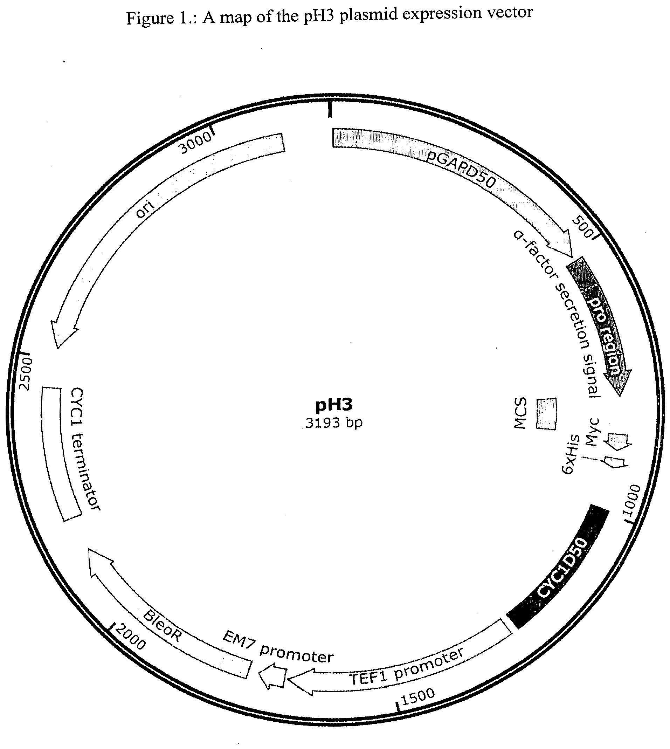

According to an embodiment of the invention, the pH3 plasmid expression vector with a DNA sequence shows in SEQ ID No. 13, contains a nucleotide sequence of the expression cassette enabling expression of a gene coding a heterologous protein, containing a *GAP* promoter from the *Debaryomyces macquariensis* strain D50 with a DNA sequence shows in SEQ ID No.: 1, a DNA sequence coding a signal peptide (pre-pro peptide) of the α-factor from *Saccharomyces cerevisiae,* a DNA sequence forming a multiple cloning site (MCS), a DNA sequence coding an epitope of the c-Myc human proto-oncogene, a DNA sequence coding a peptide composed of 6 histidine residues "6 x His" and the *CYC1* terminator from *Debaryomyces macquariensis* D50 with a DNA sequence shows in SEQ ID No.: 4. Additionally, the plasmid expression vector designated as pH3 according to the invention, with a DNA sequence shows in SEQ ID No.: 13, contains an expression cassette enabling expression of a selection marker composed of a DNA sequence of the *TEF1* promoter from *S. cerevisiae,* a DNA sequence of the synthetic transcription promoter EM7, the *Sh ble* gene of Zeocin resistance (BleoR) and of the *CYC1* terminator from *S. cerevisiae.* The pH3 plasmid expression vector with a DNA sequence shows in SEQ ID No. 13 also contains an *origin* of plasmid DNA replication enabling its autonomous replication in cells of an indirect host *E. coli.* The pH3 plasmid expression vector is presented in Fig. 1. The DNA sequence of the pH3 plasmid expression vector contains a single BspHI restriction site within the DNA sequence of the *GAP* promoter from *D. macquariensis* D50, which enables its linearisation and subsequent integration of DNA of the pH3 vector and of its derivatives, e.g. with a gene coding a heterologous protein, wherein this gene does not contain a BspHI restriction site, with genome of the *D. macquariensis* D50 yeast. It is thus possible to obtain genetically stable recombinant strains of *D. macquariensis* D50 producing heterologous proteins, products of expression of genes cloned into the pH3 plasmid expression vector.

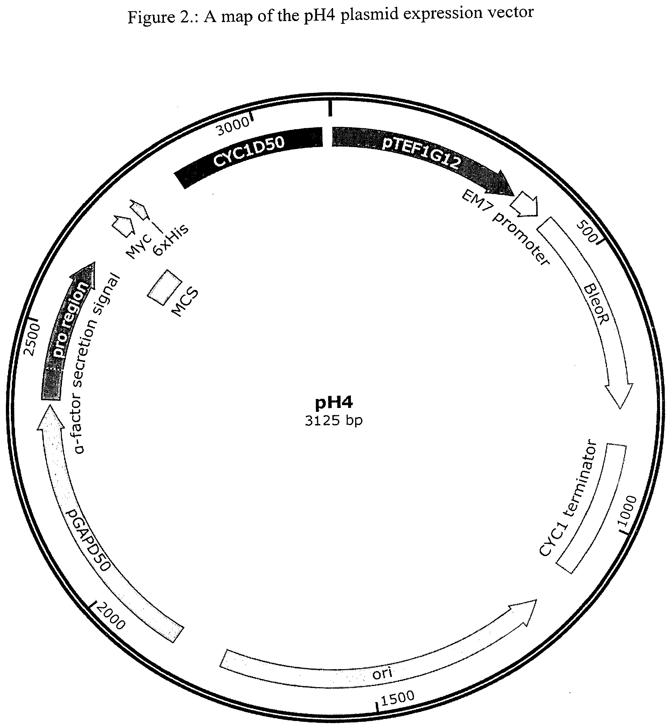

According to an embodiment of the invention, the pH4 plasmid expression vector with a DNA sequence shows in SEQ ID No. 17, contains a nucleotide sequence of the expression cassette enabling expression of a gene coding a heterologous protein, containing a *GAP* promoter from the *Debaryomyces macquariensis* strain D50 with a DNA sequence shows in SEQ ID No.: 1, a DNA sequence coding a signal peptide of the α-factor from *Saccharomyces cerevisiae,* a DNA sequence forming a multiple cloning site (MCS), a DNA sequence coding an epitope of the c-Myc human proto-oncogene, a DNA sequence coding a peptide composed of 6 histidine residues "6xHis" and the *CYC1* terminator from *Debaryomyces macquariensis* D50 with a DNA sequence shows in SEQ ID No.: 4. Additionally, the plasmid expression vector designated as pH4 according to the invention, with a DNA sequence shows in SEQ ID No.: 17, contains an expression cassette enabling expression of a selection marker composed of a DNA sequence of the *TEF1* promoter from the psychrotolerant yeast *Candida santamariae* strain G12 with a DNA sequence shows in SEQ ID No.: 3, a DNA sequence of a synthetic transcription promoter EM7, the *Sh ble* Zeocin resistance gene (BleoR) and the *CYC1* terminator from *S. cerevisiae.* The pH4 plasmid expression vector with a DNA sequence shows in SEQ ID No. 17 also contains an *origin* of plasmid DNA replication enabling its autonomous replication in cells of an indirect host *E. coli.* The pH4 plasmid expression vector is presented in Fig. 2. The DNA sequence of the pH4 plasmid expression vector contains a single BspHI restriction site within the DNA sequence of the *GAP* promoter from *D. macquariensis* D50, which enables its linearisation and subsequent integration of DNA of the pH4 vector and of its derivatives, e.g. with a gene coding a heterologous protein, wherein this gene does not contain a BspHI restriction site, with genome of the *D. macquariensis* D50 yeast. It is thus possible to obtain genetically stable recombinant strains of *D. macquariensis* D50 producing heterologous proteins, products of expression of genes cloned into the pH4 plasmid expression vector.

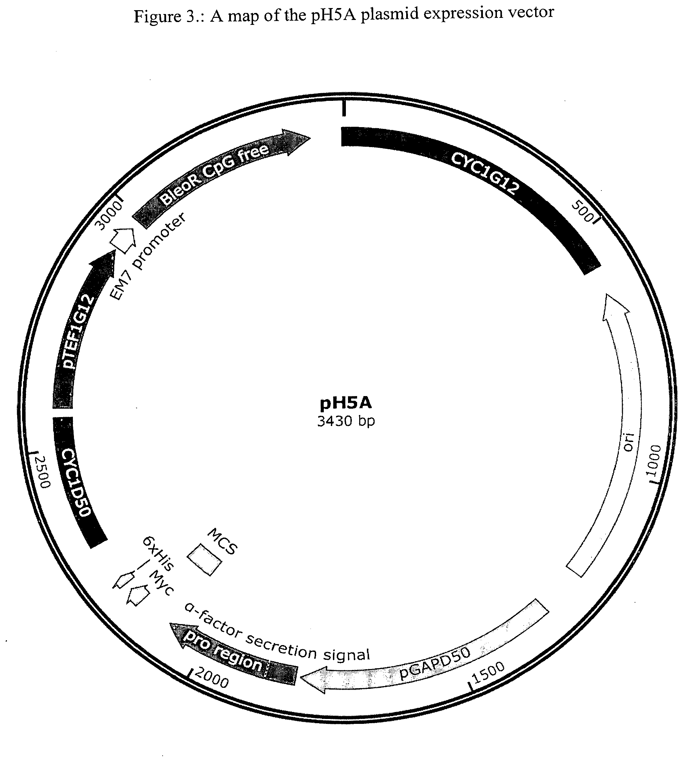

According to an embodiment of the invention, the pH5A plasmid expression vector with a DNA sequence shows in SEQ ID No. 25, contains a nucleotide sequence of the expression cassette enabling expression of a gene coding a heterologous protein, containing a *GAP* promoter from the *Debaryomyces macquariensis* strain D50 with a DNA sequence shows in SEQ ID No.: 1, a DNA sequence coding a signal peptide of the α-factor from *Saccharomyces cerevisiae,* a DNA sequence forming a multiple cloning site (MCS), a DNA sequence coding an epitope of the c-Myc human proto-oncogene, a DNA sequence coding a peptide composed of 6 histidine residues "6xHis" and the *CYC1* terminator from *Debaryomyces macquariensis* D50 with a DNA sequence shows in SEQ ID No.: 4. Additionally, the plasmid expression vector designated as pH5A according to the invention, with a DNA sequence shows in SEQ ID No.: 25, contains an expression cassette enabling expression of a selection marker composed of a DNA sequence of the *TEF1* promoter from the psychrotolerant yeast *Candida santamariae* strain G12 with a sequence shows in SEQ ID No.: 3, a DNA sequence of the synthetic transcription promoter EM7, the *Sh ble* CpG free Zeocin resistance gene (BleoR CpG free) and a sequence of the *CYC1* terminator from the psychrotolerant yeast *Candida santamariae* strain G12 with a sequence shows in SEQ ID No.: 5. The pH5A plasmid expression vector with a DNA sequence shows in SEQ ID No. 25 also contains an *origin* of plasmid DNA replication enabling its autonomous replication in cells of an indirect host *E. coli.* The pH5A plasmid expression vector is presented in Fig. 3. The DNA sequence of the pH5A plasmid expression vector contains a single BspHI restriction site within the DNA sequence of the *GAP* promoter from *D. macquariensis* D50, which enables its linearisation and subsequent integration of DNA of the pH5A vector and of its derivatives, e.g. with a gene coding a heterologous protein, wherein this gene does not contain a BspHI restriction site, with genome of the *D. macquariensis* D50 yeast. It is thus possible to obtain genetically stable recombinant strains of *D. macquariensis* D50 producing heterologous proteins, products of expression of genes cloned into the pH5A plasmid expression vector.

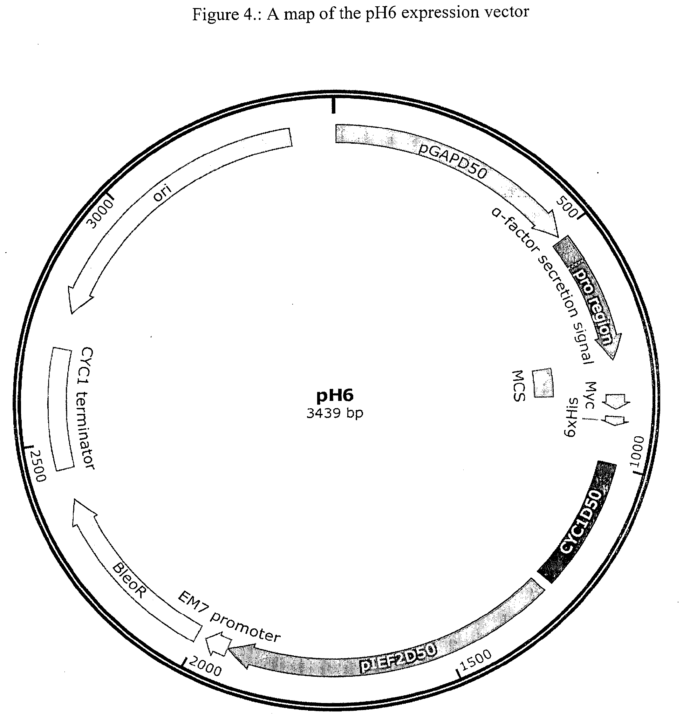

According to an embodiment of the invention, the pH6 plasmid expression vector with a DNA sequence shows in SEQ ID No.: 29, contains a nucleotide sequence of the expression cassette enabling expression of a gene coding a heterologous protein, containing a *GAP* promoter from the *Debaryomyces macquariensis* strain D50 with a DNA sequence shows in SEQ ID No.: 1, a DNA sequence coding a signal peptide of the α-factor from *Saccharomyces cerevisiae,* a DNA sequence forming a multiple cloning site (MCS), a DNA sequence coding an epitope of the c-Myc human proto-oncogene, a DNA sequence coding a peptide composed of 6 histidine residues "6xHis" and the *CYC1* terminator from *Debaryomyces macquariensis* D50 with a DNA sequence shows in SEQ ID No.: 4. Additionally, the plasmid expression vector designated as pH6 according to the invention, with a DNA sequence shows in SEQ ID No.: 29, contains an expression cassette enabling expression of a selection marker composed of a DNA sequence of the *IEF2* promoter from *Debaryomyces macquariensis* D50 with a DNA sequence shows in SEQ ID No.: 2, a DNA sequence of a synthetic transcription promoter EM7, the *Sh ble* Zeocin resistance gene (BleoR) and the *CYC1* terminator from *S. cerevisiae.* The pH6 plasmid expression vector with a DNA sequence shows in SEQ ID No.: 29 also contains an *origin* of plasmid DNA replication enabling its autonomous replication in cells of an indirect host *E. coli.* The pH6 plasmid expression vector is presented in Fig. 4. The DNA sequence of the pH6 plasmid expression vector contains a single BspHI restriction site within the DNA sequence of the *GAP* promoter from *D. macquariensis* D50, which enables its linearisation and subsequent integration of DNA of the pH6 vector and of its derivatives, e.g. with a gene coding a heterologous protein, wherein this gene does not contain a BspHI restriction site, with genome of the *D. macquariensis* D50 yeast. It is thus possible to obtain genetically stable recombinant strains of *D. macquariensis* D50 producing heterologous proteins, products of expression of genes cloned into the pH6 plasmid expression vector.

In accordance with-the invention, a method for construction of a recombinant strain of psychrotolerant yeast *Debaryomyces macquariensis* D50, accession number KKP 2066p, the gene coding a heterologous protein is cloned into the pH plasmid expression vector described above: designated as pH3 or pH4 or pH5A or pH6, wherein the gene is cloned between the *GAP* promoter from *Debaryomyces macquariensis* D50 with a DNA sequence shows in SEQ ID No.: 1 and a *CYC1* terminator from *Debaryomyces macquariensis* D50 with a DNA sequence shows in SEQ ID No.: 4. This step of the method is followed by the step of linearisation of DNA of the obtained expression plasmid in the area of the *GAP* promoter from *Debaryomyces macquariensis* D50 with a DNA sequence shows in SEQ ID No.: 1. Thus obtained DNA is used to transform *Debaryomyces macquariensis* D50 yeast cells via electroporation, followed by yeast cell incubation on a growth medium containing an antibiotic.

In the preferred embodiment of the method, a gene coding the heterologous protein as a thermolabile protein is preferably used.

A growth medium containing Zeocin is preferably used.

The object of the invention is also a method for a heterologous protein production by the recombinant strain of psychrotolerant yeast *Debaryomyces macquariensis* D50. The construction of the recombinant strain is obtained according to the described above methods, wherein a liquid culture of a recombinant yeast strain is maintained within a temperature range between 15 and 25°C.

In the preffered embodiment of the method, the culture is preferably maintained at a temperature of, or close to, 20°C.

In an expression system according to an embodiment of the invention, cloning of a heterologous gene, for example of the β-D-galactosidase *Paracoccus* sp. 32d gene (Wierzbicka-Wos A., Cieśliński H., Wanarska M., Kozlowska-Tylingo K., Hildebrandt P., Kur J. A novel cold-active β-D-galactosidase from the Paracoccus sp. 32d - gene cloning, purification and characterization. Microb. Cell Fact. 2011, 10:108), takes place via ligation of a DNA fragment carrying this gene with plasmid expression vector of a pH-series selected from vectors designated as pH3, pH4, pH5A and pH6, digested with appropriate restriction enzymes, which cleavage sites are located within the multiple cloning site (MCS). The obtained ligation mixture is used to transform *Escherichia coli* cells, e.g. of the *E. coli TOP10* strain from Invitrogen (USA) and a recombinant bacterial strain is obtained, from which DNA of an expression plasmid is subsequently isolated according to the invention, for example pH3Bgal32d or pH4Bgal32d or pH5ABga132d or pH6Bga132d.

According to an embodiment of the invention, an expression plasmid, for example pH3Bgal32d, pH4Bga132d, pH5ABga132d or pH6Bga132d, is linearised within the sequence of the *GAP* promoter from *D. macquariensis* D50 using a selected restriction enzyme, BspHI enzyme according to the invention, and subsequently the linear form of the plasmid DNA is used in electrotransformation of cells of the target host of heterologous gene expression, i.e. of *D. macquariensis* D50 yeast cells. This leads to genetically stable recombinant strains of psychrotolerant yeast *D. macquariensis* D50, as DNA of the expression plasmid becomes integrated with genomic DNA of yeast via homologous recombination.

According to an embodiment of the invention, selection of the obtained recombinant strains of *D. macquariensis* D50 yeast is performed using growth medium containing an antibiotic, Zeocin, as the selection factor. Only recombinant *D. macquariensis* D50 yeasts may grow on such as medium, as they are the only organisms expressing the *Sh ble* or *Sh ble* CpG free gene providing resistance to Zeocin.

In an expression system according to the invention, production of heterologous proteins by recombinant strains of psychrotolerant yeast *D. macquariensis* D50 takes place during cultivation of these strains in the YPD medium (2% peptone K, 1% yeast extract, 2% glucose), or the YD medium (2% yeast extract, 5% glucose), or the YP-TD medium (1% peptone-tryptone, 2% yeast extract, 5% glucose) at 15-25°C, preferably at 20°C.

The invention is explained in detail in the embodiments presented below, in sequences and in the drawings, in which Fig. 1 presents a map of the pH3 plasmid expression vector, Fig. 2 presents a map of the pH4 plasmid expression vector, Fig. 3 presents a map of the pH5A plasmid expression vector, Fig. 4 presents a map of the pH6 expression vector, Fig. 5 presents a map of the pH3Bga132d expression plasmid, Fig. 6 presents a map of the pH4Bgal32d expression plasmid, Fig. 7 presents a map of the pH5ABga132d expression plasmid, Fig. 8 presents a map of the pH6Bga132d expression plasmid, Fig. 9 presents a map of the pGAPzCYCD50bezAOX1 plasmid, and Fig. 10 presents a map of the pH4A plasmid.

### Example 1

### Construction of the pH3 plasmid expression vector

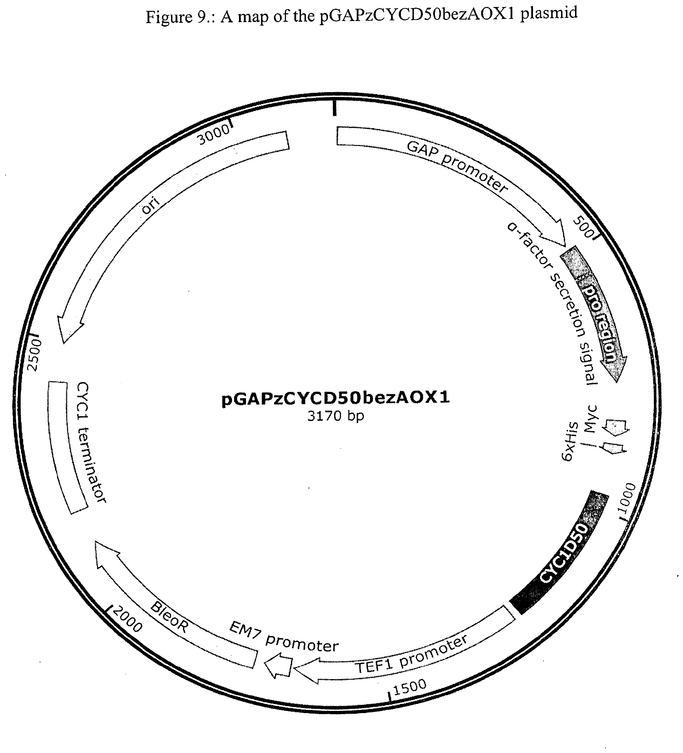

The first stage leading to construction of the pH3 plasmid expression vector is obtaining a PCR product designated as H3.1 and with a DNA sequence shows in SEQ ID No.: 8 containing a sequence of the *CYC1* terminator from *Debaryomyces macquariensis* D50 with a DNA sequence shows in SEQ ID No.: 4, table 1, in order to clone it in the pGAPZα B vector, e.g. purchased from Invitrogen (USA). This stage uses a PCR reaction with a genomic DNA of D. *macquariensis* D50 as template and primers: first designated as CYCD50zaAOX1for with a DNA sequence shows in SEQ ID No.: 6 and second designated as CYCD50zaAOX1rev with a DNA sequence shows in SEQ ID No.: 7. The obtained PCR product is purified using a commercial kit intended for DNA purification after enzymatic reactions. The purified PCR product and DNA of the pGAPZα B plasmid are digested with BamHI and AgeI restriction enzymes, followed by purification via DNA precipitation. The purified DNA fragments of adequate sizes and sequences are then combined in a DNA ligation reaction. The obtained ligation mixture is then used to transform *Escherichia coli* TOP10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This leads to the indirect DNA construct i.e. a plasmid designated as pGAPzCYCD50bezAOX1 with a DNA sequence shows in SEQ ID No.: 9 and with a topological map presented in fig. 9.

The second stage leading to construction of the pH3 plasmid expression vector is obtaining a PCR product designated as H3.2 and with a DNA sequence shows in SEQ ID No.: 12 containing a sequence of the *GAP* promoter from *D. macquariensis* D50 with a DNA sequence shows in SEQ ID No.: 1, table 1. This stage uses a PCR reaction with a genomic DNA of *D. macquariensis* D50 as template and a primer designated as pGsTD50_forward with a DNA sequence shows in SEQ ID No.: 10 and a primer designated pGsTD50_reverse with a DNA sequence shows in SEQ ID No.:ll, enabling cloning of the PCR product into the pGAPzCYCD50bezAOX1 plasmid, at BglII and BstBI restriction sites. The obtained PCR product is purified using a commercial kit intended for DNA purification after enzymatic reactions. The purified PCR product and DNA of the pGAPzCYCD50bezAOX1 plasmid are digested with BglII and BstBI restriction enzymes, and digestion products are subsequently purified by DNA precipitation. The purified DNA fragments of adequate sizes and sequences are then combined in a DNA ligation reaction. The obtained ligation mixture is then used to transform *E. coli* TOP10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This method ultimately leads to a plasmid expression vector designated as pH3 with a DNA sequence shows in SEQ ID No.: 13 and with a topological map presented in Fig. 1.

### Example 2

### Construction of the pH4 plasmid expression vector

The first stage leading to construction of the pH4 plasmid expression vector is obtaining a PCR product designated as H4 and with a DNA sequence shows in SEQ ID No.: 16 containing a sequence of the *TEF1* promoter from the psychrotolerant yeast *Candida santamariae* strain G12 with a DNA sequence shows in SEQ ID No.: 3, table 1, in order to clone it into the pH3 plasmid expression vector. For this purpose, the PCR reaction is carried out with a genomic DNA of C. *santamariae* G12 as template and a primer designated as pTEF1pH4_forward with a DNA sequence shows in SEQ ID No.: 14 and a primer designated as pTEF1pH4_reverse with a DNA sequence shows in SEQ ID No.: 15. The obtained PCR product is purified using a commercial kit intended for DNA purification after enzymatic reactions. The purified PCR product and DNA of the pH3 plasmid expression vector are then digested with BamHI and AseI restriction enzymes, and digestion products are subsequently purified by DNA precipitation. The purified DNA fragments of adequate size and sequences are then combined in a DNA ligation reaction. The obtained ligation mixture is then used to transform *E. coli* T0P10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This method ultimately leads to a plasmid expression vector designated as pH4 with a DNA sequence shows in SEQ ID No.: 17 and with a topological map presented in Fig. 2.

### Example 3

### Construction of the pH5A plasmid expression vector

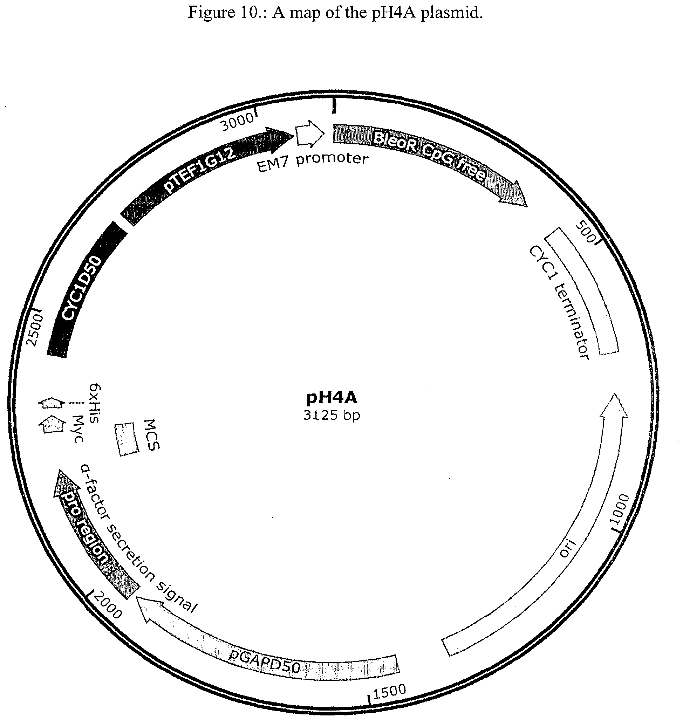

The first stage leading to construction of the pH5A plasmid expression vector is obtaining a PCR product designated as H4.1 and with a DNA sequence shows in of SEQ ID No.: 20 containing the sequence of the *Sh ble* CpG free gene designated as BleoR CpG free on the plasmid map presented in Fig. 10, in order to clone it into the pH4 plasmid expression vector. For this purpose, the PCR reaction is carried out with a DNA template of the p-MOD2-Zeo plasmid, e.g. purchased from InvivoGen (USA) and primers: first designated as BleoRCpGfree_For with a DNA sequence shows in SEQ ID No.: 18 and second designated as BleoRCpGfree_Rev and with a DNA sequence shows in SEQ ID No.: 19. The obtained PCR product is purified using a commercial kit intended for DNA purification after enzymatic reactions. The purified PCR product and DNA of the pH4 plasmid expression vector are digested with NcoI and BseRI restriction enzymes, and digestion products are subsequently purified by DNA precipitation. The purified DNA fragments of adequate size and sequences are then combined in a DNA ligation reaction. The obtained ligation mixture is then used to transform *Escherichia coli* TOP10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This leads to the indirect DNA construct i.e. a plasmid designated as pH4A with a DNA sequence shows in SEQ ID No.: 21 and with a topological map presented in fig. 10.

The second stage leading to construction of the pH5A plasmid expression vector is obtaining a PCR product designated as H5.1 and with a DNA sequence shows in of SEQ ID No.: 24 containing a sequence of the *CYC1* terminator from *Candida santamariae* G12 with a DNA sequence shows in SEQ ID No.: 5, table 1, in order to clone it into the pH4A plasmid. This stage uses a PCR reaction with a genomic DNA of C. *santamariae* G12 as template and primers: first designated as CYClG12_For with a DNA sequence shows in SEQ ID No.: 22 and second designated as CYC1G12_Rev and with a DNA sequence shows in SEQ ID No.: 23 enabling cloning of the PCR product into the pH4A plasmid, at EcoRV and PciI restriction sites. The obtained PCR product is purified using a commercial kit intended for DNA purification after enzymatic reactions. The purified PCR product and DNA of the pH4A plasmid are digested with EcoRV and PciI restriction enzymes, and digestion products are subsequently purified by DNA precipitation. The purified DNA fragments of adequate size and sequences are then combined in a DNA ligation reaction. The obtained ligation mixture is then used to transform *E. coli* TOP10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This method ultimately leads to a plasmid expression vector designated as pH5A with a DNA sequence shows in SEQ ID No.: 25 and with a topological map presented in Fig. 3.

### Example 4

### Construction of the pH6 plasmid expression vector

The first stage leading to construction of the pH6 plasmid expression vector is obtaining a PCR product designated as H6 and with a DNA sequence shows in SEQ ID No.: 28 containing a sequence of the *IEF2* promoter from *Debaryomyces macquariensis* D50 with a DNA sequence shows in SEQ ID No.: 2, table 1, in order to clone it into the pH3 plasmid expression vector. This stage uses a PCR reaction with a genomic DNA *of D. macquariensis* D50 as template and a primer designated as pIEF2pH6_forward with a DNA sequence shows in SEQ ID No.: 26 and a primer designated as pIEF2pH6_reverse with a DNA sequence shows in SEQ ID No.: 27. The obtained PCR product is purified using a commercial kit intended for DNA purification after enzymatic reactions. Next, the purified PCR product and DNA of the pH3 vector are digested with BamHI and AseI restriction enzymes. The obtained digestion products are purified through DNA precipitation. The purified DNA fragments of adequate size and sequences are then combined in a DNA ligation reaction. The obtained ligation mixture is then used to transform *E. coli* T0P10 cells, e.g. purchased from Invitrogen (USA) and recombinant plasmids are isolated from the obtained recombinant bacterial strains. The correct construction of recombinant plasmids is initially verified through restrictive analysis and finally confirmed by DNA sequencing. This method ultimately leads to a plasmid expression vector designated as pH6 with a DNA sequence shows in SEQ ID No.: 29, and with a topological map presented in Fig. 4.

### Example 5

### Construction of a pH3Bga132d expression plasmid

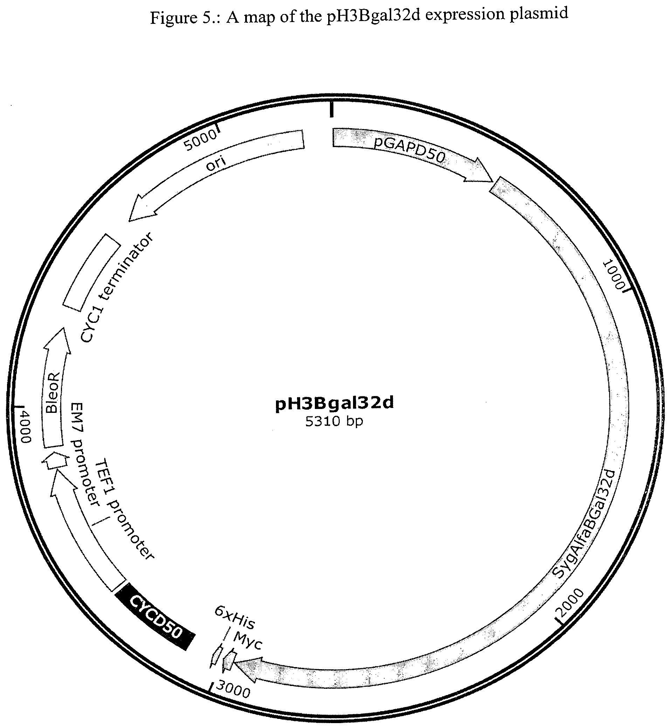

The first stage leading to construction of the pH3Bgal32d expression plasmid is obtaining a PCR product designated as H346_BGal32d and with a DNA sequence shows in SEQ ID No.: 33, containing a DNA sequence coding β-D-galactosidase of *Paracoccus* sp. 32d, in order to clone it into the pH3 plasmid expression vector. This stage uses a PCR reaction with a genomic DNA of *Paracoccus* sp. 32d as template and a primer designated as F32dBgalXho with a DNA sequence shows in SEQ ID No.: 31 and a primer designated as R32dBgalXba with a DNA sequence shows in SEQ ID No.: 32. The obtained PCR product is purified using a commercial kit intended for DNA purification after enzymatic reactions. Next, the purified PCR product and DNA of the pH3 vector are digested with XhoI and XbaI restriction enzymes. DNA digestion products are purified by precipitation. The purified DNA fragments of adequate size and sequences are combined in a DNA ligation reaction. The obtained ligation mixture is then used to transform *E. coli* TOP10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This method ultimately leads to an expression plasmid designated as pH3Bgal32d with a DNA sequence shows in SEQ ID No.: 30 and with a topological map presented in Fig. 5.

### Example 6

### Construction of a pH4Bga132d expression plasmid

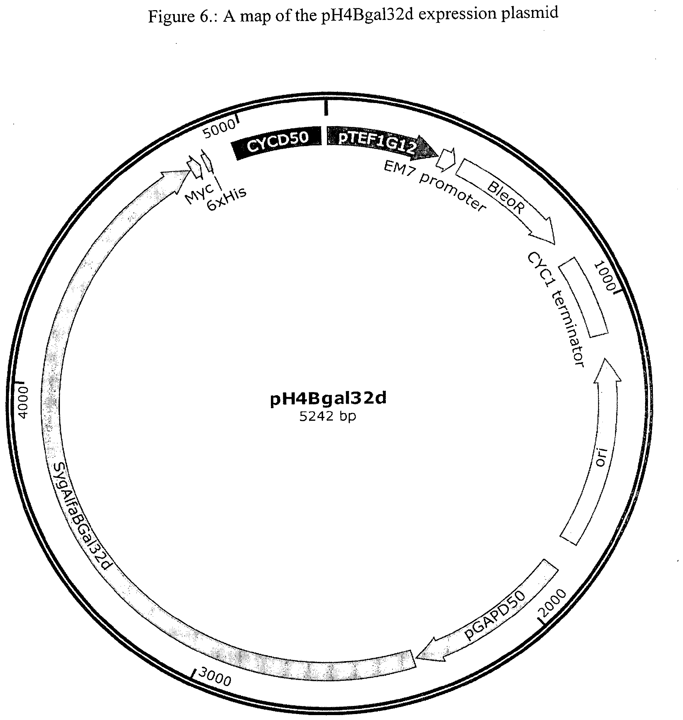

The first stage leading to construction of the pH4Bgal32d expression plasmid is obtaining a PCR product designated as H346_BGa132d and with a DNA sequence shows in SEQ ID No.: 33, containing a DNA sequence coding β-D-galactosidase of *Paracoccus* sp. 32d, in order to clone it into the pH4 plasmid expression vector. This stage uses a PCR reaction with a genomic DNA of *Paracoccus* sp. 32d as template and a primer designated as F32dBgalXho with a DNA sequence shows in SEQ ID No.: 31 and a primer designated as R32dBgalXba with a DNA sequence shows in SEQ ID No.: 32. The obtained PCR product is purified using a commercial kit intended for DNA purification after enzymatic reactions. Next, the purified PCR product and DNA of the pH4 vector are digested with XhoI and XbaI restriction enzymes. DNA digestion products are purified by precipitation. The purified DNA fragments of adequate size and sequences are combined in a DNA ligation reaction. The obtained ligation mixture is then used to transform *E. coli* T0P10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This method ultimately leads to an expression plasmid designated as pH4Bgal32d with a DNA sequence shows in SEQ ID No.: 34 and with a topological map presented in Fig. 6.

### Example 7

### Construction of a pH5ABgal32d expression plasmid

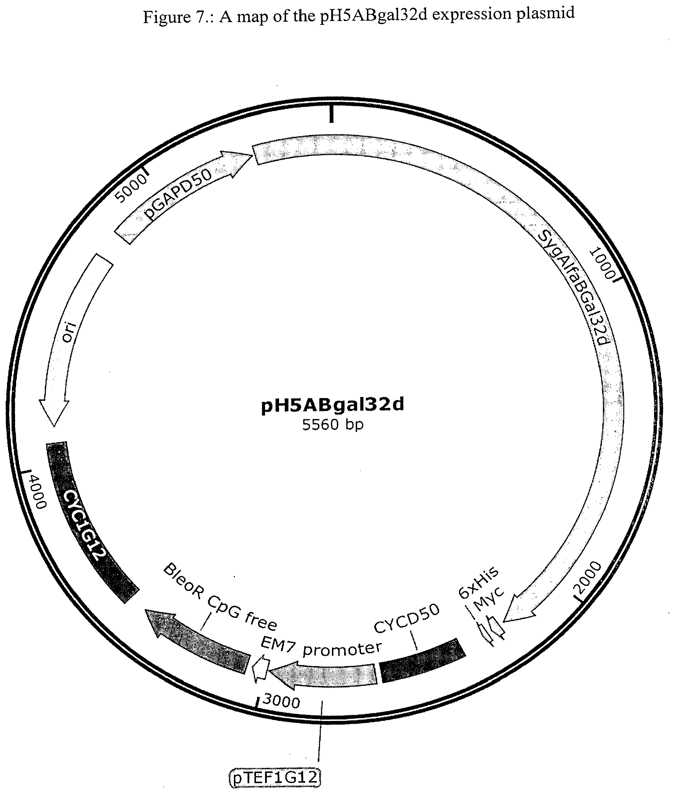

The first stage leading to construction of the pH5ABga132d expression plasmid is obtaining a PCR product designated as H5A_BGa132d and with a DNA sequence shows in SEQ ID No.: 37, containing a DNA sequence coding β-D-galactosidase of *Paracoccus* sp. 32d, in order to clone it into the pH5A plasmid expression vector. This stage uses a PCR reaction with a genomic DNA of *Paracoccus* sp. 32d as template and a primer designated as F32dBgalXho with a DNA sequence shows in SEQ ID No.: 31 and a primer designated as R32dBgalNot with a DNA sequence shows in SEQ ID No.: 36. The obtained PCR product is purified using a commercial kit intended for DNA purification after enzymatic reactions. Next, the purified PCR product and DNA of the pH5A vector are digested with XhoI and NotI restriction enzymes. DNA digestion products are purified by precipitation. The purified DNA fragments of adequate size and sequences are combined in a DNA ligation reaction. The obtained ligation mixture is then used to transform *E. coli* TOP10 cells, e.g. purchased from Invitrogen (USA) and recombinant plasmids are isolated from the obtained recombinant bacterial strains. The correct construction of recombinant plasmids is initially verified through restriction analysis and finally confirmed by DNA sequencing. This method ultimately leads to an expression plasmid designated as pH5ABgal32d with a DNA sequence shows in SEQ ID No.: 35 and with a topological map presented in Fig. 7.

### Example 8

### Construction of a pH6Bga132d expression plasmid

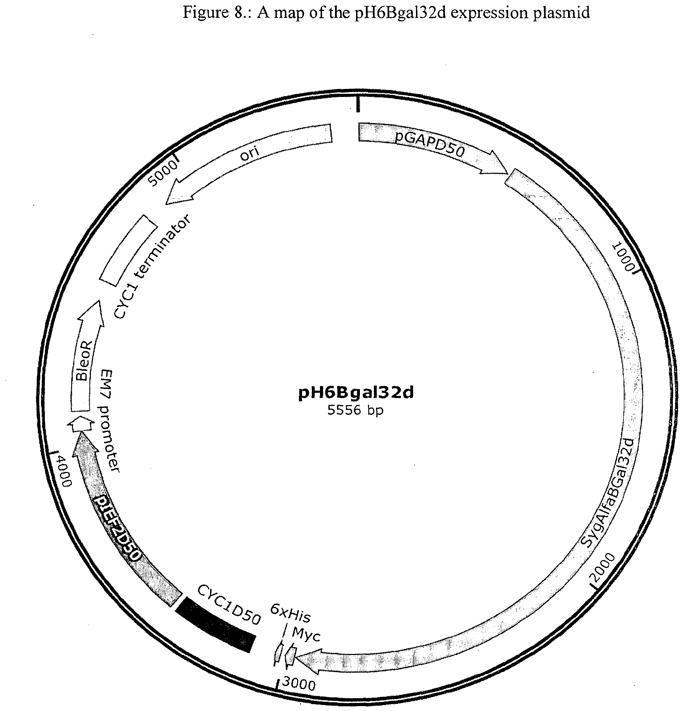

The first stage leading to construction of the pH6Bga132d expression plasmid is obtaining a PCR product designated as H346_BGa132d and with a DNA sequence shows in SEQ ID No.: 33, containing a DNA sequence coding β-D-galactosidase of *Paracoccus* sp. 32d, in order to clone it into the pH6 plasmid expression vector. This stage uses a PCR reaction with a genomic DNA of *Paracoccus* sp. 32d as template and a primer designated as F32dBgalXho with a DNA sequence shows in SEQ ID No.: 31 and a primer designated as R32dBgalXba with a DNA sequence shows in SEQ ID No.: 32. The obtained PCR product is purified using a commercial kit intended for DNA purification after enzymatic reactions. Next, the purified PCR product and DNA of the pH6 vector are digested with XhoI and XbaI restriction enzymes. DNA digestion products are purified by precipitation. The purified DNA fragments of adequate size and sequences are combined in a DNA ligation reaction. The obtained ligation mixture is then used to transform *E. coli* T0P10 cells, e.g. purchased from Invitrogen USA and recombinant plasmids are isolated from the obtained recombinant bacterial strains. The correct construction of recombinant plasmids is initially verified through restriction analysis and finally confirmed by DNA sequencing. This method ultimately leads to an expression plasmid designatd as pH6Bgal32d with a DNA sequence shows in SEQ ID No.: 38 and with a topological map presented in Fig. 8.

### Example 9

Construction of a recombinant yeast strain *D. macquariensis* D50/pH3Bga132d and confirmation of β-D-galactosidase *Paracoccus* sp. 32d activity in cells of obtained yeast and a protein production method using the expression system according to the invention.

DNA of the pH3Bga132d expression plasmid is linearised by digestion with BspHI restriction enzyme within the *GAP* promoter from *D. macquariensis* D50, followed by purification through precipitation. Linear, purified DNA of the expression plasmid is concentrated to a concentration of 1 µg/µl and used in electrotransformation of competent yeast cells of *D. macquariensis* D50.

In order to obtain competent cells, a single colony of *D. macquariensis* D50 yeast is used to inoculate 10 ml of the YPD medium (2% peptone K, 1% yeast extract, 2% glucose). The culture is maintained for 22-24 h at 25-30°C, with shaking (180 rpm). Next, a 7-30 µl sample of yeast culture in a stationary phase is collected and used to inoculate 200 ml of sterile YPD medium. The culture is then maintained at 25-30°C with shaking (180 rpm) for 12.5-13 h, until the logarithmic culture growth phase is achieved at a concentration of 5 x 10⁷ cells/ml (OD₆₀₀ = 1). The culture is centrifuged for 4 minutes at 1500 x g at room temperature. The supernatant is carefully decanted and the yeast cell pellet is washed twice with 100 ml of sterile, deionised water. The pellet is resuspended in 20 ml of a solution containing 35 mM dithiothreitol and 100 mM lithium acetate, followed by incubation for 45 minutes to 1 hour at 25-30°C with gently shaking (80 rpm). After this time, yeast cells are centrifuged for 4 minutes at 1500 x g at 4°C. From this point, the cells and all used reagents should be kept at a temperature near 0°C. The pellet is washed once with 20 ml of sterile, deionised water, followed by washing twice using 20 ml of 1 M sorbitol, with a 4 minutes centrifugation at 1500 x g at 4°C after every stage. The pellet has been resuspended in 1 M sorbitol for the final volume of 500 µ1, and the cells are subsequently divided into 10 tubes 50 µ1 each. Thus prepared cells are ready for electrotransformation.

In order to perform the electrotransformation, 50 µl of competent *D. macquariensis* D50 cells are mixed with 5 µ1 DNA (concentration 1 µg/µl), suspended in sterile, deionised water and gently mixed with a pipette, followed by incubation in ice, at ca. 0°C, for 10 minutes. The sample is then transferred to an ice-cold 0.2 cm electroporation cuvette. Then, the cuvette is placed in an electroporator and a pulse of 1.8 kV is applied for 5 ms. 1 ml of a solution made of 1 M sorbitol and YPD medium mixed at a 1:1 ratio is added to the cuvette immediately after the pulse. The yeast cells are then incubated at room temperature for 20 minutes, without shaking. After this time, the cuvette content is transferred to a 50 ml tube and mixed with 10 ml of the YPD medium containing 5% glucose (2% peptone K, 1% yeast extract, 5% glucose) and incubated at 25-30°C for 18-20 h, with shaking (180 rpm). After this time, cells are centrifuged for 10 minutes at 963 x g in order to reduce the sample volume to 1 ml, followed by spreading on the YPDS (2% peptone K, 1% yeast extract, 2% glucose, 2% agar, 1 M sorbitol) plates containing Zeocin (25 µg/ml) and X-Gal (5-bromo-4-chloro-3-indolyl-β-D-galactopyranoside, 20 µg/ml). After 7 days of incubation at 25-30°C, plates are analysed for colony staining. Thanks to the activity of β-D-galactosidase of *Paracoccus* sp. 32d, colonies of the recombinant *D. macquariensis* D50/pH3Bga132d strain hydrolyse X-Gal present in the growth medium and are stained blue. Blue colour of the colony is a confirmation of expression of a gene coding β-D-galactosidase of *Paracoccus* sp. 32d in host cells - psychrotolerant yeasts *D. macquariensis* D50. This result also confirms occurrence of correct integration of the linear form of pH3Bga132d expression plasmid DNA with the yeast genome, via homologous recombination of corresponding fragments of the *GAP* promoter from *D. macquariensis* D50.

Colonies of recombinant *D. macquariensis* D50/pH3Bga132d yeasts are picked on the YPD (2% peptone K, 1% yeast extract, 2% glucose, 2% agar) plates with Zeocin (25 µg/ml) and X-Gal (20 µg/ml) and incubated for 3 days at 25°C, followed by storage in a refrigerator (4-8°C).

In order to obtain protein the β-D-galactosidase of *Paracoccus* sp. 32d according to the invention, the previous stage is followed by a liquid culture of recombinant yeasts under conditions optimal for growth of *D. macquariensis* D50, in the YP-TD medium (1% peptone-tryptone, 2% yeast extract, 5% glucose) at pH 5.5, temperature of 20°C, aeration rate of 1.5 vvm and stirring speed of 300 rpm.

### Example 10

Construction of a recombinant yeast strain *D. macquariensis* D50/pH4Bga132d and confirmation of β-D-galactosidase *Paracoccus* sp. 32d activity in cells of obtained yeast and a protein production method using the expression system according to the invention.

DNA of the pH4Bga132d expression plasmid is linearised by digestion with BspHI restriction enzyme within the *GAP* promoter from *D. macquariensis* D50, followed by purification through precipitation. Linear, purified DNA of the expression plasmid is concentrated to a concentration of 1 µg/µl and used in electrotransformation of competent yeast cells of *D. macquariensis* D50.

In order to obtain competent cells, a single colony of *D. macquariensis* D50 yeast is used to inoculate 10 ml of the YPD medium (2% peptone K, 1% yeast extract, 2% glucose). The culture is maintained for 22-24 h at 25-30°C, with shaking (180 rpm). Next, a 7-30 µl sample of yeast culture in a stationary phase is collected and used to inoculate 200 ml of sterile YPD medium. The culture is then maintained at 25-30°C with shaking (180 rpm) for 12.5-13 h, until the logarithmic culture growth phase is achieved at a concentration of 5 x 10⁷ cells/ml (OD₆₀₀ = 1). The culture is centrifuged for 4 minutes at 1500 x g at room temperature. The supernatant is carefully decanted and the yeast cell pellet is washed twice with 100 ml of sterile, deionised water. The pellet is resuspended in 20 ml of a solution containing 35 mM dithiothreitol and 100 mM lithium acetate, followed by incubation for 45 minutes to 1 hour at 25-30°C with gently shaking (80 rpm). After this time, yeast cells are centrifuged for 4 minutes at 1500 x g at 4°C. From this point, the cells and all used reagents should be kept at a temperature near 0°C. The pellet is washed once with 20 ml of sterile, deionised water, followed by washing twice using 20 ml of 1 M sorbitol, with a 4 minutes centrifugation at 1500 x g at 4°C after every stage. The pellet has been resuspended in 1 M sorbitol for the final volume of 500 µl, and the cells are subsequently divided into 10 tubes 50 µl each. Thus prepared cells are ready for electrotransformation.

In order to perform the electrotransformation, 50 µl of competent *D. macquariensis* D50 cells are mixed with 5 µl DNA (concentration 1 µg/µl), suspended in sterile, deionised water and gently mixed with a pipette, followed by incubation in ice, at ca. 0°C, for 10 minutes. The sample is then transferred to an ice-cold 0.2 cm electroporation cuvette. Then, the cuvette is placed in an electroporator and a pulse of 1.8 kV is applied for 5 ms. 1 ml of a solution made of 1 M sorbitol and YPD medium mixed at a 1:1 ratio is added to the cuvette immediately after the pulse. The yeast cells are then incubated at room temperature for 20 minutes, without shaking. After this time, the cuvette content is transferred to a 50 ml tube and mixed with 10 ml of the YPD medium containing 5% glucose (2% peptone K, 1% yeast extract, 5% glucose) and incubated at 25-30°C for 18-20 h, with shaking (180 rpm). After this time, cells are centrifuged for 10 minutes at 963 x g in order to reduce the sample volume to 1 ml, followed by spreading on the YPDS (2% peptone K, 1% yeast extract, 2% glucose, 2% agar, 1 M sorbitol) plates containing Zeocin (25 µg/ml) and X-Gal (5-bromo-4-chloro-3-indolyl-β-D-galactopyranoside, 20 µg/ml). After 7 days of incubation at 25-30°C, plates are analysed for colony staining. Thanks to the activity of β-D-galactosidase of *Paracoccus* sp. 32d, colonies of the recombinant *D. macquariensis* D50/pH4Bgal32d strain hydrolyse X-Gal present in the growth medium and are stained blue. Blue colour of the colony is a confirmation of expression of a gene coding β-D-galactosidase of *Paracoccus* sp. 32d in host cells - psychrotolerant yeasts *D. macquariensis* D50. This result also confirms occurrence of correct integration of the linear form of pH4Bga132d expression plasmid DNA with the yeast genome, via homologous recombination of corresponding fragments of the *GAP* promoter from *D. macquariensis* D50.

Colonies of recombinant *D. macquariensis* D50/pH4Bga132d yeasts are picked on the YPD (2% peptone K, 1% yeast extract, 2% glucose, 2% agar) plates with Zeocin (25 µg/ml) and X-Gal (20 (j.g/ml) and incubated for 3 days at 25°C, followed by storage in a refrigerator (4-8°C).

In order to obtain protein the β-D-galactosidase of *Paracoccus* sp. 32d according to the invention, the previous stage is followed by a liquid culture of recombinant yeasts under conditions optimal for growth of *D. macquariensis* D50, in the YP-TD medium (1% peptone-tryptone, 2% yeast extract, 5% glucose) at pH 5.5, temperature of 20°C, aeration rate of 1.5 vvm and stirring speed of 300 rpm.

### Example 11

Construction of a recombinant yeast strain *D. macquariensis* D50/pH5ABga132d and confirmation of β-D-galactosidase *Paracoccus* sp. 32d activity in cells of obtained yeast and a protein production method using the expression system according to the invention.

DNA of the pH5ABga132d expression plasmid is linearised by digestion with BspHI restriction enzyme within the *GAP* promoter from *D. macquariensis* D50, followed by purification through precipitation. Linear, purified DNA of the expression plasmid is concentrated to a concentration of 1 µg/µl and used in electrotransformation of competent yeast cells of *D. macquariensis* D50.

In order to obtain competent cells, a single colony of *D. macquariensis* D50 yeast is used to inoculate 10 ml of the YPD medium (2% peptone K, 1% yeast extract, 2% glucose). The culture is maintained for 22-24 h at 25-30°C, with shaking (180 rpm). Next, a 7-30 µl sample of yeast culture in a stationary phase is collected and used to inoculate 200 ml of sterile YPD medium. The culture is then maintained at 25-30°C with shaking (180 rpm) for 12.5-13 h, until the logarithmic culture growth phase is achieved at a concentration of 5 x 10⁷ cells/ml (OD₆₀₀ = 1). The culture is centrifuged for 4 minutes at 1500 x g at room temperature. The supernatant is carefully decanted and the yeast cell pellet is washed twice with 100 ml of sterile, deionised water. The pellet is resuspended in 20 ml of a solution containing 35 mM dithiothreitol and 100 mM lithium acetate, followed by incubation for 45 minutes to 1 hour at 25-30°C with gently shaking (80 rpm). After this time, yeast cells are centrifuged for 4 minutes at 1500 x g at 4°C. From this point, the cells and all used reagents should be kept at a temperature near 0°C. The pellet is washed once with 20 ml of sterile, deionised water, followed by washing twice using 20 ml of 1 M sorbitol, with a 4 minutes centrifugation at 1500 x g at 4°C after every stage. The pellet has been resuspended in 1 M sorbitol for the final volume of 500 µ1, and the cells are subsequently divided into 10 tubes 50 µ1 each. Thus prepared cells are ready for electrotransformation.

In order to perform the electrotransformation, 50 µ1 of competent *D. macquariensis* D50 cells are mixed with 5 µ1 DNA (concentration 1 µg/µl), suspended in sterile, deionised water and gently mixed with a pipette, followed by incubation in ice, at ca. 0°C, for 10 minutes. The sample is then transferred to an ice-cold 0.2 cm electroporation cuvette. Then, the cuvette is placed in an electroporator and a pulse of 1.8 kV is applied for 5 ms. 1 ml of a solution made of 1 M sorbitol and YPD medium mixed at a 1:1 ratio is added to the cuvette immediately after the pulse. The yeast cells are then incubated at room temperature for 20 minutes, without shaking. After this time, the cuvette content is transferred to a 50 ml tube and mixed with 10 ml of the YPD medium containing 5% glucose (2% peptone K, 1% yeast extract, 5% glucose) and incubated at 25-30°C for 18-20 h, with shaking (180 rpm). After this time, cells are centrifuged for 10 minutes at 963 x g in order to reduce the sample volume to 1 ml, followed by spreading on the YPDS (2% peptone K, 1% yeast extract, 2% glucose, 2% agar, 1 M sorbitol) plates containing Zeocin (25 ug/ml) and X-Gal (5-bromo-4-chloro-3-indolyl-p-D-galactopyranoside, 20 µg/ml). After 7 days of incubation at 25-30°C, plates are analysed for colony staining. Thanks to the activity of β-D-galactosidase of *Paracoccus* sp. 32d, colonies of the recombinant *D. macquariensis* D50/pH5ABgal32d strain hydrolyse X-Gal present in the growth medium and are stained blue. Blue colour of the colony is a confirmation of expression of a gene coding β-D-galactosidase of *Paracoccus* sp. 32d in host cells - psychrotolerant yeasts *D. macquariensis* D50. This result also confirms occurrence of correct integration of the linear form of pH5ABga132d expression plasmid DNA with the yeast genome, via homologous recombination of corresponding fragments of the *GAP* promoter from *D. macquariensis D50.*

Colonies of recombinant *D. macquariensis* D50/pHSABga132d yeasts are picked on the YPD (2% peptone K, 1% yeast extract, 2% glucose, 2% agar) plates with Zeocin (25 µg/ml) and X-Gal (20 µg/ml) and incubated for 3 days at 25°C, followed by storage in a refrigerator (4-8°C).

In order to obtain protein the β-D-galactosidase of *Paracoccus* sp. 32d according to the invention, the previous stage is followed by a liquid culture of recombinant yeasts under conditions optimal for growth of *D. macquariensis* D50, in the YP-TD medium (1% peptone-tryptone, 2% yeast extract, 5% glucose) at pH 5.5, temperature of 20°C, aeration rate of 1.5 vvm and stirring speed of 300 rpm.

### Example 12

Construction of a recombinant yeast strain *D. macquariensis* D50/pH6Bga132d and confirmation of β-D-galactosidase *Paracoccus* sp. 32d activity in cells of obtained yeast and a protein production method using the expression system according to the invention.

DNA of the pH6Bgal32d expression plasmid is linearised by digestion with BspHI restriction enzyme within the *GAP* promoter from *D. macquariensis* D50, followed by purification through precipitation. Linear, purified DNA of the expression plasmid is concentrated to a concentration of 1 µg/µl and used in electrotransformation of competent yeast cells of *D. macquariensis* D50.

In order to obtain competent cells, a single colony of *D. macquariensis* D50 yeast is used to inoculate 10 ml of the YPD medium (2% peptone K, 1% yeast extract, 2% glucose). The culture is maintained for 22-24 h at 25-30°C, with shaking (180 rpm). Next, a 7-30 µl sample of yeast culture in a stationary phase is collected and used to inoculate 200 ml of sterile YPD medium. The culture is then maintained at 25-30°C with shaking (180 rpm) for 12.5-13 h, until the logarithmic culture growth phase is achieved at a concentration of 5 x 10⁷ cells/ml (OD₆₀₀ = 1). The culture is centrifuged for 4 minutes at 1500 x g at room temperature. The supernatant is carefully decanted and the yeast cell pellet is washed twice with 100 ml of sterile, deionised water. The pellet is resuspended in 20 ml of a solution containing 35 mM dithiothreitol and 100 mM lithium acetate, followed by incubation for 45 minutes to 1 hour at 25-30°C with gently shaking (80 rpm). After this time, yeast cells are centrifuged for 4 minutes at 1500 x g at 4°C. From this point, the cells and all used reagents should be kept at a temperature near 0°C. The pellet is washed once with 20 ml of sterile, deionised water, followed by washing twice using 20 ml of 1 M sorbitol, with a 4 minutes centrifugation at 1500 x g at 4°C after every stage. The pellet has been resuspended in 1 M sorbitol for the final volume of 500 µl, and the cells are subsequently divided into 10 tubes 50 µ1 each. Thus prepared cells are ready for electrotransformation.

In order to perform the electrotransformation, 50 µ1 of competent *D. macquariensis* D50 cells are mixed with 5 µ1 DNA (concentration 1 µg/µ1), suspended in sterile, deionised water and gently mixed with a pipette, followed by incubation in ice, at ca. 0°C, for 10 minutes. The sample is then transferred to an ice-cold 0.2 cm electroporation cuvette. Then, the cuvette is placed in an electroporator and a pulse of 1.8 kV is applied for 5 ms. 1 ml of a solution made of 1 M sorbitol and YPD medium mixed at a 1:1 ratio is added to the cuvette immediately after the pulse. The yeast cells are then incubated at room temperature for 20 minutes, without shaking. After this time, the cuvette content is transferred to a 50 ml tube and mixed with 10 ml of the YPD medium containing 5% glucose (2% peptone K, 1% yeast extract, 5% glucose) and incubated at 25-3 0°C for 18-20 h, with shaking (180 rpm). After this time, cells are centrifuged for 10 minutes at 963 x g in order to reduce the sample volume to 1 ml, followed by spreading on the YPDS (2% peptone K, 1% yeast extract, 2% glucose, 2% agar, 1 M sorbitol) plates containing Zeocin (25 µg/ml) and X-Gal (5-bromo-4-chloro-3-indolyl-β-D-galactopyranosidc, 20 µg/ml). After 7 days of incubation at 25-30°C, plates are analysed for colony staining. Thanks to the activity of β-D-galactosidase of *Paracoccus* sp. 32d, colonies of the recombinant *D. macquariensis* D50/pH6Bga132d strain hydrolyse X-Gal present in the growth medium and are stained blue. Blue colour of the colony is a confirmation of expression of a gene coding β-D-galactosidase of *Paracoccus* sp. 32d in host cells - psychrotolerant yeasts *D. macquariensis* D50. This result also confirms occurrence of correct integration of the linear form of pH6Bgal32d expression plasmid DNA with the yeast genome, via homologous recombination of corresponding fragments of the *GAP* promoter from *D. macquariensis* D50.

Colonies of recombinant *D. macquariensis* D50/pH6Bgal32d yeasts are picked on the YPD (2% peptone K, 1% yeast extract, 2% glucose, 2% agar) plates with Zeocin (25 µg/ml) and X-Gal (20 µg/ml) and incubated for 3 days at 25°C, followed by storage in a refrigerator (4-8°C).

In order to obtain protein the β-D-galactosidase of *Paracoccus* sp. 32d according to the invention, the previous stage is followed by a liquid culture of recombinant yeasts under conditions optimal for growth of *D. macquariensis* D50, in the YP-TD medium (1% peptone-tryptone, 2% yeast extract, 5% glucose) at pH 5.5, temperature of 20°C, aeration rate of 1.5 vvm and stirring speed of 300 rpm.

**Table 1. List of sequences of promoters and terminators obtained from Debaryomyces macquariensis D50 and Candida santamariae G12 with their description and function**

| Sequence | Description | Function |
|---|---|---|
| pGAPD50 | Transcription promoter of a glyceraldehyde-3-phosphate dehydrogenase gene of *D*. *macquariensis* D50 SEQ ID No.: 1 | Enables constitutive expression of a heterologous gene in host cells |
| pIEF2D50 | Transcription promoter of a gene coding an isoform of a translation elongation factor 1-α of *D. macquariensis* D50 SEQ ID No.: 2 | Enables constitutive expression of an antiobiotic resistance gene in host cells |
| pTEF1G12 | Transcription promoter of a gene coding a translation elongation factor 1-α of C. *santamariae* G12 SEQ ID No.: 3 | Enables constitutive expression of an antiobiotic resistance gene in host cells |
| CYC1D50 | Transcription terminator of a gene coding isoform 1 of cytochrome C of *D. macquariensis* D50 SEQ ID No.: 4 | Enables transcription termination for a heterologous gene in host cells |
| CYC1G12 | Transcription terminator of a gene coding isoform 1 of cytochrome C of C. *santamariae* G12 SEQ ID No.: 5 | Enables transcription termination for an antibiotic resistance gene in host cells |

## Claims

1. Expression system for production of a heterologous protein, wherein a host for cloning of a gene coding heterologous protein is a yeast strain **characterised in that**, the expression system comprises a psychrotolerant *Debaryomyces macquariensis* yeast strain and a plasmid expression vector comprising an expression cassette for expression of a heterologous gene coding a heterologous protein that contains a *GAP* promoter isolated from a *Debaryomyces macquariensis* and a *CYC1* terminator isolated from a *Debaryomyces macquariensis,* and the plasmid expression vector comprises also an expression cassette for expression of an antibiotic resistance gene.

2. The expression system according to the claim 1, wherein the gene coding a heterologous protein in the expression system is a gene coding a thermolabile protein.

3. The expression system according to the claim 1, wherein the plasmid expression vector comprises the expression cassette for expression of an antibiotic Zeocin resistance gene.

4. The expression system according to one of the claims 1-3, wherein as the yeast strain a psychrotolerant *Debaryomyces macquariensis* strain D50 is used, accession number KKP 2066p.

5. The expression system according to one of the claims 1-4, wherein the plasmid expression vector comprises the expression cassette for expression of a gene coding a heterologous protein that contains the *GAP* promoter from the *Debaryomyces macquariensis* strain D50, accession number KKP 2066p, with a DNA sequence shows in SEQ ID No.: 1 and the *CYC1* terminator from *Debaryomyces macquariensis* strain D50, accession number KKP 2066p, with a DNA sequence shows in SEQ ID No.: 4.

6. The expression system according to one of the claims 1-5, wherein the expression cassette for expression of an antibiotic resistance gene comprising an *IEF2* promoter from *Debaryomyces macquariensis* strain D50, accession number KKP 2066p, with a DNA sequence shows in SEQ ID No.: 2, or the *TEF1* promoter from a psychrotolerant yeast *Candida santamariae* strain G12, with a DNA sequence shows in SEQ ID No.: 3, or the *TEF1* promoter from *Saccharomyces cerevisiae,* and the expression cassette contains a *CYC1* terminator from the yeast *Candida santamariae* strain G12, with a DNA sequence shows in SEQ ID No.: 5 or the *CYC1* terminator from *Saccharomyces cerevisiae.*

7. Plasmid expression vector pH3 with a DNA sequence shows in SEQ ID No. 13, **characterised in that** the vector comprises an expression cassette for expression of a heterologous gene containing the *GAP* promoter from the *Debaryomyces macquariensis* strain D50, accession number KKP 2066p, with a DNA sequence shows in SEQ ID No.: 1 and a *CYC1* terminator from the *Debaryomyces macquariensis* strain D50, accession number KKP 2066p, with a DNA sequence shows in SEQ ID No.: 4, and the plasmid also comprises an expression cassette for expression of an antibiotic resistance gene containing the *TEF1* promoter from *Saccharomyces cerevisiae* and the *CYC1* terminator from *Saccharomyces cerevisiae.*

8. Plasmid expression vector according to the claim 7, wherein the heterologous gene is a gene coding a thermolabile protein.

9. Plasmid expression vector according to the claim 7, wherein the plasmid expression vector contains the expression cassette for antibiotic Zeocin resistance gene expression.

10. Plasmid expression vector pH4 with a DNA sequence shows in SEQ ID No. 17, **characterised in that**, the plasmid comprises an expression cassette for expression of a heterologous gene containing the *GAP* promoter from the *Debaryomyces macquariensis* strain D50, accession number KKP 2066p, with a DNA sequence shows in SEQ ID No.: 1 and a *CYC1* terminator from the *Debaryomyces macquariensis* strain D50, accession number KKP 2066p, with a DNA sequence shows in SEQ ID No.: 4, and the plasmid also comprises an expression cassette for expression of an antibiotic resistance gene containing the *TEF1* promoter, from the *Candida santamariae* strain G12, with a DNA sequence shows in SEQ ID No.: 3 and the *CYC1* terminator from *Saccharomyces cerevisiae.*

11. Plasmid expression vector according to the claim 10, wherein the heterologous gene is a gene coding a thermolabile protein.

12. Plasmid expression vector according to the claim 10, wherein the plasmid expression vector contains the expression cassette for antibiotic Zeocin resistance gene expression.

13. Plasmid expression vector pH5A with a DNA sequence shows in SEQ ID No. 25, **characterised in that**, the plasmid comprises an expression cassette for expression of a heterologous gene containing the *GAP* promoter from the *Debaryomyces macquariensis* strain D50, accession number KKP 2066p, with a DNA sequence shows in SEQ ID No.: 1 and a *CYC1* terminator from the *Debaryomyces macquariensis* strain D50, accession number KKP 2066p, with a DNA sequence shows in SEQ ID No.: 4, and the plasmid also comprises an expression cassette for expression of an antibiotic resistance gene containing the *TEF1* promoter from the *Candida santamariae* strain G12 with a DNA sequence shows in SEQ ID No.: 3 and a *CYC1* terminator from the *Candida santamariae* strain G12 with a DNA sequence shows in SEQ ID No.: 5.

14. Plasmid expression vector according to the claim 13, wherein the heterologous gene is a gene coding a thermolabile protein.

15. Plasmid expression vector according to the claim 13, wherein the plasmid expression vector contains the expression cassette for antibiotic Zeocin resistance gene expression.

16. Plasmid expression vector pH6 with a DNA sequence shows in SEQ ID No. 29, **characterised in that** the plasmid comprises an expression cassette for expression of a heterologous gene containing the *GAP* promoter, from the *Debaryomyces macquariensis* strain D50, accession number KKP 2066p, with a DNA sequence shows in SEQ ID No.: 1 and a *CYC1* terminator, from the *Debaryomyces macquariensis* strain D50, accession number KKP 2066p, with a DNA sequence shows in SEQ ID No.: 4, and the plasmid also comprises an expression cassette for expression of an antibiotic resistance gene containing the *IEF2* promoter, from the *Debaryomyces macquariensis* strain D50, accession number KKP 2066p, with a DNA sequence shows in SEQ ID No.: 2 and the *CYC1* terminator from *Saccharomyces cerevisiae.*

17. Plasmid expression vector according to the claim 16, wherein the heterologous gene is a gene coding a thermolabile protein.

18. Plasmid expression vector according to the claim 16, wherein the plasmid expression vector contains the expression cassette for antibiotic Zeocin resistance gene expression.

19. Method of construction of a recombinant strain of psychrotolerant yeast *Debaryomyces macquariensis* D50, accession number KKP 2066p comprising:
- the step of cloning of a gene coding a heterologous protein into the pH plasmid expression vector described according to one of the claims 7-18, wherein the gene is cloned between the *GAP* promoter from the *Debaryomyces macquariensis* strain D50, accession number KKP 2066p, with a DNA sequence shows in SEQ ID No.: 1 and the *CYC1* terminator from the *Debaryomyces macquariensis* strain D50, accession number KKP 2066p, with a DNA sequence shows in SEQ ID No.: 4,
- the step of linearisation of the expression plasmid obtained according to the step above, wherein the linearisation is performed within the *GAP* promoter from the *Debaryomyces macquariensis* strain D50 with a DNA sequence shows in SEQ ID No.: 1,
- step of transforming the *Debaryomyces macquariensis* D50 yeast cells by the obtained linear DNA via electroporation, followed by spreading on a growth medium containing an antibiotic and incubation.

20. Method according to the claim 19, wherein the heterologous gene coding a thermolabile protein is used.

21. Method according to the claim 19, wherein the growth medium containing antibiotic Zeocin is used.

22. Method for a heterologous protein production by a recombinant strain of psychrotolerant yeast *Debaryomyces macquariensis* D50, accession number KKP 2066p, the recombinant strain is obtained according to one of the claims 19-21 wherein a liquid culture of the recombinant yeast strain is performing within a temperature range between 15 and 25°C.

23. Method according to the claim 22, wherein the culture is performed maintained at a temperature of, or close to 20°C.

## Patentansprüche

1. Das Expressionssystem zur Herstellung von heterologem Protein, wobei als Wirt für die Expression eines heterologen Gens Hefezellen verwendet werden, **dadurch gekennzeichnet, dass** es einen psychrotoleranten Hefestamm der Gattung *Debaryomyces macquariensis* und einen Plasmidexpressionsvektor umfasst, der eine Expressionskassette zur Expression eines heterologes Protein codierenden Gens enthält, die einen Promotor der Transkription des Gens, das aus *Debaryomyces macquariensis* isoliert wurde, und einen Terminator der Transkription des Gens *CYC1,* das aus *Debaryomyces macquariensis* isoliert wurde, umfasst, darüber hinaus umfasst der Plasmidexpressionsvektor eine Expressionskassette zur Expression eines Antibiotikaresistenzgens.

2. Das Expressionssystem nach Anspruch 1, **dadurch gekennzeichnet, dass** das heterologes Protein kodierende Gen ein thermolabiles Protein kodierendes Gen ist.

3. Das Expressionssystem nach Anspruch 1, **dadurch gekennzeichnet, dass** der Plasmidexpressionsvektor eine Expressionskassette zur Expression des Antibiotikaresistenzgens Zeosin umfasst.

4. Das Expressionssystem nach einem der Ansprüche 1-3, **dadurch gekennzeichnet, dass** als psychrotoleranter Hefestamm der Gattung *Debaryomyces macquariensis* der Stamm *Debaryomyces macquariensis* D50, Zugangsnummer KKP 2066p, verwendet wird.

5. Das Expressionssystem nach einem der Ansprüche 1-4, **dadurch gekennzeichnet, dass** der Plasmidexpressionsvektor eine Expressionskassette umfasst, die eine Expression eines heterologes Protein kodierenden Gens ermöglicht, die einen Promotor der Transkription des Gens *GAP,* das aus dem Stamm *Debaryomyces macquariensis* D50, Zugangsnummer KKP 2066p, mit Sequenzmuster DNA SEQ ID No.: 1 isoliert wurde, und einen Terminator der Transkription des Gens *CYC1,* das aus dem Stamm *Debaryomyces macquariensis* D50, Zugangsnummer KKP 2066p, mit Sequenzmuster DNA SEQ ID No.: 4 isoliert wurde, umfasst.

6. Das Expressionssystem nach einem der Ansprüche 1-5, **dadurch gekennzeichnet, dass** der Plasmidexpressionsvektor eine Expressionskassette zur Expression eines Antibiotikaresistenzgens umfasst, die einen Promotor der Transkription des Gens *IEF2,* das aus dem psychrotoleranten Hefestamm *Debaryomyces macquariensis* D50, Zugangsnummer KKP 2066p, mit Sequenzmuster DNA SEQ ID No.: 2, isoliert wurde, oder einen Promotor der Transkription des Gens *TEF1* des psychrotoleranten Hefestamms *Candida santamariae* G12 mit Sequenzmuster DNA SEQ ID No.: 3, oder einen Promotor der Transkription des Gens *TEF1* aus der Hefe *Saccharomyces cerevisiae* und einen Terminator der Transkription des Gens *CYCI* aus psychrotolerantem Hefestamm *Candida santamariae* G12 mit Sequenzmuster DNA SEQ ID No.: 5, oder einen Terminator der Transkription des Gens *CYCI* aus Hefe *Saccharomyces cerevisiae* enthält.

7. Der Plasmidexpressionsvektor mit Zeichen pH3 und Sequenzmuster DNA SEQ ID No. 13, **dadurch gekennzeichnet, dass** er eine Expressionskassette zur Expression eines heterologen Gens enthält, die einen Promotor der Transkription des Gens *GAP* aus dem Stamm *Debaryomyces macquariensis* D50, Zugangsnummer KKP 2066p, mit Sequenzmuster DNA SEQ ID No.: 1 und einen Terminator der Transkription des Gens *CYCI* aus dem Stamm *Debaryomyces macquariensis* D50, Zugangsnummer KKP 2066p, mit Sequenzmuster DNA SEQ ID No.: 4 enthält, und darüber hinaus eine Expressionskassette zur Expression eines Antibiotikaresistenzgens umfasst, die einen Promotor der Transkription des Gens *TEFI* aus *Saccharomyces cerevisiae* und einen Terminator des Gens *CYCI* aus *Saccharomyces cerevisiae* enthält.

8. Der Plasmidexpressionsvektor nach Anspruch 7, **dadurch gekennzeichnet, dass** das heterologe Gen ein heterologes Protein in Form eines thermolabilen Proteins kodierendes Gen ist.

9. Der Plasmidexpressionsvektor nach Anspruch 7, **dadurch gekennzeichnet, dass** der Plasmidexpressionsvektor eine Expressionskassette zur Expression des Antibiotikaresistenzgens Zeosine umfasst.

10. Der Plasmidexpressionsvektor mit Zeichen pH4 und Sequenzmuster DNA SEQ ID No. 17 **dadurch gekennzeichnet, dass** er eine Expressionskassette zur Expression eines heterologen Gens umfasst, die einen Promotor der Transkription des Gens *GAP* aus dem Stamm *Debaryomyces macquariensis* D50, Zugangsnummer KKP 2066p, mit Sequenzmuster DNA SEQ ID No.: 1 und einen Terminator der Transkription des Gens *CYC1* aus dem Stamm *Debaryomyces macquariensis* D50, Zugangsnummer KKP 2066p, mit Sequenzmuster DNA SEQ ID No.: 4 enthält, und darüber hinaus eine Expressionskassette zur Expression eines Antibiotikaresistenzgens umfasst, die einen Promotor der Transkription des Gens *TEFI* z *Candida santamariae* G12 mit Sequenzmuster DNA SEQ ID No.: 3 und einen Terminator der Transkription des Gens *CYCI* aus *Saccharomyces cerevisiae* umfasst.

11. Der Plasmidexpressionsvektor nach Anspruch 10, **dadurch gekennzeichnet, dass** das heterologe Gen ein heterologes Protein in Form eines thermolabilen Proteins kodierendes Gen ist.

12. Der Plasmidexpressionsvektor nach Anspruch 10, **dadurch gekennzeichnet, dass** Plasmidexpressionsvektor eine Expressionskassette zur Expression des Antibiotikaresistenzgens Zeosine umfasst.

13. Der Plasmidexpressionsvektor mit Zeichen pH5A und Sequenzmuster DNA SEQ ID No. 25 **dadurch gekennzeichnet, dass** er eine Expressionskassette zur Expression eines hetero logen Gens umfasst, die einen Promotor der Transkription des Gens *GAP* aus dem Stamm *Debaryomyces macquariensis* D50, Zugangsnummer KKP 2066p, mit Sequenzmuster DNA SEQ ID No.: 1 einen Terminator der Transkription des Gens *CYCI* aus dem Stamm *Debaryomyces macquariensis* D50, Zugangsnummer KKP 2066p, mit Sequenzmuster DNA SEQ ID No.: 4 enthält, und darüber hinaus eine Expressionskassette zur Expression eines Antibiotikaresistenzgens umfasst, die einen Promotor der Transkription des Gens *TEF1* z *Candida santamariae* G12 mit Sequenzmuster DNA SEQ ID No.: 3 und einen Terminator der Transkription des Gens *CYC1* aus *Candida santamariae* G12 mit Sequenzmuster DNA SEQ ID No.: 5 umfasst.

14. Der Plasmidexpressionsvektor nach Anspruch 13, **dadurch gekennzeichnet, dass** das heterologe Gen ein heterologes Protein in Form eines thermolabilen Proteins kodierendes Gen ist.

15. Der Plasmidexpressionsvektor nach Anspruch 13, **dadurch gekennzeichnet, dass** der Plasmidexpressionsvektor eine Expressionskassette zur Expression des Antibiotikaresistenzgens Zeosine umfasst.

16. Der Plasmidexpressionsvektor mit Zeichen pH6 und Sequenzmuster DNA SEQ ID No. 29 **dadurch gekennzeichnet, dass** er eine Expressionskassette zur Expression eines heterologen Gens umfasst, die einen Promotor der Transkription des Gens *GAP* aus dem Stamm *Debaryomyces macquariensis* D50, Zugangsnummer KKP 2066p, mit Sequenzmuster DNA SEQ ID No.: 1 einen Terminator der Transkription des Gens *CYC1* aus dem Stamm *Debaryomyces macquariensis* D50, Zugangsnummer KKP 2066p, mit Sequenzmuster DNA SEQ ID No.: 4 enthält, und darüber hinaus eine Expressionskassette zur Expression eines Antibiotikaresistenzgens umfasst, die einen Promotor der Transkription des Gens *IEF2* aus dem Stamm *Debaryomyces macquariensis* D50, Zugangsnummer KKP 2066p, mit Sequenzmuster DNA SEQ ID No.: 2 und einen Terminator der Transkription des Gens *CYC1* aus *Saccharomyces cerevisiae* umfasst.

17. Der Plasmidexpressionsvektor nach Anspruch 16, **dadurch gekennzeichnet, dass** das heterologe Gen ein heterologes Protein in Form eines thermolabilen Proteins kodierendes Gen ist.

18. Der Plasmidexpressionsvektor nach Anspruch 16, **dadurch gekennzeichnet, dass** der Plasmidexpressionsvektor eine Expressionskassette zur Expression des Antibiotikaresistenzgens Zeosine umfasst.

19. Das Verfahren zur Herstellung eines rekombinanten Stamms von psychrotoleranter Hefe *Debaryomyces macquariensis* D50, Zugangsnummer KKP 2066p, darin bestehend, dass das heterologes Protein kodierende Gen in einem Plasmidexpressionsvektor nach einem der Ansprüche von 7 bis 18 zwischen dem Promotor der Transkription des Gens *GAP* aus *Debaryomyces macquariensis* D50, Zugangsnummer KKP 2066p, mit Sequenzmuster DNA SEQ ID No.: 1 und dem Terminator der Transkription des Gens *CYCI* aus *Debaryomyces macquariensis* D50 mit Sequenzmuster DNA SEQ ID No.: 4 geklont wird, und danach die DNA des erzeugten Expressionsplasmids im Bereich des Promotors der Transkription des Gens *GAP* aus *Debaryomyces macquariensis* D50 mit Sequenzmuster DNA SEQ ID No.: 1 linearisiert wird, danach werden die Hefezellen *Debaryomyces macquariensis* D50, Zugangsnummer KKP 2066p mit der erzeugten DNA im Elektrotransformationsverfahren transformiert und danach auf den ein Antibiotikum enthaltenden Boden ausgesät.

20. Das Verfahren nach Anspruch 19, **dadurch gekennzeichnet, dass** ein heterologes Protein in Form eines thermolabilen Proteins kodierendes Gen verwendet wird.

21. Das Verfahren nach Anspruch 19, **dadurch gekennzeichnet, dass** ein das Antibiotikum Zeosine enthaltender Boden verwendet wird.

22. Das Verfahren zur Herstellung eines heterologen Proteins durch einen rekombinanten Stamm von psychrotoleranter Hefe *Debaryomyces macquariensis* D50, Zugangsnummer KKP 2066p, der nach einem der Ansprüche 19-21 erzeugt wird, bestehend darin, dass eine flüssige Zucht von rekombinantem Hefestamm bei Temperaturen von 15 bis 25 °C geführt wird.

23. Das Verfahren nach Anspruch 22, **dadurch gekennzeichnet, dass** die Zucht bei Temperaturen um 20 °C geführt wird.

## Revendications

1. Système d'expression pour produire une protéine hétérologue dans laquelle des cellules de levure sont utilisées comme hôte pour l'expression d'un gène hétérologue, **caractérisé en ce qu'**il comprend une souche de levure psychrotolérante de l'espèce *Debaryomyces macquariensis* et un vecteur d'expression plasmidique contenant une cassette d'expression pour exprimer l'expression du gène codant la protéine hétérologue qui contient le promoteur de transcription du gène *GAP* isolée de *Debaryomyces macquariensisi* le terminateur de transcription du gène *CYC1* isolé de *Debaryomyces macquariensis* et en plus le vecteur d'expression plasmidique contient une cassette d'expression pour exprimer l'expression du gène de résistance aux antibiotiques.

2. Système d'expression selon la revendication 1, **caractérisé en ce que** le gène codant pour la protéine hétérologue est le gène codant pour la protéine thermolabile.

3. Système d'expression selon la revendication 1, **caractérisé en ce que** le vecteur d'expression plasmidique comprend une cassette d'expression pour exprimer l'expression du gène de résistance à l'antibiotique Zéocine.

4. Système d'expression selon l'une quelconque des revendications 1 à 3, **caractérisé en ce que** qu'en tant que souche de levure psychrotolérante de l'espèce *Debaryomyces macquariensis* est utilisée la souche *Debaryomyces macquariensis D50,* le numéro d'acceptation du dépôt KKP 2066p

5. Système d'expression selon l'une quelconque des revendications 1 à 4, **caractérisé en ce que** le vecteur d'expression plasmidique contient une cassette d'expression permettant l'expression d'un gène codant pour une protéine hétérologue contenant le promoteur de transcription du gène *GAP,* isolée de la souche *Debaryomyces macquariensisD50,* le numéro d'acceptation de dépôt KKP 2066p, avec la formule de séquence d'ADN SEQ ID No: 1, et le terminateur de transcription du gène *CYC1* isolé de la souche *Debaryomyces macquariensis*D50, le numéro d'acceptation de dépôt KKP 2066p, avec le la formule de de séquence d'ADN SEQ ID No: 4.

6. Système d'expression selon l'une quelconque des revendications 1 à 5, **caractérisé en ce que** le vecteur d'expression plasmidique contient une cassette d'expression pour exprimer l'expression du gène de résistance aux antibiotiques contenant le promoteur de transcription du gène *IEF2,* isolé de la souche de levure psychotolérante *Debaryomyces macquariensis*D50*,* le numéro d'acceptation du dépôt KKP 2066p, ayant la formule de séquence d'ADN SEQ ID No: 2, ou le promoteur de transcription du gène TEF1 de la souche de levure psychrotolérante *Candida santamariae*G12 avec la formule de de séquence d'ADN SEQ ID No: 3, ou le promoteur de transcription du gène *TEF1* de la levure *Saccharomycescerevisiae* et le terminateur de transcription du gène *CYC1* de la souche psychrotolérante *Candida santamariae*G12 ayant la séquence d'ADN SEQ ID N °: 5, ou le terminateur de transcription du gène *CYC1* de la levure *Saccharomycescerevisiae.*

7. Vecteur d'expression plasmidique avec le symbole pH3 et la formule de séquence d'ADN SEQ ID No.13 **caractérisé en ce qu'**il comprend une cassette d'expression pour l'expression d'un gène hétérologue qui contient le promoteur de transcription du gène *GAP* de la souche *Debaryomyces macquariensis*D50*,* le numéro d'acceptation de dépôt KKP 2066p, avec la formule de séquence d'ADN SEQ ID n °: 1, et le terminateur de transcription du gène *CYC1* de la souche *Debaryomyces macquariens*D50, le numéro d'acceptation de dépôt KKP 2066p, ayant la formule de séquence d'ADN SEQ ID N °: 4, et comprend en plus une cassette d'expression pour exprimer l'expression du gène de résistance aux antibiotiques, qui contient le promoteur de transcription du gène *TEF1* de *Saccharomycescerevisiae* et le terminateur de transcription du gène *CYC1* de *Saccharomycescerevisiae.*

8. Vecteur d'expression plasmidique selon la revendication 7, **caractérisé en ce que** le gène hétérologue est un gène codant pour une protéine hétérologue sous forme d'une protéine thermolabile.

9. Vecteur d'expression plasmidique selon la revendication 7, **caractérisé en ce que** le vecteur d'expression plasmidique contient une cassette d'expression pour exprimer l'expression du gène de résistance à l'antibiotique Zéocine.

10. Vecteur d'expression plasmidique avec le symbole pH4 et la formule de séquence d'ADN SEQ ID No. 17, **caractérisé en ce qu'**il contient une cassette d'expression pour l'expression d'un gène hétérologue qui contient le promoteur de transcription du gène *GAP* de la souche *Debaryomyces macquariensis*D50*,* le numéro d'acceptation de dépôt KKP 2066p, avec la formule de séquence d'ADN SEQ ID n °: 1, et le terminateur de transcription du gène *CYC1* de la souche *Debaryomyces macquariens*D50*,* le numéro d'acceptation de dépôt KKP 2066p, avec la formule de séquence d'ADN SEQ ID N °: 4, et comprend en plus une cassette d'expression pour l'expression du gène de résistance aux antibiotiques, qui contient le promoteur de transcription du gène *TEF1* de *Candida santamariae*G12 avec la formule de séquence d'ADN SEQ ID N °: 3 et le terminateur de transcription du gène *CYC1* de *Saccharomycescerevisiae.*

11. Vecteur d'expression plasmidique selon la revendication 10, **caractérisé en ce que** le gène hétérologue est un gène codant pour une protéine hétérologue sous forme d'une protéine thermolabile.

12. Vecteur d'expression plasmidique selon la revendication 10, **caractérisé en ce que** le vecteur d'expression plasmidique comprend une cassette d'expression pour exprimer l'expression du gène de résistance à l' antibiotique Zéocine.

13. Vecteur d'expression plasmidique avec le symbole pH5A et ayant la formule de séquence d'ADN SEQ ID No25 **caractérisé en ce qu'**il comprend une cassette d'expression pour l'expression d'un gène hétérologue qui contient le promoteur de transcription du gène *GAP* de la souche *Debaryomyces macquariensis*D50, le numéro d'acceptation de dépôt KKP 2066p, avec la formule de séquence d'ADN SEQ ID No: 1 et le terminateur de transcription du gène *CYC1* de la souche *Debaryomyces macquariens*D50, le numéro d'acceptation de dépôt KKP 2066p, avec la formule de séquence d'ADN SEQ ID N °: 4, et comprend en plus une cassette d'expression pour l'expression du gène de résistance aux antibiotiques, qui contient le promoteur de transcription du gène *TEF1* de *Candida santamariae*G12 avec la formule de séquence d'ADN SEQ ID N °: 3 et le terminateur de transcription du gène *CYC1* de *Candida santamariae* G12 avec la formule de séquence d'ADN SEQ ID N °: 5.

14. Vecteur d'expression plasmidique selon la revendication 13, **caractérisé en ce que** le gène hétérologue est un gène codant pour une protéine hétérologue sous forme d'une protéine thermolabile.

15. Vecteur d'expression plasmidique selon la revendication 13, **caractérisé en ce que** le vecteur d'expression plasmidique contient une cassette d'expression pour exprimer l'expression du gène de résistance à l'antibiotique Zéocine.

16. Vecteur d'expression plasmidique avec le symbole pH6 et la formule de séquence d'ADN SEQ ID No. 29, **caractérisé en ce qu'**il comprend une cassette d'expression pour l'expression d'un gène hétérologue qui contient le promoteur de transcription du gène *GAP* de la souche *Debaryomyces macquariensis*D50*,* le numéro d'acceptation de dépôt KKP 2066p, avec la formule de séquence d'ADN SEQ ID No: 1 et le terminateur de transcription du gène *CYC1* de la souche *Debaryomyces macquariens*D50, le numéro d'acceptation du dépôt KKP 2066p, avec la formule de séquence d'ADN SEQ ID N °: 4, et comprend en oplus une cassette d'expression pour exprimer l'expression du gène de résistance aux antibiotiques qui contient le promoteur de transcription du gène *IEF2* de la souche *Debaryomyces macquariensis*D50*,* le numéro d'acceptation du dépôt KKP 2066p, avec la formule de séquence d'ADN SEQ ID N °: 2 et le terminateur de transcription du gène *CYC1* de *Saccharomycescerevisiae.*

17. Vecteur d'expression plasmidique selon la revendication 16, **caractérisé en ce que** le gène hétérologue est un gène codant pour une protéine hétérologue sous forme d'une protéine thermolabile.

18. Vecteur d'expression plasmidique selon la revendication 16, **caractérisé en ce que** le vecteur d'expression plasmidique contient une cassette d'expression pour exprimer l'expression du gène de résistance à l'antibiotique Zéocine.

19. Procédé d'obtention de la souche de levure psychotolérante recombinante *Debaryomyces macquariensis*D50, le numéro d'acceptation de dépôt KKP 2066p, dans laquelle le gène codant pour la protéine hétérologue est cloné dans un vecteur d'expression plasmidique selon l'une des revendications de 7 à 18, entre le promoteur de transcription du gène *GAP,* de *Debaryomyces macquariensis*D50*,* le numéro d'acceptation du dépôt KKP 2066p, avec la formule de séquence d'ADN SEQ ID n °: 1 et le terminateur de transcription du gène *CYC1* de *Debaryomyces macquariensis*D50 avec la formule de séquence d'ADN SEQ ID n °: 4, suivi de la linéarisation de l'ADN du plasmide d'expression reçu dans le promoteur de transcription du gène *GAP* de *Debaryomyces macquariensis*D50 avec la formule de séquence d'ADN SEQ ID N °: 1, après quoi l'ADN résultant est transformé en cellules de levure *Debaryomyces macquariensis*D50*,* le numéro d'acceptation de dépôt KKP 2066p, par la électrotransformation, puis semé sur un milieu contenant des antibiotiques.

20. Procédé selon la revendication 19, **caractérisé en ce que** le gène codant pour la protéine hétérologue sous forme de protéine thermolabile est utilisé.

21. Procédé selon la revendication 19, **caractérisé en ce que** le milieu contenant l'antibiotique Zeocin est utilisé.

22. Procédé de production d'une protéine hétérologue par la souche de levure psychotolérante recombinante *Debaryomyces macquariensis* D50, le numéro d'acceptation de dépôt KKP 2066p, obtenue selon l'une des revendications 19-21, consistant en une culture liquide de la souche de levure recombinante à une température de 15 à 25 ° C,

23. Procédé selon la revendication 22, **caractérisé en ce que** la culture est réalisée à une température voisine de 20 ° C.
